# EUROPEAN PATENT APPLICATION

(11) **EP 1 374 896 A1**
(43) Date of publication of application: **02.01.2004**
(21) Application number: 02708671.9
(22) Date of filing: 26.03.2002
(51) Int. Cl.: A61K 39/395, A61P 7/02, A61P 9/00

(54) **BLOOD RHEOLOGY IMPROVING AGENTS**

(30) Priority: 26.03.2001 JP 2001088387
(71) Applicant: Suzuki, Koji, Tsu-shi, Mie 514-0008 (JP)
(72) Inventor: Suzuki, Koji, Tsu-shi, Mie 514-0008 (JP)
(74) Representative: Harding, Charles Thomas
(86) International application number: PCT/JP2002/002933
(87) International publication number: WO 2002/078738

(57) **Abstract**

A blood rheology-improving agent comprising an antibody against human tissue factor (human TF).

## Description

### TECHNICAL FIELD

The present invention relates to a blood rheology-improving agent.

### BACKGROUND ART

At the onset of pulmonary thrombosis or deep venous thrombosis, which are on the increase in recent years, thrombus formation due to reduced blood flow at rest or at recumbency is believed to be a leading cause. Thus, in order to prevent or treat these diseases, there has been a demand for blood fluidity-improving agents i.e. blood rheology-improving agents that prevent reduction in blood flow. However, no drugs are known that have an activity of effectively improving blood rheology.

Thus, there has been a need for the development of drugs that improve blood rheology (fluidity).

### DISCLOSURE OF THE INVENTION

The present invention provides a novel blood rheology-improving agent.

After intensive and extensive study to solve the above problems, the present inventors have found that an antibody against human tissue factor (sometimes referred to as anti-human TF antibody, or anti-TF antibody) can improve blood rheology.

Thus, the present invention provides a blood rheology-improving agent comprising an anti-human TF antibody.

The above anti-human TF antibody can be a polyclonal antibody or a monoclonal antibody, or an altered antibody or an antibody modification. Monoclonal antibodies can be generally produced by hybridomas and can also be produced by gene recombinant technology, and altered antibodies and antibody modifications may generally be produced by gene recombinant technology. As altered antibodies, there can be mentioned chimeric antibodies, for example human-mouse chimeric antibodies, and as humanized antibodies, there can be mentioned versions b-b, i-b, and i-b2 specifically described below. As antibody modifications, there can be mentioned antibody fragments such as Fab, F(ab')₂, and Fv, and single chain Fv (referred to as scFv) that has been rendered single stranded by ligating the variable regions of antibody.

### BRIEF EXPLANATION OF THE DRAWINGS

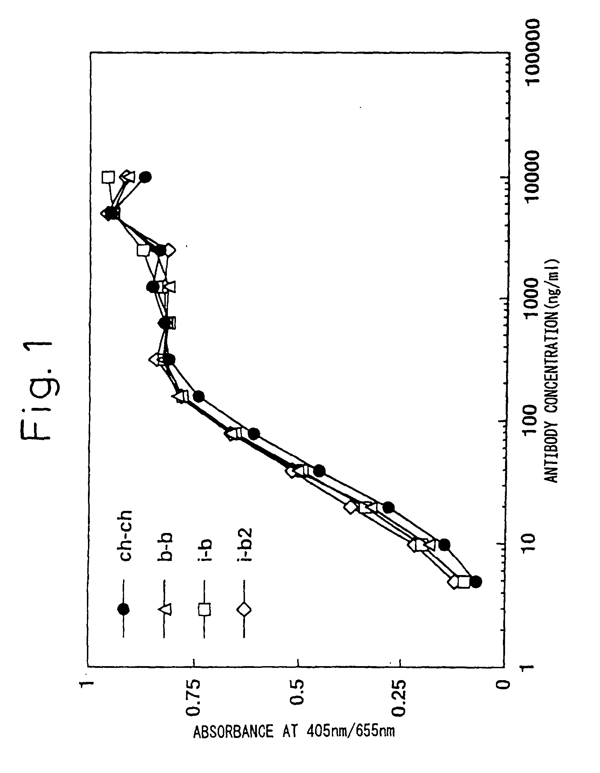

Fig. 1 is a graph that compares the antigen-binding activity of a H chain chimeric / L chain chimeric antibody, H chain humanized version b / L chain humanized version b antibody, H chain humanized version i / L chain humanized version b antibody, and H chain humanized version i / L chain humanized version b2 antibody.

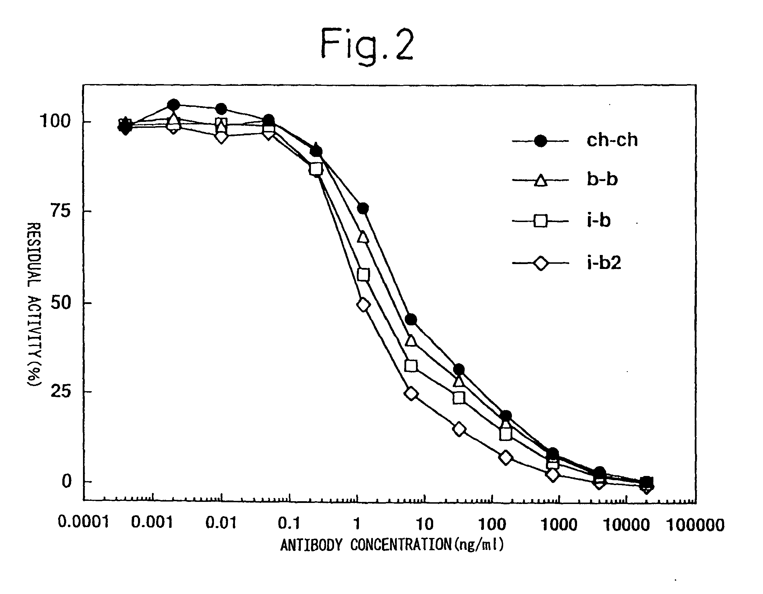

Fig. 2 is a graph that compares the neutralizing activity (activity of inhibiting Factor Xa production by TF) against human TF of a H chain chimeric / L chain chimeric antibody, H chain humanized version b / L chain humanized version b antibody, H chain humanized version i / L chain humanized version b antibody, and H chain humanized version i / L chain humanized version b2 antibody.

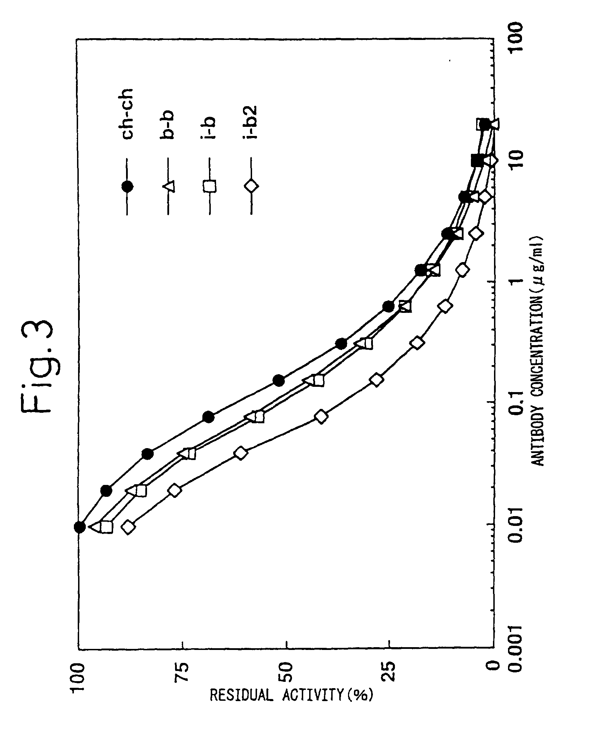

Fig. 3 is a graph that compares the neutralizing activity (activity of inhibiting Factor X binding) against human TF of a H chain chimeric / L chain chimeric antibody, H chain humanized version b / L chain humanized version b antibody, H chain humanized version i / L chain humanized version b antibody, and H chain humanized version i / L chain humanized version b2 antibody.

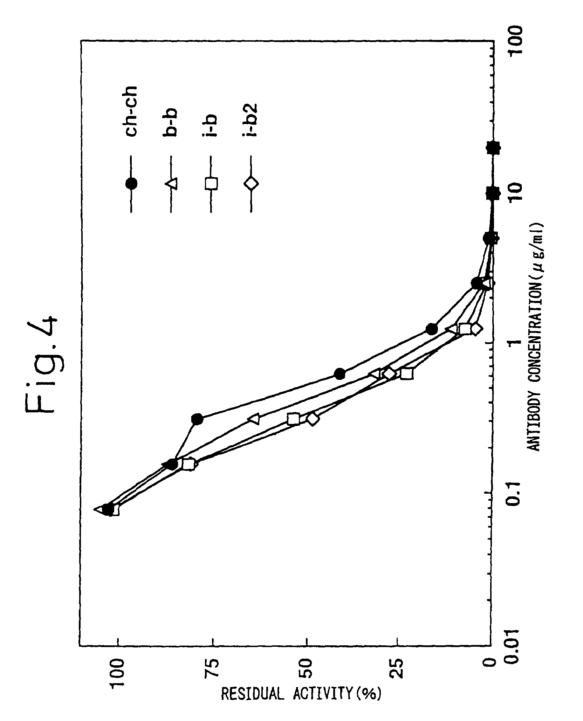

Fig. 4 is a graph that compares the neutralizing activity (activity of inhibiting blood coagulation by TF) against human TF of a H chain chimeric / L chain chimeric antibody, H chain humanized version b / L chain humanized version b antibody, H chain humanized version i / L chain humanized version b antibody, and H chain humanized version i / L chain humanized version b2 antibody.

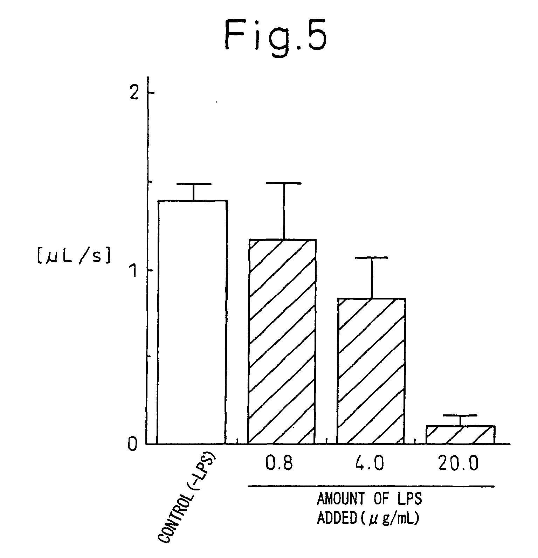

Fig. 5 is a graph showing that mononuclear cells treated with lipopolysaccharide (LPS) reduce blood fluidity.

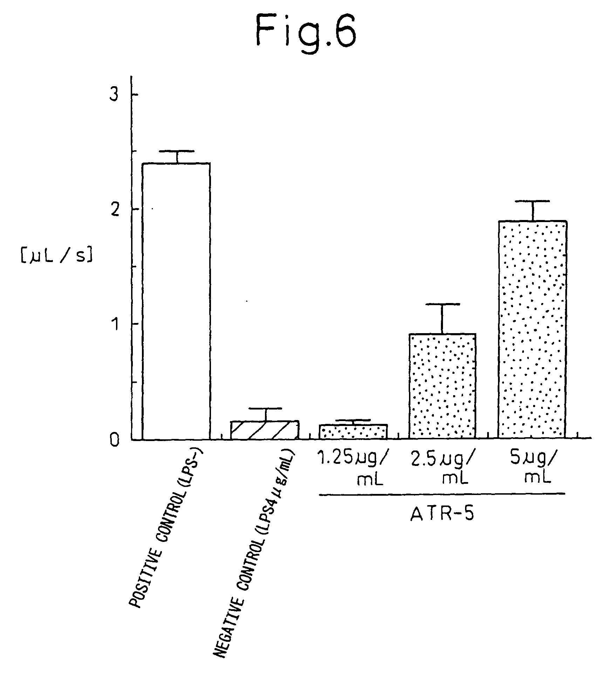

Fig. 6 is a graph showing that blood fluidity that is reduced by lipopolysaccharide can be improved by anti-human tissue factor antibody.

### EMBODIMENTS FOR CARRYING OUT THE INVENTION

As used herein, the improvement of blood rheology means that the flow of blood becomes well.

Antibodies for use in the present invention may be any of polyclonal antibody and monoclonal antibody as long as they can improve the rheology of blood, and monoclonal antibodies are preferred. There can be also used chimeric antibodies, humanized antibodies, single chain Fv etc. based on monoclonal antibody. Humanized antibodies are most preferred.

### 1. Anti-human TF antibody

Anti-human TF antibodies for use in the present invention may be of any origin, any type (monoclonal, polyclonal), and any shape, as long as they have an effect of improving blood rheology.

Anti-human TF antibodies for use in the present invention can be obtained as polyclonal or monoclonal antibodies using a known method. As anti-human TF antibodies for use in the present invention, monoclonal antibodies of, in particular, a mammalian origin, are preferred. Monoclonal antibodies of a mammalian origin include those produced by a hybridoma and those produced by a host which has been transformed with an expression vector containing genetically engineered antibody gene. These antibodies, via binding to human TF, inhibit human TF to induce a hypercoagulable state.

### 2. Antibody-producing hybridoma

A monoclonal antibody-producing hybridoma can be basically created using a known procedure as described below. Thus, human TF or a portion (fragment) thereof may be used as a sensitizing antigen and is immunized by the conventional method of immunization. The immune cells thus obtained are fused with known parent cells in the conventional cell fusion process, and then the monoclonal antibody-producing hybridomas are screened by the conventional screening method to prepare the desired hybridoma.

Specifically, the monoclonal antibody may be obtained in the following manner.

First, human TF used as the sensitizing antigen for antibody generation can be obtained by expressing the human TF gene/amino acid sequence disclosed in J. H. Morrissey et al., Cell, Vol. 50, p. 129-135 (1987). Thus, after the gene sequence encoding human TF is inserted into a known expression vector system and a suitable host cell is transformed, the human TF protein of interest is purified from the host cell or the culture supernatant thereof. This method is described in Reference Example 1 of this specification. Furthermore, human TF used as the antigen may be extracted from a TF-containing biological sample such as a human placenta according to a method described in Reference Example 2 and purified for use.

Then, the purified human TF protein is used as the sensitizing antigen. Alternatively, a soluble TF in which the transmembrane region at the C-terminal end of human TF has been removed may be generated by, for example, recombinant DNA technology and may be used as the sensitizing antigen.

Mammals to be immunized with the sensitizing antigen are preferably, but are not limited to, those selected in consideration of their compatibility with the parent cell for use in cell fusion. They generally include rodents such as mice, rats and hamsters, or rabbits, monkeys and the like.

Immunization of animals with a sensitizing antigen is carried out using a known method. A general method, for example, involves the intraperitoneal or subcutaneous administration of a sensitizing antigen to the mammal. Specifically, a sensitizing antigen which has been diluted and suspended in an appropriate amount of phosphate buffered saline (PBS) or physiological saline etc. is mixed, as desired, with an appropriate amount of a common adjuvant, for example Freund's complete adjuvant. After being emulsified, it is preferably administered to a mammal for several times every 4 to 21 days. Alternatively a suitable carrier may be used at the time of immunization of the sensitizing antigen.

After immunization of the mammal and the confirmation of the increase in the desired antibody levels in the serum, the immune cells are taken out from the mammal and are subjected to cell fusion. Preferred immune cells include in particular the spleen cells.

The mammalian myeloma cells are used as the other parent cells which are subjected to cell fusion with the above-mentioned immune cells. The myeloma cells preferably include various known cell lines such as P3(P3X63Ag8.653) (Kearney, J. F. et al., J. Immunol. (1979) 123: 1548-1550), P3X63Ag8U.1 (Yelton, D. E. et al., Current Topics in Microbiology and Immunology (1978) 81: 1-7), NS-1 (Kohler, G. and Milstein, C., Eur. J. Immunol. (1976) 6: 511-519), MPC-11 (Margulies, D.H. et al., Cell (1976) 8: 405-415), SP2/0 (Shulman, M. et al., Nature (1978) 276: 269-270), FO (de St. Groth, S. F. et al., J. Immunol. Methods (1980) 35: 1-21), S194 (Trowbridge, I.S., J. Exp. Med. (1978) 148: 313-323), R210 (Galfre, G. et al., Nature (1979) 277: 131-133) and the like.

Cell fusion between the above immune cells and the myeloma cells may be essentially conducted in accordance with a known method such as is described in Milstein et al. (Kohler, G. and Milstein, C., Methods Enzymol. (1981) 73: 3-46).

More specifically, the above cell fusion is carried out in the conventional nutrient broth in the presence of, for example, a cell fusion accelerator. As the cell fusion accelerator, for example, polyethylene glycol (PEG), Sendai virus (HVJ) and the like may be used, and, in addition, an adjuvant such as dimethyl sulfoxide etc. may be added as desired to enhance the efficiency of fusion.

The preferred ratio of the immune cells and the myeloma cells to be used is, for example, 1 to 10 times more immune cells than the myeloma cells. Examples of culture media to be used for the above cell fusion include RPMI1640 medium and MEM culture medium suitable for the growth of the above myeloma cell lines, and the conventional culture medium used for this type of cell culture and, besides, a serum supplement such as fetal calf serum (FCS) may be added.

In cell fusion, predetermined amounts of the above immune cells and the myeloma cells are mixed well in the above culture liquid, to which a PEG solution previously heated to about 37 °C, for example a PEG solution (a mean molecular weight of about 1000 to 6000), is added at a concentration of 30 to 60%(w/v) and mixed to obtain the desired fusion cells (hybridomas). Then, by repeating the sequential addition of a suitable culture liquid and centrifugation to remove the supernatant, cell fusion agents etc. which are undesirable for the growth of the hybridoma, can be removed.

The hybridoma thus obtained is selected by culturing in the conventional selection medium, for example, the HAT culture medium (a culture liquid containing hypoxanthine, aminopterin, and thymidine). Culturing in said HAT culture medium is continued generally for a period of time sufficient to effect killing of the cells (non-fusion cells) other than the desired hybridoma, generally several days to several weeks. Then, the conventional limiting dilution method is conducted in which the hybridomas that produce the desired antibody are screened and monoclonally cloned.

In addition to obtaining the above hybridoma by immunizing an animal other than the human with an antigen, it is also possible to sensitize human lymphocytes in vitro with human TF, and the resulting sensitized B lymphocytes are fused with a human myeloma cell capable of dividing permanently to obtain the desired human antibody having the activity of binding to human TF (see Japanese Post-examined Patent Publication (Kokoku) No. 1-59878). Furthermore, a transgenic animal having a repertoire of all or some human antibody genes is immunized with the antigen human TF to obtain human TF antibody-producing cells, which are then immortalized, from which the desired human antibody against human TF may be obtained (see International Patent Publication WO 94/25585, WO 93/12227, WO 92/03918, WO 94/02602, WO 96/34096, and WO 96/33735).

The monoclonal antibody-producing hybridomas thus constructed can be subcultured in the conventional culture liquid, or can be stored for a prolonged period of time in liquid nitrogen.

In order to obtain monoclonal antibodies from said hybridoma, a method can be used in which said hybridoma is cultured in the conventional method and the antibodies are obtained as the supernatant, or a method in which the hybridoma is administered to and grown in a mammal compatible with said hybridoma and the antibodies are obtained as the ascites. The former method is suitable for obtaining high-purity antibodies, whereas the latter is suitable for a large scale production of antibodies.

An example of monoclonal antibody production is specifically described in Reference Example 2. In this example, six monoclonal antibodies (termed as ATR-2, 3, 4, 5, 7, and 8) have been obtained. All of them can be used in the present invention, but ATR-5 is most preferred.

### 3. Recombinant antibody

A recombinant antibody, which was produced by the recombinant gene technology in which an antibody gene was cloned from the hybridoma and integrated into a suitable vector which was then introduced into a host, can be used in the present invention as monoclonal antibody (see, for example, Vandamme, A. M. et al., Eur. J. Biochem. (1990) 192, 767-775).

Specifically, mRNA encoding the variable (V) region of anti-human TF antibody is isolated from the anti-human TF antibody-producing hybridoma. The isolation of mRNA is conducted by preparing total RNA using, for example, a known method such as the guanidine ultracentrifuge method (Chirgwin, J.M. et al., Biochemistry (1979) 18, 5294-5299), the AGPC method (Chomczynski, P. et al., Anal. Biochem. (1987) 162, 156-159), and then mRNA is purified from the total RNA using an mRNA purification kit (manufactured by Pharmacia) and the like. Alternatively, mRNA can be directly prepared using the Quick Prep mRNA Purification Kit (manufactured by Pharmacia).

cDNA of the V region of antibody may be synthesized from the mRNA thus obtained using a reverse transcriptase. cDNA may be synthesized using the AMV Reverse Transcriptase First-strand cDNA Synthesis Kit (manufactured by Seikagaku Kogyo) and the like. Alternatively, for the synthesis and amplification of cDNA, the 5'-Ampli FINDER RACE Kit (manufactured by Clontech) and the 5'-RACE method (Frohman, M.A. et al., Proc. Natl. Acad. Sci. USA (1988) 85, 8998-9002; Belyavsky, A. et al., Nucleic Acids Res. (1989) 17, 2919-2932) that employs polymerase chain reaction (PCR) may be used.

The desired DNA fragment is purified from the PCR product obtained and may be ligated to vector DNA. Moreover, a recombinant vector is constructed therefrom, and then is introduced into E. coli etc., from which colonies are selected to prepare the desired recombinant vector. The base sequence of the desired DNA may be confirmed by a known method such as the dideoxy nucleotide chain termination method.

Once DNA encoding the V region of the desired anti-human TF antibody has been obtained, it may be integrated into an expression vector containing DNA encoding the constant region (C region) of the desired antibody.

In order to produce anti-human TF antibody for use in the present invention, the antibody gene is integrated as described below into an expression vector so as to be expressed under the control of an expression regulatory region, for example an enhancer and/or a promoter. Subsequently, a host cell may be transformed by the expression vector and the antibody can then be expressed therein.

For expression of the antibody gene, DNA encoding the heavy chain (H chain) or the light chain (L chain) of antibody may be separately integrated into expression vectors and the hosts are transformed simultaneously, or DNA encoding the H chain and the L chain may be integrated into a single expression vector and the host is transformed therewith (see International Patent Publication WO 94-11523).

Furthermore, in addition to the above host cells, transgenic animals may be used for the production of recombinant antibody. For example, an antibody gene is inserted into the middle of the gene encoding protein (such as goat β casein) which is inherently produced in the milk to prepare fusion genes. DNA fragments containing the fusion gene into which the antibody gene has been inserted are injected into a goat embryo, and the embryo is introduced into a female goat. The desired antibody is obtained from the milk produced by the transgenic goat born to the goat who received the embryo or offsprings thereof. In order to increase the amount of milk containing the desired antibody produced by the transgenic goat, hormones may be given to the transgenic goat as appropriate. (Ebert, K.M. et al., Bio/Technology (1994) 12, 699-702).

An example of the method of producing recombinant antibody is specifically described in Reference Example 3.

### 4. Altered antibody

In accordance with the present invention, artificially altered recombinant antibody such as chimeric antibody and humanized antibody can be used for the purpose of lowering heterologous antigenicity against humans. These altered antibody can be produced using known methods.

Chimeric antibody can be obtained by ligating the thus obtained DNA encoding the V region of antibody to DNA encoding the C region of human antibody, which is then integrated into an expression vector and introduced into a host for the production of the antibody therein. Using this known method, chimeric antibody useful for the present invention can be obtained.

Humanized antibody which is also called reshaped human antibody has been generated by transplanting the complementarity determining region (CDR) of antibody of a mammal other than the human, for example mouse antibody, into the CDR of human antibody. A general recombinant DNA technology for preparation of such antibodies is also known (see European Patent Application EP 125023 and WO 96/02576).

Specifically, a DNA sequence which was designed to ligate the CDR of mouse antibody with the framework region (FR) of human antibody is synthesized by the PCR method using, as the primers, several divided oligonucleotides that were constructed so as to have sections overlapping with one another at the ends of the CDR and the FR (see the method described in International Patent Publication WO 98/13388)

For the framework region of human antibody ligated through CDR, a complementarity determining region that forms a favorable antigen binding site is selected. When desired, amino acids in the framework region of the antibody variable region may be substituted so that the complementarity determining region of reshaped human antibody may form an appropriate antigen biding site (Sato, K. et al., Cancer Res. (1993) 53, 851-856).

For example, for chimeric antibody or humanized antibody, the C region of human antibody is used. For H chain, for example, Cγ1, Cγ2, Cγ3, and Cγ4 can be used, and for L chain, Cκ and Cλ can be used. The C region of human antibody may be modified to improve the stability of antibody or the production thereof.

Chimeric antibody consists of the variable region of antibody derived from a mammal other than the human and the constant region derived from human antibody, whereas humanized antibody consists of the complementarity determining region of antibody derived from a mammal other than the human and the framework region and the C region derived from human antibody. Accordingly, antigenicity thereof in the human body has been reduced so that they are useful as an active ingredient of a therapeutic agent for use in the present invention.

The method of constructing chimeric antibody is specifically described in Reference Example 4.

The method of constructing humanized antibody is specifically described in Reference Example 5.

In this Reference Example, as the humanized heavy chain (H chain) variable region (V region), versions a, b, c, d, e, f, g, h, i, j, b1, d1, b3 and d3 having the amino acid sequence shown in Table 1 and Table 2 were used.

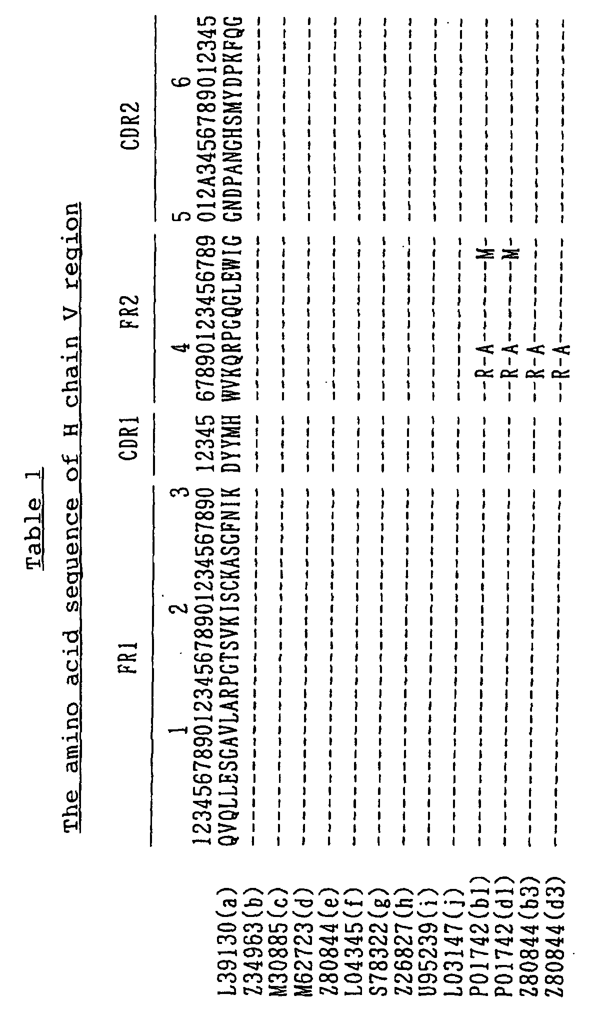

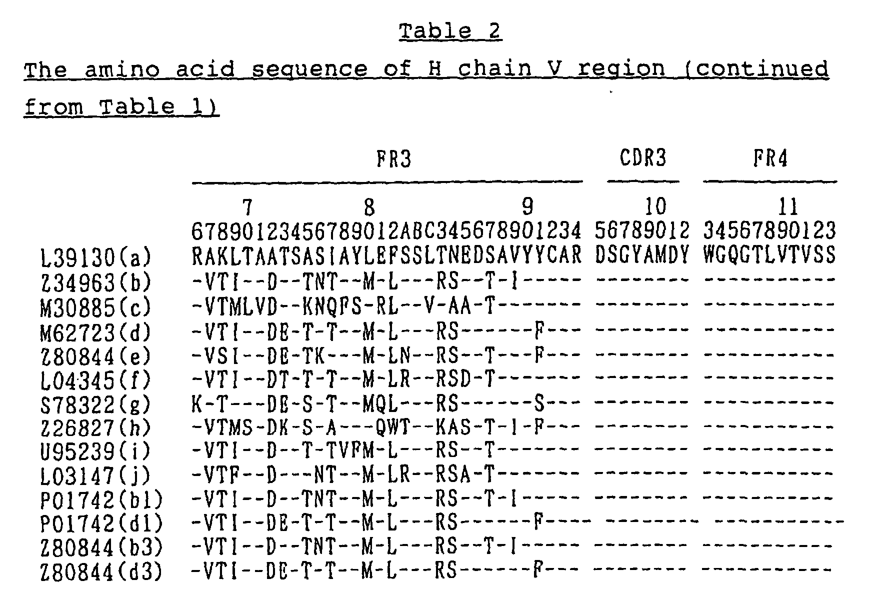

Also, as the humanized light chain (L chain) V region, versions a, b, c, b1 and b2 having the amino acid sequence shown in Table 3 were used.

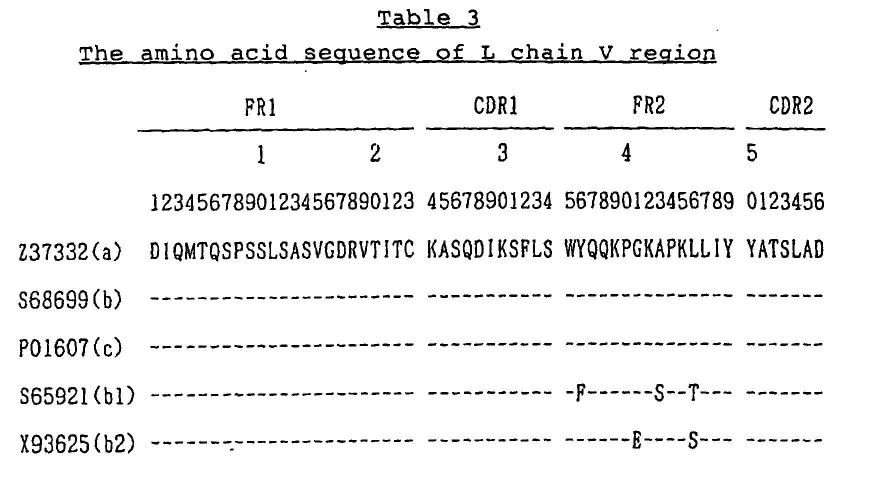

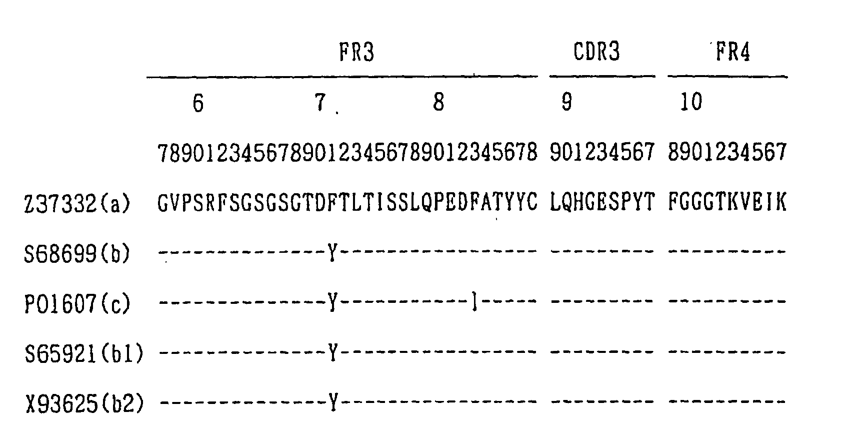

Various versions of the above H chain V region and various versions of the L chain V region were combined and their antigen binding ability and the TF-neutralizing activity were evaluated. As shown in Reference Example 6 and Reference Example 7, it was shown that when expressed as "H chain V region version" - "L chain V region version", "b-b", "i-b" and "i-b2" exhibited particularly high activities. The antigen-binding ability of these humanized antibodies is shown in Figure 1, the activity of neutralizing human TF (the activity of inhibiting the production of Factor Xa by TF) is shown in Figure 2, the activity of neutralizing human TF (the activity of inhibiting the production of Factor X by TF) is shown in Figure 3, and the activity of neutralizing human TF (the activity of inhibiting plasma coagulation by TF) is shown in Figure 4.

### 5. Antibody modifications

Antibodies for use in the present invention may be antibody fragments or modified versions thereof as long as they bind to human TF and inhibit the activity of human TF. For example, as fragments of antibody, there may be mentioned Fab, F(ab')₂, Fv or single-chain Fv (scFv) in which Fv's of H chain or L chain were ligated via a suitable linker.

Specifically, antibodies are treated with an enzyme, for example, papain or pepsin, to produce antibody fragments, or genes encoding these antibody fragments are constructed, and then introduced into an expression vector, which is expressed in a suitable host cell (see, for example, Co, M. S. et al., J. Immunol. (1994) 152, 2968-2976; Better, M. and Horwitz, A.H., Methods in Enzymology (1989) 178, 476-496; Plucktrun, A. and Skerra, A., Methods in Enzymology (1989) 178, 497-515; Lamoyi, E., Methods in Enzymology (1986) 121, 652-663; Rousseaux, J. et al., Methods in Enzymology (1986) 121, 663-669; Bird, R.E. et al., TIBTECH (1991) 9, 132-137).

scFv can be obtained by ligating the V region of H chain and the V region of L chain of antibody. In the scFv, the V region of H chain and the V region of L chain are ligated via a linker, preferably a peptide linker (Huston, J.S. et al., Proc. Natl. Acad. Sci. USA (1988) 85, 5879-5883). The V region of H chain and the V region of L chain in the scFv may be derived from any of the above-mentioned antibodies. As the peptide linker for ligating the V regions, any single-chain peptide comprising, for example, 12-19 amino acid residues may be used.

DNA encoding scFv can be obtained using DNA encoding the H chain or the H chain V region of the above antibody and DNA encoding the L chain or the L chain V region of the above antibody as the template by amplifying the whole or the portion of the DNA encoding the desired amino acid sequence among the above sequences by the PCR technique with the primer pair specifying the both ends thereof, and by further amplifying the combination of DNA encoding the peptide linker portion and the primer pair which defines that both ends of said DNA be ligated to the H chain and the L chain, respectively.

Once DNA encoding scFv is constructed, an expression vector containing it and a host transformed with said expression vector can be obtained by conventional methods, and scFv can be obtained using the resultant host by the conventional methods.

These antibody fragments can be produced by obtaining the gene thereof in a similar manner to that mentioned above and by allowing it to be expressed in a host. "Antibody" as used herein encompasses these antibody fragments.

As antibody modifications, anti-human TF antibody associated with various molecules such as polyethylene glycol (PEG) can be used. "Antibody" as used herein encompasses these antibody modifications. These antibody modifications can be obtained by chemically modifying the antibodies thus obtained. These methods have already been established in the art.

### 6. Expression and production of recombinant antibody or altered antibody

The antibody gene constructed as described above can be expressed and obtained using a known method. In the case of a mammal, a commonly used useful promoter, the human tissue factor gene, and a poly A signal to 3'-end downstream thereof can be operably linked and can be expressed. As the promoter/enhancer, for example, there can be mentioned human cytomegalovirus immediate early promoter/enhancer.

As other promoters/enhancers that may be used for the expression of antibody for use in the present invention, there can be mentioned viral promoters/enhancers of retrovirus, polyoma virus, adenovirus, simian virus 40 (SV40), and the like, and promoters/enhancers derived from mammalian cells such as human elongation factor 1α (HEF1α).

For example, gene expression may be readily accomplished by the method of Mulligan et al. (Nature (1979) 277, 108-114) when SV40 promoter/enhancer is used, or by the method of Mizushima et al. (Nucleic Acids Res. (1990) 18, 5322) when HEF1α promoter/enhancer is used.

In the case of E. coli, expression may be conducted by operably linking a commonly used useful promoter, a signal sequence for antibody secretion, and the antibody gene to be expressed, followed by expression thereof. As the promoter, for example, there can be mentioned lacz promoter and araB promoter. The method of Ward et al. (Nature (1098) 341, 544-546; Ward, E.S. et al., FASEB J. (1992) 6, 2422-2427) may be used when lacz promoter is used, and the method of Better et al. (Science (1988) 240, 1041-1043) may be used when araB promoter is used.

As the signal sequence for antibody secretion, when produced in the periplasm of E. coli, the pelB signal sequence (Lei, S. P. et al., J. Bacteriol. (1987) 169, 4379-4383) can be used. After separating the antibody produced in the periplasm, the structure of the antibody is appropriately refolded before use.

As the origin of replication, there can be used those derived from SV40, polyoma virus, adenovirus, bovine papilloma virus (BPV) and the like. Furthermore, for the amplification of the gene copy number in the host cell system, expression vectors can include as selectable markers the aminoglycoside phosphotransferase (APH) gene, the thymidine kinase (TK) gene, E. coli xanthine guaninephosphoribosyl transferase (Ecogpt) gene, the dihydrofolate reductase (dhfr) gene and the like.

For the production of antibody for use in the present invention, any production system can be used, for example a production system which employs eukaryotic cells and the production system which employs prokaryotic cells. As the eukaryotic cells, there can be mentioned, for example, the mammalian cell system, the insect cell system, and the fungal cell system such as filamentous fungal cells and yeast cells, and as the prokaryotic cells, there can be mentioned, for example, bacterial cells such as E. coli cells.

Preferably the antibody for use in the present invention may be expressed in mammalian cells such as CHO cells, COS cells, myeloma cells, baby hamster kidney (BHK) cells, Vero cells, and HeLa cells.

By culturing the transformed cells in vivo or in vitro, the antibody of interest can be obtained. Culturing is conducted by a known method. For example, as the culture liquid, DMEM, MEM, RPMI1640, and IMDM can be used, and serum supplements such as fetal calf serum (FCS) may be used in combination.

### 7. Separation and purification of antibody

Antibodies produced and expressed as described above can be separated from the host cell and then may be purified to homogeneity. Separation and purification of the antibody for use in the present invention may be accomplished by affinity chromatography. As the column that employs Protein A column, there can be mentioned Hyper D, POROS, Sepharose F. F. (manufactured by Pharmacia) and the like. Alternatively, methods for separation and purification conventionally used for proteins can be used without any limitation. Separation and purification of the antibody may be accomplished by combining, as appropriate, chromatography other than the above-mentioned affinity chromatography, filtration, ultrafiltration, salting-out, dialysis and the like. (Antibodies: A Laboratory Manual. Ed. Harlow and David Lane, Cold Spring Harbor Laboratory, 1988).

### 8. Confirmation of the effect of improving blood rheology

Blood rheology can be observed by determining the blood flow of peripheral blood in vitro using a cell micro rheology measurement instrument, etc. Treatment with LPS caused reduced blood flow, but the blood flow was improved by the addition of the anti-human TF antibody of the present invention. It was confirmed therefore that the anti-human TF antibody of the present invention is effective for improving blood rheology. Diseases that require such improvement include arteriosclerosis, pulmonary embolism, deep venous thrombosis, chronic arterial obstruction, etc., and the reduced blood flow associated with these diseases can be improved by anti-human TF antibody of the present invention.

The effect is specifically described in the Examples.

### 9. Method of administration and formulation

The therapeutic agent of the present invention is used for the purpose of improving blood rheology.

Effective dosage per administration is selected from the range of 0.001 mg to 1000 mg/kg body weight. Alternatively, the dosage of 0.01 to 100 mg/kg, preferably 0.1 to 10 mg/kg may be selected. However, the therapeutic agent containing anti-human TF antibody of the present invention is not limited to these dosages.

Preferably the method of administration is, but is not limited to, intravenous injection, intravenous drip, and the like.

The therapeutic agent of the present invention comprising anti-human TF antibody as an active ingredient may be formulated using a standard method (Remington's Pharmaceutical Science, the latest edition, Mark Publishing Company, Easton, USA), and may contain pharmaceutically acceptable carriers and/or additives.

Examples of such carriers or additives include water, a pharmaceutically acceptable organic solvent, collagen, polyvinyl alcohol, polyvinylpyrrolidone, a carboxyvinyl polymer, carboxymethylcellulose sodium, polyacrylic sodium, sodium alginate, water-soluble dextran, carboxymethyl starch sodium, pectin, methyl cellulose, ethyl cellulose, xanthan gum, gum Arabic, casein, agar, polyethylene glycol, diglycerin, glycerin, propylene glycol, Vaseline, paraffin, stearyl alcohol, stearic acid, human serum albumin (HSA), mannitol, sorbitol, lactose, a pharmaceutically acceptable surfactant and the like.

Additives used are chosen from, but not limited to, the above or combinations thereof, as appropriate, depending on the dosage form of the present invention. For example, when used as injections, purified anti-human TF antibody may be dissolved in a solvent such as physiological saline, a buffer, and a glucose solution, to which an anti-adsorbent such as Tween 80, Tween 20, gelatin, and human serum albumin may be added. Alternatively, they may be lyophilized so as to be dissolved and reconstituted into a dosage form before use. As the excipient for lyophilization, sugar alcohols and sugars such as mannitol and glucose may be used.

### EXAMPLES

The present invention will now be explained more specifically with reference to Examples.

Three-point-five ml of peripheral blood is added to 3 ml of Mono-Poly separation solution, and is centrifuged at 1,500 rpm (x 700G) for 20 minutes. The mononuclear cell fraction thus collected is washed with phosphate buffered saline (PBS) in a 50 ml Falcon tube, and centrifuged to collect cell pellets, which are dispersed at various concentrations in a 10% PBS-containing RPMI 1640 medium containing lipopolysaccharide (LPS). The concentration of lipopolysaccharide was the 0 µg/ml no-addition group (negative control) and the 4.0 µg/ml addition group.

The cell suspension thus prepared was incubated in a 100 mm culture dish in a CO₂ incubator for 6 hours. Then, the cells attached to the culture dish were scraped by a scraper, and after adjusting the cell concentration to 6 x 10⁵ in phosphate buffered saline (PBS), they were centrifuged at 800 rpm (x 200G) for 10 minutes, and the cell pellets were collected, washed in phosphate buffered saline (PBS), and centrifuged to collect cell pellets.

Then, in order to investigate blood coagulation activity of the above lipopolysaccharide-treated mononuclear cells, 6 x 10⁵ cells were added to 1000 µl of whole blood. After the mixture was incubated at room temperature for 10 minutes, 500 ng of activated Factor 7 (FVIIa) and CaCl₂ at a final concentration of 5 mM were added thereto, which was applied to a cell micro rheology measurement instrument (MCFAN-KH; Hitachi Haramachi Electronics Co., Ltd). This instrument simulates the capillary blood vessels, and is constructed in such a way that a multitude of fine V-shaped grooves are formed on silicon single crystal, whereupon a glass substrate has been pressure-deposited to form artificial capillary blood vessels, and through the glass substrate, the flow of blood cells can be observed using a microscope. The result is expressed in the volume of blood that flowed through the capillary blood vessels per unit time and is shown in Fig. 5. From the figure, it was confirmed that the fluidity of the blood decreases depending on the amount of polyliposaccharide.

Thus, at the time of incubation of mixture of the above lipopolysaccharide-treated (4 µg/ml) mononuclear cells and whole blood, anti-human tissue factor antibody ATR-5 (produced in Reference Example 2) was added at 1.25 µg/ml, 2.5 µg/ml, or 5 µg/ml, or not added (negative control), and incubated. As the positive control, a test was carried out by adding mononuclear cells not treated with lipopolysaccharide to whole blood. Then, the fluidity of the incubated mixture was measured as described above using the cell micro rheology measurement instrument. The result is shown in Fig. 6. It was confirmed that anti-human tissue factor antibody improves blood fluidity.

### Reference Example 1. Method of preparing soluble human TF

Soluble human TF (shTF) was prepared in the following manner.

The gene encoding the human TF penetrating region (amino acid at position 220) and thereafter that had been replaced with the FLAG peptide M2 was inserted to a mammalian cell expression vector (containing the neomycin resistant gene and the DHFR gene), and introduced into CHO cells. For the cDNA sequence of human TF, reference was made to an article by James H. Morrissey et al. (Cell (1987) 50: 129-135). The gene sequence and the amino acid sequence of this soluble human TF are shown in SEQ ID NO: 101 and 102. After drug selection with G418, the expressing cells were selected, which were then subjected to expression amplification with methotrexate, and the shTF-expressing cells were established.

The cells were cultured in the serum-free medium CHO-S-SFMII (GIBCO) to obtain a culture supernatant containing shTF. The supernatant was diluted two times with an equal volume of a 40 mM Tris-HCl buffer (pH 8.5), which was added to the Q-Sepharose Fast Flow column (100 ml, Pharmacia Biotech) equilibrated with a 20 mM Tris-HCl buffer (pH 8.5). After washing with the same buffer containing 0.1 M NaCl, the concentration of NaCl was changed to 0.3 M, and shTF was eluted from the column. To the shTF fraction obtained, ammonium sulfate was added to a final concentration of 2.5 M, and was centrifuged (10,000 rpm, 20 minutes) to precipitate the contaminating proteins. The supernatant was added to Butyl TOYOPEARL (30 ml, TOSOH), and then was washed with a 50 mM Tris-HCl buffer (pH 6.8) containing 2.5 M ammonium sulfate.

In the 50 mM Tris-HCl buffer (pH 6.8), the concentration of ammonium sulfate was linearly reduced from 2.5 M to 0 M to permit the elution of shTF. The peak fractions containing shTF were concentrated by the Centri-Prep 10 (Amicon). The concentrate was added to the TSKgel G3000SWG column (21.5 × 600 mm, TOSOH) equilibrated with a 20 mM Tris-HCl buffer (pH 7.0) containing 150 mM NaCl, and the peak fraction of shTF was collected. It was filter sterilized with a 0.22 µm membrane filter and the product was set as the soluble human TF (shTF). The concentration of the sample was calculated assuming that the molar extinction coefficient of the sample ε = 40,130 and molecular weight = 43,210.

### Reference Example 2. Preparation of anti-TF monoclonal antibody

### 1. Purification of human TF

The purification of TF from human placenta was carried out according to the method of Ito (Ito, T. et al., J. Biol. Chem., 114: 691-696, 1993). Thus, human placenta was homogenized in a Tris buffered saline (TBS, pH 7.5) containing 10 mM benzamidine hydrochloride, 1 mM phenylmethylsulfonyl fluoride, 1 mM diisopropylfluoro phosphate, and 0.02% sodium azide, and then the precipitate was defatted with cold acetone. The defatted powder obtained was suspended in the above buffer containing 2% Triton X-100 to solubilize TF.

The supernatant was subjected to affinity chromatography using Concanavalin A-Sepharose 4B column (Pharmacia) and anti-TF antibody-bound Sepharose 4B column (Pharmacia) to obtain purified TF. This was concentrated with an ultrafiltration membrane (PM-10, Amicon) and was stored, as the purified sample, at 4 °C.

TF content in the purified sample was quantitated by Sandwich ELISA that combined a commercially available anti-TF monoclonal antibody (American Diagnostica) and polyclonal antibody (American Diagnostica) with recombinant TF as a standard.

The purity in the purified sample was confirmed by subjecting the sample to SDS-PAGE using a 4-20% density gradient polyacrylamide gel, and silver-staining the product.

### 2. Immunization and the preparation of hybridoma

After mixing the purified human TF (about 70 µg/ml) with an equal volume of Freund's complete adjuvant (Difco), it was immunized subcutaneously into the abdomen of 5-week old Balb/c male mice (Nippon Charles River) at 10 µg TF/mouse. On day 12, 18, and 25 after the initial immunization, TF mixed with Freund's incomplete adjuvant was subcutaneously boosted at 5 µg/mouse TF and, as a final immunization, the TF solution diluted with PBS was intraperitoneally given at 5 µg/mouse on day 32.

Three days after the final immunization, the spleen cells were prepared from four mice, and were fused to the mouse myeloma cell line P3U1 at about 1/5 cell count thereof by the polyethylene glycol method. The fused cells were suspended into the RPMI-1640 medium (hereinafter referred to as RPMI-medium) (Lifetechoriental) containing 10% fetal bovine serum, which was inoculated in 400 wells per mouse of a 96-well plate. On day 1, 2, 3, and 5 after the fusion, half the volume of the medium was exchanged with the RPMI-medium (hereinafter referred to as HAT-medium) containing HAT (Dainippon Seiyaku) and condimed H1 (Boehringer Mannheim GmbH) to perform HAT selection of the hybridoma.

The hybridomas selected by the screening method described below were cloned by conducting limiting dilution twice.

For the limiting dilution, 0.8 cells was inoculated per well in two 96-well plates. For the wells in which a single colony was confirmed by microscopic examination, clones were selected by the following measurement of the TF-binding activity and TF-neutralizing activity. The clones obtained were acclimated from the HAT-medium to the RPMI-medium. After confirming the absence of reduction in antibody production ability due to acclimation, limiting dilution was performed again for complete cloning. By the foregoing procedure, hybridomas that produce six antibodies (ATR-2, 3, 4, 5, 7, and 8) that strongly inhibit the binding of TF/Factor VIIa complex and Factor X were established.

### 3. Ascites formation and antibody purification

The ascites formation of the established hybridomas were carried out according to the standard method. Thus, 10⁶ hybridomas that were subcultured in vitro were intraperitoneally grafted into BALB/c male mice that had previously received two intravenous administrations of mineral oil. Ascites was collected from the mice that showed a bloated abdomen 1-2 weeks after the grafting.

The purification of antibody from ascites was carried out using the ConSepLC100 system (Millipore) equipped with the Protein A column (Nippon Gaishi).

### 4. Cell-ELISA

Human cystocarcinoma cell line J82 (Fair D. S. et al., J. Biol. Chem., 262: 11692-11698, 1987) that is known to express TF at a high level was obtained from ATCC, and subcultured and maintained in the RPMI-medium under the condition of 37°C, 5% CO₂, and 100% humidity.

Cell-ELISA plates were prepared by inoculating J82 cells to a 96-well plate at 10⁵ cells/well, culturing for one day under the above condition, removing the medium and then washing twice with phosphate buffered saline (PBS), adding a 4% paraformaldehyde solution (PFA), and allowing to stand on ice, for 10 minutes, for immobilization. After PFA was removed, the plate was washed with PBS, the Tris buffer (Blocking buffer) containing 1% BSA and 0.02% sodium azide was added thereto, and the plate was stored at 4 °C until use.

Cell-ELISA was carried out in the following manner. Thus, the Blocking buffer was removed from the plate prepared as above, to which an anti-TF antibody solution or a hybridoma culture supernatant was added and was reacted at room temperature for 1.5 hours. After washing with PBS containing 0.05% Tween 20, alkaline phosphatase-conjugated goat anti-mouse IgG (H+L) (Zymed) was reacted for 1 hour. After washing, 1 mg/ml p-nitrophenyl phosphate disodium (Sigma) was added, and one hour later absorbance at 405 nm was measured to determine the amount of anti-TF antibody that bound to the J82 cells.

### 5. Assay system of TF-neutralizing activity with Factor Xa activity as an index

To 50 µl of Tris buffered saline (TBS: pH 7.6) containing 5 mM CaCl₂ and 0.1% bovine serum albumin, 10 µl of a human placenta-derived thromboplastin solution (5 mg/ml) (Thromborel S) (Boehring) and 10 µl of a Factor VIIa solution (82.5 ng/ml) (American Diagnostica) were added, and reacted at room temperature for 1 hour to permit the formation of the TF/Factor VIIa complex. After 10 µl of a predetermined concentration of a diluted anti-TF antibody solution or the hybridoma culture supernatant and 10 µl of a Factor X solution (3.245 µg/ml) (Celsus Laboratories) were added and reacted for 45 minutes, 10 µl of 0.5 M EDTA was added to stop the reaction. Fifty µl of 2 mM S-2222 solution (Daiichi Kagaku Yakuhin) was added thereto, and changes in absorbance at 405 nm over 30 minutes were measured and were set as the Factor X-producing activity of TF. In this method, the activity of antibody that inhibits the binding of the TF/Factor VIIa complex and Factor X can be determined.

### 6. Assay system of plasma coagulation-inhibiting activity

Fifty µl of an appropriately diluted anti-TF antibody solution was mixed with 100 µl of a commercially available normal human plasma (Kojin Bio) and reacted at 37°C for 3 minutes. Then 50 µl of a human placenta-derived thromboplastin solution (1.25 mg/ml) was added thereto, and the time to coagulation of the plasma was measured using the plasma coagulation measuring instrument (CR-A: Amelung).

### 7. Determination of antibody isotype

For the culture supernatant of the hybridoma and the purified antibody, the mouse monoclonal antibody isotyping kit (manufactured by Amersham) was used to confirm the isotype of antibody. The result is shown below.

**Table 5**

| Immunoglobulin isotype of anti-TF monoclonal antibody | |
|---|---|
| ATR-2 | IgG1, k |
| ATR-3 | IgG1, k |
| ATR-4 | IgG1, k |
| ATR-5 | IgG1, k |
| ATR-7 | IgG2a, k |
| ATR-8 | IgG2a, k |

### Reference Example 3. Cloning of DNA encoding the V region of a mouse monoclonal antibody against human TF

### (1) Preparation of mRNA

mRNA was prepared from hybridoma obtained in Reference Example 2 ATR-5 using the QuickPrep mRNA Purification Kit (Pharmacia Biotech). Each hybridoma cell was completely homogenized in the extraction buffer according to instructions attached to the kit, and then mRNA was purified by the oligo (dT)-cellulose spun column, followed by ethanol precipitation. The mRNA precipitate was dissolved in the elution buffer.

### (2) Preparation and amplification of cDNA of the gene encoding a mouse antibody V region

### (i) Cloning of H chain V region cDNA

The cloning of the gene encoding the H chain V region of a mouse monoclonal antibody against human TF was carried out using the 5'-RACE method (Frohman, M.A. et al., Proc. Natl. Acad. Sci. USA 85: 8998-9002, 1988; Belyavsky, A. et al., Nucleic Acids Res. 17: 2919-2932, 1989). For the 5'-RACE method, the Marathon cDNA Amplification Kit (CLONTECH) was used and the procedure carried out according to the instructions attached to the kit.

Using about 1 µg of mRNA prepared as above as a template, the cDNA synthesis primer attached to the kit was added, which was reacted with a reverse transcriptase at 42°C for 60 minutes to effect reverse transcription to cDNA. This was reacted with DNA polymerase I, DNA ligase, and RNaseH at 16°C for 1.5 hour, and with T4 DNA polymerase at 16°C for 45 minutes thereby to synthesize a double stranded cDNA. The double stranded cDNA was extracted with phenol and chloroform, and recovered by ethanol precipitation.

By overnight reaction with T4 DNA ligase at 16°C, a cDNA adapter was ligated to both ends of the double stranded cDNA. The reaction mixture was diluted 50-fold with a 10 mM Tricine-KOH (pH 8.5) containing 0.1 mM EDTA. Using this as a template, the gene encoding the H chain V region was amplified by PCR. The adapter primer 1 attached to the kit was used for the 5'-end primer, and for the 3'-end primer the MHC-G1 primer (SEQ ID NO: 1) (S. T. Jones, et al., Biotechnology, 9: 88-89, 1991) were used.

PCR solutions for the ATR-5 antibody H chain V region contained, in 100 µl, 120 mM Tris-HCl (pH 8.0), 10 mM KCl, 6 mM (NH₄)₂SO₄, 0.1% Triton X-100, 0.001% BSA, 0.2 mM dNTPs (dATP, dGTP, dCTP, dTTP), 1 mM MgCl₂, 2.5 units of KOD DNA polymerase (Toyo Boseki), 30-50 pmole of adapter primer 1, as well as MHC-G1 primer, and 1-5 µl of a reaction mixture of cDNA to which the cDNA adapter was ligated.

All PCRs were carried out using the DNA Thermal Cycler 480 (Perkin-Elmer), and the PCR was performed for thirty cycles at a temperature cycle of 94°C for 30 seconds, 55°C for 30 seconds, and 74°C for 1 minute.

### (ii) Cloning of L chain V region cDNA

The cloning of the gene encoding the L chain V region of a mouse monoclonal antibody against human TF was carried out using the 5'-RACE method (Frohman, M.A. et al., Proc. Natl. Acad. Sci. USA 85: 8998-9002, 1988; Belyavsky, A. et al., Nucleic Acids Res. 17: 2919-2932, 1989). For the 5'-RACE method, the Marathon cDNA Amplification Kit (CLONTECH) was used according to the instructions attached to the kit. Using about 1 µg of mRNA prepared as above as a template, the cDNA synthesis primer was added, which was reacted with a reverse transcriptase at 42°C for 60 minutes to effect reverse transcription to cDNA.

This was reacted with DNA polymerase I, DNA ligase, and RNaseH at 16°C for 1.5 hour, and with T4 DNA polymerase at 16°C for 45 minutes thereby to synthesize a double stranded cDNA. The double stranded cDNA was extracted with phenol and chloroform, and recovered by ethanol precipitation. By overnight reaction with T4 DNA ligase at 16°C, a cDNA adapter was ligated to both ends of the double stranded cDNA. The reaction mixture was diluted 50 times with a 10 mM Tricine-KOH (pH 8.5) containing 0.1 mM EDTA. Using this as a template, the gene encoding the L chain V region was amplified by PCR. The adapter primer 1 was used for the 5'-end primer, and for the 3'-end primer the MKC primer (SEQ ID NO: 2) (S. T. Jones, et al., Biotechnology, 9: 88-89, 1991) was used.

PCR solutions contained, in 100 µl, 120 mM Tris-HCl (pH 8.0), 10 mM KCl, 6 mM (NH₄)₂SO₄, 0.1% Triton X-100, 0.001% BSA, 0.2 mM dNTPs (dATP, dGTP, dCTP, dTTP), 1 mM MgCl₂, 2.5 units of KOD DNA polymerase (Toyo Boseki), 30-50 pmole of adapter primer 1, as well as MKC primer, and 1 µl of a reaction mixture of cDNA to which the cDNA adapter was ligated.

All PCRs were carried out using the DNA Thermal Cycler 480 (Perkin-Elmer), and the PCR was performed for thirty cycles at a temperature cycle of 94°C for 30 seconds, 55°C for 30 seconds, and 74°C for 1 minute.

### (3) Purification and fragmentation of PCR products

The above PCR reaction mixture was extracted with phenol and chloroform, and the amplified DNA fragments were recovered by ethanol precipitation. DNA fragments were digested with the restriction enzyme XmaI (New England Biolabs) at 37°C for 1 hour. The xmaI-digestion mixture was separated by agarose gel electrophoresis using 2%-3% NuSieve GTG agarose (FMC BioProducts), and the agarose strips containing DNA fragments about 500 bp long as the H chain V region and DNA fragments about 500 bp long as the L chain V region were excised. The agarose strips were extracted with phenol and chloroform, DNA fragments were precipitated with ethanol, which were then dissolved in 10 µl of 10 mM Tris-HCl (pH 8.0) containing 1 mM EDTA (hereinafter referred to as TE).

The XmaI-digested DNA fragments prepared as above containing a genes encoding a mouse H chain V region and L chain V region and the pUC19 plasmid vector prepared by digesting with XmaI were ligated using the DNA ligation kit ver.2 (Takara Shuzo) by reacting at 16°C for 1 hour according to the instructions attached to the kit.

The ligation mixture was added to 100 µl of E. coli JM109 competent cells (Nippongene) and was incubated for 30 minutes on ice and for 1 minute at 42°C.

Then, 300 µl of the Hi-Competence Broth (Nippongene) was added thereto, incubated at 37°C for 1 hour. Then, Escherichia coli was plated on a LB agar medium (Molecular Cloning: A Laboratory Manual, Sambrook, et al., Cold Spring Harbor Laboratory Press, 1989) containing 100 µg/ml ampicillin (hereinafter referred to as LBA agar medium), and incubated overnight at 37°C to obtain an E. coli transformant.

The transformant was cultured overnight in 3 ml or 4 ml of a LB medium containing 50 µg/ml ampicillin (hereinafter referred to as LBA medium) at 37°C, and from the cell fractions, plasmid DNA was prepared using the QIAprep Spin Plasmid Kit (QIAGEN), and then the nucleotide sequence was determined.

### (4) Determination of the nucleotide sequence of the gene encoding a mouse antibody V region

The nucleotide sequence of the cDNA coding region in the above plasmid was determined using the Dye Terminator Cycle Sequencing FS Ready Reaction Kit (Perkin-Elmer) by the DNA Sequencer 373A (Perkin-Elmer). As the sequencing primer, M13 Primer M4 (Takara Shuzo) (SEQ ID NO: 3) and M13 Primer RV (Takara Shuzo) (SEQ ID NO: 4) were used, and the sequence was determined by confirming the nucleotide sequence in both directions.

Thus obtained plasmid containing the gene encoding the mouse H chain V region derived from the hybridoma ATR-5 was designated as ATR-5Hv/pUC19, and the thus obtained plasmid containing the gene encoding a mouse L chain V region derived from the hybridoma ATR-5 was designated as ATR-5Lv/pUC19. The nucleotide sequences of the genes encoding the H chain V region of each mouse antibody contained in the plasmid ATR-5Hv/pUC19 (including the corresponding amino acid sequences) is shown in SEQ ID NO: 5 and 99, respectively, and the nucleotide sequences of the genes encoding the L chain V region of each mouse antibody contained in the plasmid ATR-5Lv/pUC19 (including the corresponding amino acid sequences) is shown in SEQ ID NO: 6 and 100, respectively.

### Reference Example 4. Construction of chimeric antibody

A chimeric ATR-5 antibody was generated in which the mouse ATR-5 antibody V region was ligated to the human antibody C region. A chimeric antibody expression vector was constructed by ligating the gene encoding the ATR-5 antibody V region to an expression vector encoding the human antibody C region.

### (1) Construction of a chimeric antibody H chain V region

The ATR-5 antibody H chain V region was modified by the PCR method in order to ligate it to an expression vector encoding the human antibody H chain C region. The 5'-end primer ch5HS (SEQ ID NO: 7) was designed so as to hybridize the 5'-end of DNA encoding the V region and to have the Kozak consensus sequence (Kozak, M. et al., J. Mol. Biol. 196: 947-950, 1987) and a recognition sequence of the restriction enzyme SalI. The 3'-end primer ch5HA (SEQ ID NO: 8) was designed so as to hybridize the 3'-end of DNA encoding the J region and to have a recognition sequence of the restriction enzyme NheI.

The PCR solutions contained, in 100 µl, 120 mM Tris-HCl (pH 8.0), 10 mM KCl, 6 mM (NH₄)₂SO₄, 0.1% Triton X-100, 0.001% BSA, 0.2 mM dNTPs (dATP, dGTP, dCTP, dTTP), 1 mM MgCl₂, 2.5 units of KOD DNA polymerase (Toyo Boseki), 50 pmole of the ch5HS primer and the ch5HA primer, as well as 1 µl of the plasmid ATR5Hv/pUC19 as a template DNA. For PCR, the DNA Thermal Cycler 480 (Perkin-Elmer) was used, and the PCR was performed for thirty cycles at a temperature cycle of 94°C for 30 seconds, 55°C for 30 seconds, and 74°C for 1 minute.

The PCR reaction mixture was extracted with phenol and chloroform, and the amplified DNA fragments were recovered by ethanol precipitation. The DNA fragments were digested with the restriction enzyme NheI (Takara Shuzo) at 37°C for 1 hour, and then with the restriction enzyme SalI (Takara Shuzo) at 37°C for 1 hour. The digestion mixture was separated by agarose gel electrophoresis using a 3% NuSieve GTG agarose (FMC BioProducts), and the agarose strips containing about 450 bp long DNA fragments were excised. The agarose strips were extracted with phenol and chloroform, and the DNA fragments were precipitated with ethanol, which were then dissolved in 20 µl of TE.

As the cloning vector, an altered promoter vector (hereinafter referred to as CVIDEC) was used in which the recognition sequences of the restriction enzymes NheI, salI, and SplI, BglII were introduced. The gene fragment prepared as above encoding the mouse H chain V region and the CVIDEC vector prepared by digesting with NheI and SalI were ligated using the DNA ligation kit ver.2 (Takara Shuzo) by reacting at 16°C for 1 hour according to the instructions attached to the kit.

The ligation mixture was added to 100 µl of E. coli JM109 competent cells (Nippongene) and was incubated for 30 minutes on ice and for 1 minute at 42°C. Then, 300 µl of Hi-Competence Broth (Nippongene) was added thereto, incubated at 37°C for 1 hour, and then the E. coli was plated on a 100 µg/ml LBA agar medium and incubated overnight at 37°C to obtain an E. coli transformant. The transformant was cultured overnight at 37°C in 3 ml of the LBA medium, and from the cell fractions, plasmid DNA was prepared using the QIAprep Spin Plasmid Kit (QIAGEN).

The nucleotide sequence of the cDNA coding region in the plasmid was determined using the Dye Terminator Cycle Sequencing FS Ready Reaction Kit (Perkin-Elmer) by the DNA Sequencer 373A (Perkin-Elmer). As the sequencing primer, M13 Primer M4 (Takara Shuzo) and M13 Primer RV (Takara Shuzo) were used, and the sequence was determined by confirming the nucleotide sequence in both directions. The plasmid that contains the gene encoding the ATR-5 antibody H chain V region, a SalI recognition sequence and the Kozak consensus sequence at the 5'-end, and a NheI recognition sequence at the 3'-end was designated as chATR5Hv/CVIDEC.

### (2) Construction of a chimeric antibody L chain V region

The ATR-5 antibody L chain V region was modified by the PCR method in order to ligate it to an expression vector encoding the human antibody L chain C region. The 5'-end primer ch5LS (SEQ ID NO: 9) was designed so as to hybridize to the 5'-end of the DNA encoding the V region and to have the Kozak consensus sequence (Kozak, M. et al., J. Mol. Biol. 196: 947-950, 1987) and a recognition sequence of the restriction enzyme BglII. The 3'-end primer ch5LA (SEQ ID NO: 10) was designed so as to hybridize to the 3'-end of the DNA encoding the J region and to have a recognition sequence of the restriction enzyme SplI.

The PCR solutions contained, in 100 µl, 120 mM Tris-HCl (pH 8.0), 10 mM KCl, 6 mM (NH₄)₂SO₄, 0.1% Triton X-100, 0.001% BSA, 0.2 mM dNTPs (dATP, dGTP, dCTP, dTTP), 1 mM MgCl₂, 2.5 units of KOD DNA polymerase (Toyo Boseki), 50 pmole of the ch5LS primer and the ch5LA primer, as well as 1 µl of the plasmid ATR5Lv/pUC19 as a template DNA. For PCR the DNA Thermal Cycler 480 (Perkin-Elmer) was used, and the PCR was performed for thirty cycles at a temperature cycle of 94°C for 30 seconds, 55°C for 30 seconds, and 74°C for 1 minute.

The PCR reaction mixture was extracted with phenol and chloroform, and the amplified DNA fragments were recovered by ethanol precipitation. The DNA fragments were digested with the restriction enzyme SplI (Takara Shuzo) at 37°C for 1 hour, and then with the restriction enzyme BglII (Takara Shuzo) at 37°C for 1 hour. The digestion mixture was separated by agarose gel electrophoresis using a 3% NuSieve GTG agarose (FMC BioProducts), and the agarose strips containing about 400 bp long DNA fragments were excised. The agarose strips were extracted with phenol and chloroform, the DNA fragments were precipitated with ethanol, which were then dissolved in 20 µl of TE.

The gene fragment prepared as above encoding the mouse L chain V region, and the CVIDEC vector prepared by digesting with SplI and BglII, were ligated using the DNA ligation kit ver.2 (Takara Shuzo) by reacting at 16°C for 1 hour according to the instructions attached to the kit.

The ligation mixture was added to 100 µl of E. coli JM109 competent cells (Nippongene) and was incubated for 30 minutes on ice and for 1 minute at 42°C. Then, 300 µl of Hi-Competence Broth (Nippongene) was added thereto, incubated at 37°C for 1 hour, and then the E. coli was plated on a 100 µg/ml LBA agar medium and incubated overnight at 37°C to obtain an E. coli transformant. The transformant was cultured overnight at 37°C in 3 ml of the LBA medium and, from the cell fractions, plasmid _{DNA} was prepared using the QIAprep Spin Plasmid Kit (QIAGEN).

The nucleotide sequence of the cDNA coding region in the plasmid was determined using the Dye Terminator Cycle Sequencing FS Ready Reaction Kit (Perkin-Elmer) by the DNA Sequencer 373A (Perkin-Elmer). As the sequencing primer, M13 Primer M4 (Takara Shuzo) and M13 Primer RV (Takara Shuzo) were used, and the sequence was determined by confirming the nucleotide sequence in both directions. The plasmid that contains the gene encoding the ATR-5 antibody L chain V region and that has a BglII recognition sequence and the Kozak consensus sequence at the 5'-end and a SplI recognition sequence at the 3'-end was designated as chATR5Lv/CVIDEC.

### (3) Construction of a chimeric antibody expression vector

A chimeric antibody expression vector was constructed using an antibody expression vector introduced from IDEC Pharmaceuticals. As the vector, the IgGl-type antibody expression vector N5KG1(V) and the IgG4-type antibody expression vector N5KG4P were used. The chimeric ATR-5 antibody expression vector was generated by ligating a gene encoding the H chain V region of ATR-5 to the SalI-NheI site located immediately before the human antibody H chain C region of the expression vector N5KG1(V) or N5KG4P and ligating a gene encoding the L chain V region of ATR-5 to the BglII-SplI site located immediately before the human antibody L chain C region of the expression vector N5KG1(V) or N5KG4P.

### (i) Introduction of H chain V region

The plasmid chATR5Hv/CVIDEC was digested with the restriction enzyme NheI (Takara Shuzo) at 37°C for 3 hours, and with the restriction enzyme SalI (Takara Shuzo) at 37°C for 3 hours. The digestion mixture was separated by agarose gel electrophoresis using 1.5% NuSieve GTG agarose (FMC BioProducts), and the agarose strips containing about 450 bp long DNA fragments were excised. The agarose strips were extracted with phenol and chloroform, and the DNA fragments were precipitated with ethanol, which were then dissolved in 20 µl of TE.

The expression vector N5KG1(V) and N5KG4P were digested with the restriction enzyme NheI (Takara Shuzo) at 37°C for 3 hours, and with the restriction enzyme SalI (Takara Shuzo) at 37°C for 3 hours. The digestion mixture was separated by agarose gel electrophoresis using 1.5% NuSieve GTG agarose (FMC BioProducts), and the agarose strips containing about 9000 bp long DNA fragments were excised. The agarose strips were extracted with phenol and chloroform, and the DNA fragments were precipitated with ethanol, which were then dissolved in 60 µl of TE.

The SalI-NheI DNA fragment prepared as above containing the gene encoding the H chain V region and N5KG1(V) or N5KG4P digested with SalI and NheI were ligated using the DNA ligation kit ver.2 (Takara Shuzo) by reacting at 16°C for 1 hour according to the attached instructions.

The ligation mixture was added to 100 µl of E. coli JM109 competent cells (Nippongene) and was incubated for 30 minutes on ice and for 1 minute at 42°C. Then, 300 µl of Hi-Competence Broth (Nippongene) was added thereto, incubated at 37°C for 1 hour, and then the E. coli was plated on a 100 µg/ml LBA agar medium and incubated overnight at 37°C to obtain an E. coli transformant. The transformant was cultured overnight at 37°C in 3 ml of the LBA medium, and from the cell fractions, plasmid DNA was prepared using the QIAprep Spin Plasmid Kit (QIAGEN). These plasmids containing the genes encoding the chimeric ATR-5 antibody H chain were designated as chATR5Hv/N5KG1(V) and chATR5Hv/N5KG4P, respectively.

### (ii) Introduction of the L chain V region

The plasmid chATR5Lv/CVIDEC was digested with the restriction enzymes BglII (Takara Shuzo) and SplI (Takara Shuzo) at 37°C for 1.5 hour. The digestion mixture was separated by agarose gel electrophoresis using 1.5% NuSieve GTG agarose (FMC BioProducts), and the agarose strips containing about 400 bp long DNA fragments were excised. The agarose strips were extracted with phenol and chloroform, and the DNA fragments were precipitated with ethanol, which were then dissolved in 20 µl of TE.

The plasmids chATR5Hv/N5KG1(V) and chATR5Hv/N5KG4P were digested with the restriction enzymes BglII (Takara Shuzo) and SplI (Takara Shuzo) at 37°C for 1.5 hour. The digestion mixture was separated by agarose gel electrophoresis using 1.5% NuSieve GTG agarose (FMC Bioproducts), and the agarose strips containing about 9400 bp long DNA fragments were excised. The agarose strips were extracted with phenol and chloroform, DNA fragments were precipitated with ethanol, which were then dissolved in 20 µl of TE.

The SplI-BglII DNA fragment prepared as above containing the gene encoding the L chain V region and chATR5Hv/N5KG1(V) or chATR5Hv/N5KG4P digested with SplI and BglII were ligated using the DNA ligation kit ver.2 (Takara Shuzo) by reacting at 16°C for 1 hour according to the attached instructions.

The ligation mixture was added to 100 µl of E. coli JM109 competent cells (Nippongene) and was incubated for 30 minutes on ice and for 1 minute at 42°C. Then, 300 µl of Hi-Competence Broth (Nippongene) was added thereto, incubated at 37°C for 1 hour, and then the E. coli was plated on a 100 µg/ml LBA agar medium and incubated overnight at 37°C to obtain an E. coli transformant. The transformant was cultured overnight at 37°C in 1 liter of the 2xYT medium containing 50 µg/ml ampicillin, and from the cell fractions, plasmid DNA was prepared using the Plasmid Maxi Kit (QIAGEN). These plasmids containing the gene encoding the chimeric ATR-5 antibody were designated as chATR5/N5KG1(V) and chATR5/N5KG4P, respectively.

### (4) Transfection into COS-7 cells

In order to evaluate the activity of binding to the antigen and the neutralizing activity of chimeric antibody, the above expression plasmid was transfected to COS-7 cells and the antibody was transiently expressed.

The plasmid chATR5/N5KG1(V) or chATR5/N5KG4P was transduced into COS-7 cells by electroporation using a Gene Pulser instrument (Bio Rad). Fifty µg of the plasmid was added to 0.78 ml of the COS-7 cells suspended in the Dulbecco PBS (-) (hereinafter referred to as PBS) at a cell concentration of 1 × 10⁷ cells/ml, which was subjected to pulses of 1,500 V and 25 µF capacity.

After 10 minutes of the recovery period at room temperature, the electroporated cells were suspended in a DMEM medium containing 5% Ultra low IgG fetal bovine serum (GIBCO), and cultured using a 10 cm culture dish in a 5% CO₂ incubator. After culturing for 24 hours, the culture supernatant was aspirated off and, then, a serum-free medium HBCHO (Irvine Scientific) was added. After further culturing for 72 hours, the culture supernatant was collected and centrifuged to remove cell debris.

### (5) Purification of antibody

From the culture supernatant of the COS-7 cells, chimeric antibody was purified using rProtein A Sepharose Fast Flow (Pharmacia Biotech) as follows.

One ml of rProtein A Sepharose Fast Flow was filled into a column and the column was equilibrated by 10 volumes of TBS. The culture supernatant of COS-7 cells was applied to the equilibrated column, which was then washed with 10 volumes of TBS.

The adsorbed antibody fraction was then eluted by 13.5 ml of 2.5 mM HCl (pH 3.0), and the eluate was immediately neutralized by adding 1.5 ml of 1 M Tris-HCl (pH 8.0).

By performing ultrafiltration twice for the purified antibody fraction using the Centriprep 100 (Amicon), the solvent was replaced with 50 mM Tris-HCl (pH 7.6) containing 150 mM NaCl (hereinafter referred to as TBS), and was finally concentrated to about 1.5 ml.

### (6) Establishment of a stably-producing CHO cell line

In order to establish a cell line that stably produces chimeric antibody, the above expression plasmid was introduced into CHO cells (DG44) acclimated to the CHO-S-SFMII serum-free medium (GIBCO).

The plasmid chATR5/N5KG1(V) or chATR5/N5KG4P was cleaved with the restriction enzyme SspI (Takara Shuzo) to linearize DNA, and after extraction with phenol and chloroform, DNA was recovered by ethanol precipitation. The linearized plasmid was transduced into the DG44 cells by electroporation using a Gene Pulser instrument (Bio Rad). Ten µg of the plasmid was added to 0.78 ml of DG44 cells suspended in PBS at a cell concentration of 1 × 10⁷ cells/ml, which was subjected to pulses of 1,500 V and 25 µF capacity.

After a 10 minute recovery period at room temperature, the electroporated cells were suspended in a CHO-S-SFMII medium (GIBCO) containing hypoxanthine/thymidine (GIBCO), and cultured using two 96-well plates (Falcon) in a 5% CO₂ incubator. On the day after the start of culturing, the medium was changed to a selection medium containing the CHO-S-SFMII medium (GIBCO) containing hypoxanthine/thymidine (GIBCO) and 500 µg/ml GENETICIN (G418Sulfate, GIBCO) to select cells into which the antibody gene had been introduced. About two weeks after changing the selection medium, the cells were examined under a microscope. After favorable cell growth was observed, the amount of antibody produced was measured by the ELISA described below for determining antibody concentration, and cells having a high production yield of antibody were selected.

### Reference Example 5. Construction of humanized antibody

### (1) Construction of humanized antibody H chain

### (i) Construction of the humanized H chain version "a"

Humanized ATR-5 antibody H chain was generated using CDR-grafting by the PCR method. In order to generate the humanized antibody H chain version "a" having the FRs derived from human antibody L39130 (DDBJ, Gao L. et al., unpublished, 1995), seven PCR primers were used. The CDR-grafting primers hR5Hv1S (SEQ ID NO: 11), hR5Hv2S (SEQ ID NO: 12), and hR5Hv4S (SEQ ID NO: 13) have a sense DNA sequence, and the CDR grafting primers hR5Hv3A (SEQ ID NO: 14) and hR5Hv5A (SEQ ID NO: 15) have an antisense DNA sequence, each primer having a 18-35 bp complementary sequence on both ends thereof.

hR5Hv1S was designed to have the Kozak consensus sequence (Kozak, M. et al., J. Mol. Biol. 196: 947-950, 1987) and a SalI recognition site, and hR5Hv5A was designed to have a NheI recognition site. The exogenous primer hR5HvPrS (SEQ ID NO: 16) has a homology with the CDR-grafting primer hR5Hv1S, and hR5HvPrA (SEQ ID NO: 17) has a homology with the CDR-grafting primer hR5Hv5A.

The CDR-grafting primers hR5Hv1S, hR5Hv2S, hR5Hv3A, hR5Hv4S, and hR5Hv5A, and exogenous primers hR5HvPrS and hR5HvPrA were synthesized and purified by Pharmacia Biotech.

PCR was performed using the KOD DNA polymerase (Toyo Boseki) and using the attached buffer under the condition of containing 120 mM Tris-HCl (pH 8.0), 10 mM KCl, 6 mM (NH₄)₂SO₄, 0.1% Triton X100, 0.001% BSA, 0.2 mM dNTPs (dATP, dGTP, dCTP, dTTP), 1 mM MgCl₂, 2.5 units of KOD DNA polymerase (Toyo Boseki), and 5 pmole each of the CDR-grafting primers hR5Hv1S, hR5Hv2S, hR5Hv3A, hR5Hv4S, and hR5Hv5A in 98 µl, for 5 cycles at a temperature cycle of 94°C for 30 seconds, 50°C for 1 minute, and 72°C for 1 minute. After further addition of 100 pmole of exogenous primers hR5HvPrS and hR5HvPrA, PCR was performed for 25 cycles in a system of 100 µl with the same temperature cycle. DNA fragments amplified by the PCR method were separated by agarose gel electrophoresis using a 2% NuSieve GTG agarose (FMC BioProducts).

The agarose strips containing about 430 bp long DNA fragments were excised, to which 3 volumes (ml/g) of TE was added, and then were extracted with phenol, phenol/chloroform, and chloroform to purify the DNA fragments. After precipitating the purified DNA with ethanol, one third the volume thereof was dissolved in 17 µl of water. The PCR reaction mixture obtained was digested with NheI and SalI, and was ligated to the plasmid vector CVIDEC prepared by digesting with NheI and SalI, using the DNA ligation kit ver.2 (Takara Shuzo) according to the instructions attached to the kit.

The ligation mixture was added to 100 µl of E. coli JM109 competent cells (Nippongene) and was incubated for 30 minutes on ice and for 1 minute at 42°C. Then, 300 µl of the Hi-Competence Broth (Nippongene) was added thereto, incubated at 37°C for 1 hour, and then the E. coli was plated on a 100 µg/ml LBA agar medium and incubated overnight at 37°C to obtain an E. coli transformant. The transformant was cultured overnight at 37°C in 3 ml of the LBA medium, and from the cell fractions, plasmid DNA was prepared using the QIAprep Spin Plasmid Kit (QIAGEN).

The nucleotide sequence of the cDNA coding region in the plasmid was determined using the Dye Terminator Cycle Sequencing FS Ready Reaction Kit (Perkin-Elmer) by the DNA Sequencer 373A (Perkin-Elmer). As the sequencing primer, M13 Primer M4 (Takara Shuzo) and M13 Primer RV (Takara Shuzo) were used, and the sequence was determined by confirming the nucleotide sequence in both directions.

Since mutation and/or deletion were observed before or after the EcoT221 recognition site, each of fragments having the correct sequence was ligated and then subcloned again to CVIDEC to determine the nucleotide sequence. The plasmid having the correct sequence was designated as hATR5Hva/CVIDEC. The nucleotide sequence and the corresponding amino acid sequence of the humanized H chain version "a" contained in the plasmid hATR5Hva/CVIDEC are shown in SEQ ID NO: 18. The amino acid sequence of version "a" is also shown in SEQ ID NO: 19.

### (ii) Construction of humanized H chain versions "b" and "c"

versions "b" and "c" were generated by replacing the FR3 of version "a" with the FR3 derived from another human antibody using the FR-shuffling method. In order to replace the FR3 in version "b" with one derived from human antibody Z34963 (DDBJ, Borretzen M. et al., Proc. Natl. Acad. Sci. USA, 91: 12917-12921, 1994), the four DNA primers encoding the FR3 were generated. The FR-shuffling primers F3RFFS (SEQ ID NO: 20) and F3RFBS (SEQ ID NO: 21) have a sense DNA sequence and F3RFFA (SEQ ID NO: 22) and F3RFBA (SEQ ID NO: 23) have an antisense DNA sequence. F3RFFS and F3RFFA have a sequence complementary to each other, and have BalI and XhoI recognition sequences on both ends. F3RFBS and F3RFBA have a sequence complementary to each other, and have XhoI and NcoI recognition sequences on both ends.

In order to replace the FR3 in version "c" with one derived from human antibody P01825 (SWISS-PROT, Poljak RJ. et al., Biochemistry, 16: 3412-3420, 1977), four DNA primers encoding the FR3 were generated. The FR-shuffling primers F3NMFS (SEQ ID NO: 24) and F3NMBS (SEQ ID NO: 25) have a sense DNA sequence and F3NMFA (SEQ ID NO: 26) and F3NMBA (SEQ ID NO: 27) have an antisense DNA sequence. F3RFBS and F3RFBA have a sequence complementary to each other, and have BalI and XhoI recognition sequences on both ends.

F3RFFS, F3RFBS, F3RFFA, F3RFBA, F3NMFS, F3NMBS, F3NMFA, and F3NMBA were synthesized by Pharmacia Biotech. F3RFFS and F3RFFA, and F3RFBS and F3RFBA were annealed, and were digested with BalI and XhoI, and NcoI and XhoI, respectively. They were introduced to the plasmid hATR5Hva/CVIDEC (BalI/NcoI) prepared by digesting with BalI and NcoI, and the nucleotide sequence was determined. The plasmid having the correct sequence was designated as hATR5Hvb/CVIDEC. The nucleotide sequence and the corresponding amino acid sequence of the humanized H chain version "b" contained in the plasmid hATR5Hvb/CVIDEC are shown in SEQ ID NO: 28. The amino acid sequence of version "b" is also shown in SEQ ID NO: 29.

F3NMFS and F3NMFA, and F3NMBS and F3NMBA were annealed, and were digested with BalI and XhoI, and NcoI and XhoI, respectively. They were introduced to the plasmid hATR5Hva/CVIDEC (BalI/NcoI) prepared by digesting with BalI and NcoI, and the nucleotide sequence was determined. The plasmid having the correct sequence was designated as hATR5Hvc/CVIDEC. The nucleotide sequence and the corresponding amino acid sequence of the humanized H chain version "c" contained in the plasmid hATR5Hvc/CVIDEC are shown in SEQ ID NO: 30. The amino acid sequence of version "c" is also shown in SEQ ID NO: 31.

### (iii) Construction of humanized H chain versions "d" and "e"

Versions "d" and "e" were generated by replacing the FR3 of version "a" with the FR3 derived from another human antibody using the FR-shuffling method. In order to replace the FR3 in version "d" with one derived from human antibody M62723 (DDBJ, Pascual V. et al., J. Clin. Invest., 86: 1320-1328, 1990), four DNA primers encoding the FR3 were generated. The FR-shuffling primer F3EPS (SEQ ID NO: 32) has a sense DNA sequence and F3EPA (SEQ ID NO: 33) has an antisense DNA sequence, and the 3'-end of the primers has a complementary sequence of 18 bp.

Exogenous primers F3PrS (SEQ ID NO: 34) and F3PrA (SEQ ID NO: 35) have a homology with the FR-shuffling primers F3EPS and F3EPA, and can also be used for other FR3 FR-shuffling. In order to replace the FR3 in version "e" with one derived from the human antibody Z80844 (DDBJ, Thomsett AR. et al., unpublished), two DNA primers encoding the FR3 were generated. The FR-shuffling primers F3VHS (SEQ ID NO: 36) has a sense DNA sequence and F3VHA (SEQ ID NO: 37) has an antisense DNA sequence, and the 3'-end of the primers has a complementary sequence of 18 bp. F3EPS, F3EPA, F3PrS, F3PrA, F3VHS and F3VHA were synthesized by Pharmacia Biotech.

PCR was performed using the KOD DNA polymerase (Toyo Boseki) using the attached buffer under the condition of containing 5 µl each of 1 µM FR-shuffling primers F3EPS and F3EPA, or F3VHS and F3VHA, 0.2 mM dNTPs, 1.0 mM MgCl₂, and 2.5 units of KOD DNA polymerase in 100 µl of the reaction mixture, for 5 cycles at a temperature cycle of 94°C for 30 seconds, 50°C for 1 minute, and 74°C for 1 minute. After further addition of 100 pmole of exogenous primers F3PrS and F3PrA, PCR was performed for 25 cycles with the same temperature cycle.

DNA fragments amplified by the PCR method were separated by agarose gel electrophoresis using a 2% Nu Sieve GTG agarose (FMC BioProducts). The agarose strips containing about 424 bp long DNA fragments were excised, to which 3 volumes (ml/g) of TE was added, and then were extracted with phenol, phenol/chloroform, and chloroform to purify the DNA fragments. After precipitating the purified DNA with ethanol, one third the volume thereof was dissolved in 14 µl of water. The PCR reaction mixture obtained was digested with BalI and NcoI, and was introduced to the plasmid hATR5Hva/CVIDEC (BalI/NcoI) prepared by digesting with BalI and NcoI, and the nucleotide sequence was determined.

The plasmids having the correct sequence were designated as hATR5Hvd/CVIDEC and hATR5Hve/CVIDEC. The nucleotide sequence and the corresponding amino acid sequence of the humanized H chain version "d" contained in the plasmid hATR5Hvd/CVIDEC are shown in SEQ ID NO: 38, and the amino acid sequence of version "d" is also shown in SEQ ID NO: 39. The nucleotide sequence and the corresponding amino acid sequence of the humanized H chain version "e" contained in the plasmid hATR5Hve/CVIDEC are shown in SEQ ID NO: 40, and the amino acid sequence of version "e" is also shown in SEQ ID NO: 41.

### (iv) Construction of humanized H chain versions "f" and "g"

Versions "f" and "g" were generated by replacing the FR3 of version "a" with the FR3 derived from another human antibody using the FR-shuffling method. In order to replace the FR3 in version "f" with one derived from human antibody L04345 (DDBJ, Hillson JL. et al., J. Exp. Med., 178: 331-336, 1993) and to replace the FR3 in version "g" with one derived from human antibody S78322 (DDBJ, Bejcek BE. et al., Cancer Res., 55: 2346-2351, 1995), two primers each encoding the FR3 were synthesized. The FR-shuffling primer F3SSS (SEQ ID NO: 42) of version "f" has a sense DNA sequence and F3SSA (SEQ ID NO: 43) has an antisense DNA sequence, and the 3'-end of the primers has a complementary sequence of 18 bp.

F3CDS (SEQ ID NO: 44) of version "g" has a sense DNA sequence and F3CDA (SEQ ID NO: 45) has an antisense DNA sequence, and the 3'-end of the primers has a complementary sequence of 18 bp. F3SSS, F3SSA, F3CDS, and F3CDA were synthesized and purified by Pharmacia Biotech. PCR was performed using the KOD DNA polymerase (Toyo Boseki) using the attached buffer under the condition of containing 5 µl each of 1 µM FR-shuffling primers F3SSS and F3SSA, or F3CDS and F3CDA, 0.2 mM dNTPs, 1.0 mM MgCl₂, and 2.5 units of KOD DNA polymerase in 100 µl of the reaction mixture, for 5 cycles at a temperature cycle of 94°C for 30 seconds, 50°C for 1 minute, and 74°C for 1 minute. After further addition of 100 pmole of exogenous primers F3PrS and F3PrA, PCR was performed for 25 cycles with the same temperature cycle.

DNA fragments amplified by the PCR method were separated by agarose gel electrophoresis using a 2% NuSieve GTG agarose (FMC BioProducts). The agarose strips containing about 424 bp long DNA fragments were excised, to which 3 volumes (ml/g) of TE was added, and then were extracted with phenol, phenol/chloroform, and chloroform to purify the DNA fragments. After precipitating the purified DNA with ethanol, one third the volume thereof was dissolved in 14 µl of water. The PCR reaction mixture obtained was digested with BalI and NcoI, and was introduced to the plasmid hATR5Hva/CVIDEC (BalI/NcoI) prepared by digesting with BalI and NcoI, and the nucleotide sequence was determined.

The plasmids having the correct sequence were designated as hATR5Hvf/CVIDEC and hATR5Hvg/CVIDEC. The nucleotide sequence and the corresponding amino acid sequence of the humanized H chain version "f" contained in the plasmid hATR5Hvf/CVIDEC, and the amino acid sequence of version "f" are shown in SEQ ID NO: 46 and 47. The nucleotide sequence and the corresponding amino acid sequence of the humanized H chain version "g" contained in the plasmid hATR5Hvg/CVIDEC, and the amino acid sequence of version "g" are shown in SEQ ID NO: 48 and 49.

### (v) Construction of the humanized H chain version "h"

Version "h" was generated by replacing the FR3 of version "a" with the FR3 derived from another human antibody using the FR-shuffling method. In order to replace the FR3 in version "h" with one derived from the human antibody Z26827 (DDBJ, van Der Stoep et al., J. Exp. Med., 177: 99-107, 1993), two primers each encoding the FR3 were synthesized. The FR-shuffling primer F3ADS (SEQ ID NO: 50) of version "h" has a sense DNA sequence and F3ADA (SEQ ID NO: 51) has an antisense DNA sequence, and the 3'-end of the primers has a complementary sequence of 18 bp.

F3ADS and F3ADA were synthesized and purified by Pharmacia Biotech. PCR was performed using the KOD DNA polymerase (Toyo Boseki) using the attached buffer under the condition of containing 5 µl each of 1 µM FR-shuffling primers F3ADS and F3ADA, 0.2 mM dNTPs, 1.0 mM MgCl₂, and 2.5 units of KOD DNA polymerase in 100 µl of the reaction mixture, for 5 cycles at a temperature cycle of 94°C for 30 seconds, 50°C for 1 minute, and 74C for 1 minute. After further addition of 100 pmole of exogenous primers F3PrS and F3PrA, PCR was performed for 25 cycles with the same temperature cycle. DNA fragments amplified by the PCR method were separated by agarose gel electrophoresis using a 2% NuSieve GTG agarose (FMC BioProducts).

The agarose strips containing about 424 bp long DNA fragments were excised, to which 3 volumes (ml/g) of TE was added, and then were extracted with phenol, phenol/chloroform, and chloroform to purify the DNA fragments. After precipitating the purified DNA with ethanol, one third the volume thereof was dissolved in 14 µl of water. The PCR reaction mixture obtained was digested with BalI and NcoI, and was introduced to the plasmid hATR5Hva/CVIDEC (BalI/NcoI) prepared by digesting with BalI and NcoI, and the nucleotide sequence was determined. The plasmids having the correct sequence were designated as hATR5Hvh/CVIDEC. The nucleotide sequence and the corresponding amino acid sequence of the humanized H chain version "h" contained in the plasmid hATR5Hvh/CVIDEC, and the amino acid sequence of version "h" are shown in SEQ ID NO: 52. The amino acid sequence of version "h" is shown in SEQ ID NO: 53.

### (vi) Construction of humanized H chain versions "i" and "j"

Versions "i" and "j" were generated by replacing the FR3 of version "a" with the FR3 derived from another human antibody using the FR-shuffling method. In order to replace the FR3 in version "i" with one derived from the human antibody U95239 (DDBJ, Manheimer-Lory AAJ., unpublished) and to replace the FR3 in version "j" with one derived from the human antibody L03147 (DDBJ, Collect TA. et al., Proc. Natl. Acad. Sci. USA, 89: 10026-10030, 1992), two primers each encoding the FR3 were synthesized. The FR-shuffling primer F3MMS (SEQ ID NO: 54) of version "i" has a sense DNA sequence and F3MMA (SEQ ID NO: 55) has an antisense DNA sequence, and the 3'-end of the primers has a complementary sequence of 18 bp.

F3BMS (SEQ ID NO: 56) of version "j" has a sense DNA sequence and F3BMA (SEQ ID NO: 57) has an antisense DNA sequence, and the 3'-end of the primers has a complementary sequence of 18 bp. F3MMS, F3MMA, F3BMS, and F3BMA were synthesized and purified by Pharmacia Biotech. PCR was performed using the Ampli Taq Gold (Perkin-Elmer) using the attached buffer under the condition of containing 5 µl each of 1 µM FR-shuffling primers F3MMS and F3MMA, or F3BMS and F3BMA, 0.2 mM dNTPs, 1.0 mM MgCl₂, and 2.5 units of Ampli Taq Gold in 100 µl of the reaction mixture, for 5 cycles at a temperature cycle of 94°C for 30 seconds, 50°C for 1 minute, and 74°C for 1 minute. After further addition of 100 pmole of exogenous primers F3PrS and F3PrA, PCR was performed for 25 cycles with the same temperature cycle.

DNA fragments amplified by the PCR method were separated by agarose gel electrophoresis using a 2% Nu Sieve GTG agarose (FMC BioProducts). The agarose strips containing about 424 bp long DNA fragments were excised, to which 3 volumes (ml/g) of TE was added, and then were extracted with phenol, phenol/chloroform, and chloroform to purify the DNA fragments. After precipitating the purified DNA with ethanol, one third the volume thereof was dissolved in 14 µl of water. The PCR reaction mixture obtained was digested with BalI and NcoI, and was introduced to the plasmid hATR5Hva/CVIDEC (BalI/NcoI) prepared by digesting with BalI and NcoI, and the nucleotide sequence was determined.

The plasmids having the correct sequence were designated as hATR5Hvi/CVIDEC and hATR5Hvj/CVIDEC. The nucleotide sequence and the corresponding amino acid sequence of the humanized H chain version "i" contained in the plasmid hATR5Hvi/CVIDEC, and the amino acid sequence of version "i" are shown in SEQ ID NO: 58 and 59. The nucleotide sequence and the corresponding amino acid sequence of the humanized H chain version "j" contained in the plasmid hATR5Hvj/CVIDEC, and the amino acid sequence of version "j" are shown in SEQ ID NO: 60 and 61.

### (vii) Construction of humanized H chain versions "b1" and "d1"

Versions "b1" and "d1" were generated by replacing the FR2 of versions "b" and "d" with the FR2 derived from another human antibody using the FR-shuffling method. In order to replace the FR2 with one derived from the human antibody P01742 (SWISS-PROT, Cunningham BA. et al., Biochemistry, 9: 3161-3170, 1970), two DNA primers encoding the FR2 were synthesized. The FR-shuffling vector F2MPS (SEQ ID NO: 62) has a sense DNA sequence and F2MPA (SEQ ID NO: 63) has an antisense DNA sequence. They also have a sequence complementary to each other, and have recognition sequences of EcoT221 and BalI on both ends thereof.

F2MPS and F2MPA were synthesized and purified by Pharmacia Biotech. F2MPS and F2MPA were annealed and were digested with EcoT22I and BalI. They were introduced to plasmids hATR5Hvb/CVIDEC (EcoT22I/BalI) and hATR5Hvd/CVIDEC (EcoT22I/BalI) prepared by digesting with EcoT22I and BalI, and the nucleotide sequence was determined. The plasmids having the correct sequence were designated as hATR5Hvb1/CVIDEC and hATR5Hvd1/CVIDEC. The nucleotide sequence and the corresponding amino acid sequence of the humanized H chain version "b1" contained in the plasmid hATR5Hvb1/CVIDEC, and the amino acid sequence of version "b1" are shown in SEQ ID NO: 64 and 65. The nucleotide sequence and the corresponding amino acid sequence of the humanized H chain version "d1" contained in the plasmid hATR5Hvd1/CVIDEC, and the amino acid sequence of version "d1" are shown in SEQ ID NO: 66 and 67.

### (viii) Construction of humanized H chain versions "b3" and "d3"

Versions "b3" and "d3" were generated by replacing the FR2 of versions "b" and "d" with the FR2 derived from another human antibody using the FR-shuffling method. In order to replace the FR2 with one derived from the human antibody z80844 (DDDJ, Thomsett AR. et al., unpublished), two DNA primers encoding the FR2 were synthesized. The FR-shuffling vector F2VHS (SEQ ID NO: 68) has a sense DNA sequence and F2VHA (SEQ ID NO: 69) has an antisense DNA sequence. They also have a sequence complementary to each other, and have recognition sequences of EcoT221 and Ball on both ends thereof. The synthesis and purification of F2VHS and F2VHA was referred to Pharmacia Biotech.

F2VHS and F2VHA were annealed and were digested with EcoT22I and BalI. They were introduced to plasmids hATR5Hvb/CVIDEC (EcoT22I/BalI) and hATR5Hvd/CVIDEC (EcoT22I/BalI) prepared by digesting with EcoT22I and BalI, and the nucleotide sequence was determined. The plasmids having the correct sequence were designated as hATR5Hvb3/CVIDEC and hATR5Hvd3/CVIDEC. The nucleotide sequence and the corresponding amino acid sequence of the humanized H chain version "b3" contained in the plasmid hATR5Hvb3/CVIDEC, and the amino acid sequence of version "b3" are shown in SEQ ID NO: 70 and 71. The nucleotide sequence and the corresponding amino acid sequence of the humanized H chain version "d3" contained in the plasmid hATR5Hvd3/CVIDEC, and the amino acid sequence of version "d3" are shown in SEQ ID NO: 72 and 73.

### (2) Construction of a humanized antibody L chain V region

### (i) version "a"

The humanized ATR-5 antibody L chain V region was generated by the CDR-grafting using the PCR method. For the generation of a humanized antibody L chain (version "a") having framework regions derived from human antibody Z37332 (DDBJ, Welschof M. et al., J. Immunol. Methods, 179: 203-214, 1995), seven PCR primers were used.

CDR-grafting primers h5Lv1S (SEQ ID NO: 74) and h5Lv4S (SEQ ID NO: 75) have a sense DNA sequence, CDR-grafting primers h5Lv2A (SEQ ID NO: 76), h5Lv3A (SEQ ID NO: 77), and h5Lv5A (SEQ ID NO: 78) have an antisense DNA sequence, and each primer has 20 bp complementary sequences on both ends thereof. Exogenous primers h5LvS (SEQ ID NO: 79) and h5LvA (SEQ ID NO: 80) have a homology with CDR-grafting primers h5Lv1S and h5Lv5A. The synthesis and purification of CDR-grafting primers h5Lv1S, h5Lv4S, h5Lv2A, h5Lv3A, h5Lv5A, h5LvS, and h5LvA were referred to Pharmacia Biotech.

The PCR solutions contain, in 100 µl, 120 mM Tris-HCl (pH 8.0), 10 mM KCl, 6 mM (NH₄)₂SO₄, 0.1% Triton X-100, 0.001% BSA, 0.2 mM dNTPs (dATP, dGTP, dCTP, dTTP), 1 mM MgCl₂, 2.5 units of KOD DNA polymerase (Toyo Boseki), 50 pmole of the CDR-grafting primers h5Lv1S, h5Lv2A, h5Lv3A, h5Lv4S, and h5Lv5A.

PCR was performed using the DNA Thermal Cycler 480 (Perkin-Elmer) for 5 cycles with the temperature cycle of 94°C for 30 seconds, 50°C for 1 minute, and 72°C for 1 minute to assemble 5 CDR-grafting primers. After further addition of 100 pmole of exogenous primers h5LvS and h5LvA to the reaction mixture, PCR was performed for 30 cycles with the temperature cycle of 94°C for 30 seconds, 52°C for 1 minute, and 72°C for 1 minute to amplify the assembled DNA fragments.

The PCR reaction mixture was separated by agarose gel electrophoresis using a 3% NuSieve GTG agarose (FMC Bioproducts), and the agarose strips containing about 400 bp long DNA fragments were excised. The agarose strips were extracted with phenol and chloroform, DNA fragments were recovered by ethanol precipitation. The recovered DNA fragments were digested with the restriction enzymes SplI (Takara Shuzo) and BglII (Takara Shuzo) at 37°C for 4 hours. The digestion mixture was extracted with phenol and chloroform, and after the ethanol precipitation of the DNA fragments, they were dissolved in 10 µl of TE. The SplI-BgIII DNA fragment prepared as above encoding the humanized L chain V region and the CVIDEC vector prepared by digesting with SplI and BglII were ligated using the DNA ligation kit ver.2 (Takara Shuzo) by reacting at 16°C for 1 hour according to the instructions attached to the kit.

The ligation mixture was added to 100 µl of E. coli JM109 competent cells (Nippongene) and was incubated for 30 minutes on ice and for 1 minute at 42°C. Then, 300 µl of Hi-Competence Broth (Nippongene) was added thereto, incubated at 37°C for 1 hour, and then the E. coli was plated on a 100 µg/ml LBA agar medium and incubated overnight at 37°C to obtain an E. coli transformant. The transformant was cultured overnight in 3 ml of the LBA medium, and from the cell fractions, plasmid DNA was prepared using the QIAprep Spin Plasmid Kit (QIAGEN).

The nucleotide sequence of the cDNA coding region in the plasmid was determined using the Dye Terminator Cycle Sequencing FS Ready Reaction Kit (Perkin-Elmer) by the DNA Sequencer 373A (Perkin-Elmer). As the sequencing primer, M13 Primer M4 (Takara Shuzo) and M13 Primer RV (Takara Shuzo) were used, and the sequence was determined by confirming the nucleotide sequence in both directions. The plasmid that contains the gene encoding the humanized antibody L chain V region and that has a BglII recognition sequence and the Kozak sequence at the 5'-end, and a SplI recognition sequence at the 3'-end was designated as hATR5Lva/CVIDEC. The nucleotide sequence (including the corresponding amino acid sequence) of the humanized L chain version "a" is shown in SEQ ID NO: 81. The amino acid sequence of version "a" is also shown in SEQ ID NO: 82.

### (ii) Versions "b" and "c"

Versions "b" and "c" were generated by replacing (FR-shuffling) the FR3 of version "a". For version "b" the FR3 derived from human antibody S68699 (DDBJ, Hougs L. et al., Exp. Clin. Immunogen et., 10: 141-151, 1993) was used, and for version "c" the FR3 derived from human antibody P01607 (SWISS-PROT, Epp O et al., Biochemistry, 14: 4943-4952, 1975) was used, respectively.

Primers F3SS (SEQ ID NO: 83) and F3SA (SEQ ID NO: 84) encoding the FR3 of version "b", or primers F3RS (SEQ ID NO: 85) and F3RA (SEQ ID NO: 86) encoding the FR3 of version "c" have a sequence complementary to each other, and have the recognition sequences of the restriction enzymes KpnI and PstI on both ends thereof. The synthesis and purification of F3SS, F3SA, F3RS, and F3RA were referred to Pharmacia Biotech. 100 pmole each of F3SS and F3SA, or F3RS and F3RA were annealed by treating at 96°C for 2 minutes and at 50°C for 2 minutes and the double stranded DNA fragments were generated.

These double stranded DNA fragments were digested with the restriction enzyme KpnI (Takara Shuzo) at 37°C for 1 hour, and then with the restriction enzyme PstI (Takara Shuzo) at 37°C for 1 hour. The digestion mixture was extracted with phenol and chloroform and, after it was precipitated with ethanol, it was dissolved in TE.

The plasmid hATR5Lva/CVIDEC was digested with the restriction enzyme KpnI (Takara Shuzo) at 37°C for 1 hour, and then with the restriction enzyme PstI (Takara Shuzo) at 37°C for 1 hour. The digestion mixture was separated by agarose gel electrophoresis using a 1.5% NuSieve GTG agarose (FMC BioProducts), and the agarose strips having about 3000 bp long DNA fragments were excised. The agarose strip was extracted with phenol and chloroform, and after the DNA fragments were precipitated with ethanol, they were dissolved in TE.

The KpnI-PstI DNA fragment prepared as above encoding the FR3 of versions "b" or "c" and the hATR5Lva/CVIDEC vector in which the FR3 was removed by digesting with KpnI and PstI were ligated using the DNA ligation kit ver.2 (Takara Shuzo) by reacting at 16°C for 1 hour according to the instructions attached to the kit.

The ligation mixture was added to 100 µl of E. coli JM109 competent cells (Nippongene) and was incubated for 30 minutes on ice and for 1 minute at 42°C. Then, 300 µl of Hi-Competence Broth (Nippongene) was added thereto, incubated at 37°C for 1 hour, and then the E. coli was plated on a 100 µg/ml LBA agar medium and incubated overnight at 37°C to obtain an E. coli transformant. The transformant was cultured overnight in 3 ml of the LBA medium, and from the cell fractions, plasmid DNA was prepared using the QIAprep Spin Plasmid Kit (QIAGEN).

The nucleotide sequence of the cDNA coding region in the plasmid was determined using the Dye Terminator Cycle Sequencing FS Ready Reaction Kit (Perkin-Elmer) and the DNA Sequencer 373A (Perkin-Elmer). As the sequencing primer, M13 Primer M4 (Takara Shuzo) and M13 Primer RV (Takara Shuzo) were used, and the sequence was determined by confirming the nucleotide sequence in both directions.

The plasmids that contain the gene encoding version "b" or version "c" in which the FR3 of humanized antibody L chain version "a" was replaced was designated as hATR5Lvb/CVIDEC or hATR5Lvc/CVIDEC, respectively. The nucleotide sequence and the corresponding amino acid sequence of the humanized L chain version "b" contained in plasmid hATR5Lvb/CVIDEC and the amino acid sequence of version "b" are shown in SEQ ID NO: 87 and 88. The nucleotide sequence and the corresponding amino acid sequence of the humanized L chain version "c" contained in plasmid hATR5Lvc/CVIDEC and the amino acid sequence of version "c" are shown in SEQ ID NO: 89 and 90.

### (iii) versions "b1" and "b2"

Versions "b1" and "b2" were generated by replacing the FR2 of version "b". For version "b1" the FR2 derived from human antibody S65921 (DDBJ, Tonge DW et al., Year Immunol., 7: 56-62, 1993) was used, and for version "b2" the FR2 derived from human antibody X93625 (DDBJ, Cox JP et al., Eur. J. Immunol., 24: 827-836, 1994) was used, respectively.

Primers F2SS (SEQ ID NO: 91) and F2SA (SEQ ID NO: 92) encoding the FR2 of version "b1", or primers F2XS (SEQ ID NO: 93) and F2XA (SEQ ID NO: 94) encoding the FR2 of version "b2" have a sequence complementary to each other, and have the recognition sequences of the restriction enzymes AflII and SpeI on both ends thereof. F2SS, F2SA, F2XS, and F2XA were synthesized by Pharmacia Biotech. 100 pmole each of F2SS and F2SA, or F2XS and F2XA were annealed by treating at 96°C for 2 minutes and at 50°C for 2 minutes, and the double stranded DNA fragments were generated.

These double stranded DNA fragments were digested with the restriction enzymes AflII (Takara Shuzo) and SpeI (Takara Shuzo) at 37°C for 1 hour. The digestion mixture was extracted with phenol and chloroform, and after the DNA fragments were precipitated with ethanol, they were dissolved in TE.

The plasmid hATR5Lvb/CVIDEC was digested with the restriction enzymes AflII (Takara Shuzo) and SpeI (Takara Shuzo) at 37°C for 1 hour. The digestion mixture was separated by agarose gel electrophoresis using a 1.5% NuSieve GTG agarose (FMC BioProducts), and the agarose strips having about 3000 bp long DNA fragments were excised. The agarose strip was extracted with phenol and chloroform, and after the DNA fragments were precipitated with ethanol, they were dissolved in TE.

The AflII-SpeI DNA fragment prepared as above encoding the FR2 of version "b1" or "b2" and the hATR5Lvb/CVIDEC vector in which the FR2 was removed by digesting with AflII and SpeI were ligated using the DNA ligation kit ver.2 (Takara Shuzo) by reacting at 16°C for 1 hour according to the instructions attached to the kit.

The ligation mixture was added to 100 µl of E. coli JM109 competent cells (Nippongene) and was incubated for 30 minutes on ice and for 1 minute at 42°C. Then, 300 µl of the Hi-Competence Broth (Nippongene) was added thereto, incubated at 37°C for 1 hour, and then the E. coli was plated on a 100 µg/ml LBA agar medium and incubated overnight at 37°C to obtain an E. coli transformant. The transformant was cultured overnight at 37°C in 4 ml of the LBA medium and, from the cell fractions, plasmid DNA was prepared using the QIAprep Spin Plasmid Kit (QIAGEN).

The nucleotide sequence of the cDNA coding region in the plasmid was determined using the Dye Terminator Cycle Sequencing FS Ready Reaction Kit (Perkin-Elmer) by the DNA Sequencer 373A (Perkin-Elmer). As the sequencing primer, M13 Primer M4 (Takara Shuzo) and M13 Primer RV (Takara Shuzo) were used, and the sequence was determined by confirming the nucleotide sequence in both directions.

The plasmids that contain the gene encoding version "b1" or "b2" in which the FR2 of humanized antibody L chain version "b" was replaced was designated as hATR5Lvbl/CVIDEC and hATR5Lv2/CVIDEC, respectively. The nucleotide sequence and the corresponding amino acid sequence of the humanized L chain version "b1" contained in plasmid hATR5Lvb1/CVIDEC and the amino acid sequence of version "b1" are shown in SEQ ID NO: 95 and 96. The nucleotide sequence and the corresponding amino acid sequence of the humanized L chain version "b2" contained in plasmid hATR5Lvb2/CVIDEC and the amino acid sequence of version "b2" are shown in SEQ ID NO: 97 and 98.

### (3) Construction of the expression vector of humanized antibody

### (i) Combination of humanized H chain ad chimeric L chain

The plasmid hATR5Hva/CVIDEC containing a H chain v region was digested with NheI and SalI, and a cDNA fragment of the humanized H chain v region was recovered and introduced to chATR5/N5KG4P (SalI/NheI) prepared by digesting chATR5/N5KG4P, a chATR-5 antibody expression plasmid vector, with NheI and SalI. The plasmid thus generated was designated as hHva-chLv/N5KG4P.

The plasmid hATR5Hvb/CVIDEC containing a H chain V region was digested with NheI and SalI, and a cDNA fragment of the humanized H chain V region was recovered and introduced to chATR5/N5KG4P (SalI/NheI) prepared by digesting chATR5/N5KG4P, a chATR-5 antibody expression plasmid vector, with NheI and SalI. The plasmid thus generated was designated as hHvb-chLv/N5KG4P.

The plasmids hATR5Hvc/CVIDEC, hATR5Hvd/CVIDEC, and hATR5Hve/CVIDEC containing a H chain V region were digested with NheI and SalI, and cDNA fragments of the humanized H chain V region were recovered and introduced to chATR5/N5KG4P (SalI/NheI) prepared by digesting chATR5/N5KG4P, a chATR-5 antibody expression plasmid vector, with NheI and SalI. The plasmids thus generated were designated as hHvc-chLv/N5KG4P, hHvd-chLv/N5KG4P, and hHve-chLv/N5KG4P.

The plasmids hATR5Hvf/CVIDEC and hATR5Hvh/CVIDEC containing a H chain V region were digested with NheI and SalI, and cDNA fragments of the humanized H chain V region were recovered and introduced to chATR5/N5KG4P (SalI/NheI) prepared by digesting chATR5/N5KG4P, a chATR-5 antibody expression plasmid vector, with NheI and SalI. The plasmids thus generated were designated as hHvf-chLv/N5KG4P and hHvh-chLv/N5KG4P.

The plasmids hATR5Hvi/CVIDEC and hATR5Hvj/CVIDEC containing a H chain V region were digested with NheI and SalI, and cDNA fragments of the humanized H chain V region were recovered and introduced to chATR5/N5KG4P (SalI/NheI) prepared by digesting chATR5/N5KG4P, a chATR-5 antibody expression plasmid vector, with NheI and SalI. The plasmids thus generated were designated as hHvi-chLv/N5KG4P and hHvj-chLv/N5KG4P.

The plasmids hATR5Hb1/CVIDEC and hATR5Hvd1/CVIDEC containing a H chain V region were digested with NheI and SalI, and cDNA fragments of the humanized H chain V region were recovered and introduced to chATR5/N5KG4P (SalI/NheI) prepared by digesting chATR5/N5KG4P, a chATR-5 antibody expression plasmid vector, with NheI and SalI. The plasmids thus generated were designated as hHvb1-chLv/N5KG4P and hHvdl-chLv/N5KG4P.

### (ii) Combination of humanized L chain ad chimeric H chain

Using an antibody expression vector N5KG4P, it was combined with a chimeric H chain and was expressed, and the humanized L chain was evaluated.

The plasmids hATR5Lva/CVIDEC, hATR5Lvb/CVIDEC, hATR5Lvc/CVIDEC, hATR5Lvb1/CVIDEC, and hATR5Lvb2/CVIDEC were digested with the restriction enzymes BglII (Takara Shuzo) and SplI (Takara Shuzo) at 37°C for 2-3 hours. The digestion mixture was separated by agarose gel electrophoresis using a 1.5% or 2% NuSieve GTG agarose (FMC BioProducts), and the agarose strips having about 400 bp long DNA fragments were excised. The agarose strips were extracted with phenol and chloroform, and after the DNA fragments were precipitated with ethanol, they were dissolved in TE.

The SplI-BglII DNA fragment containing the gene encoding the a humanized L chain V region of each of these versions and the hATR5Hv/N5KG4P digested with SplI and BglII were ligated using the DNA ligation kit ver.2 (Takara Shuzo) by reacting at 16°C for 1 hour according to the instructions attached to the kit.

The ligation mixture was added to 100 µl of E. coli JM109 competent cells (Nippongene) and was incubated for 30 minutes on ice and for 1 minute at 42°C. Then, 300 µl of the Hi-Competence Broth (Nippongene) was added thereto, incubated at 37°C for 1 hour, and then the E. coli was plated on a 100 µg/ml LBA agar medium and incubated overnight at 37°C to obtain an E. coli transformant.

The transformant was cultured overnight at 37°C in 250 ml or 500 ml of the LBA medium, and from the cell fractions, plasmid DNA was prepared using the Plasmid Maxi Kit (QIAGEN). The plasmids in which a gene encoding the chimeric H chain and humanized L chain was introduced were designated as chHv-hLva/N5KG4P, chHv-hLvb/N5KG4P, chHv-hLvc/N5KG4P, chHv-hLvb1/N5KG4P, and chHv-hLvb2/N5KG4P.

### (iii) Combination of humanized H chain and humanized L chain

The plasmid hATR5Hva/CVIDEC containing a H chain V region was digested with NheI and SalI, and a cDNA fragment of the humanized H chain V region was recovered and introduced to hLva/N5KG4P (SalI/NheI) prepared by digesting plasmid chHv-hLva/N5KG4P containing the cDNA sequence of humanized ATR-5 antibody L chain version "a" with NheI and SalI. The plasmid thus generated was designated as hHva-hLva/N5KG4P.

The plasmids hATR5Hvb/CVIDEC and hATR5Hvc/CVIDEC containing a H chain V region were digested with NheI and SalI, and cDNA fragments of the humanized H chain V region were recovered and introduced to hLva/N5KG4P (SalI/NheI) prepared by digesting plasmid chHv-hLva/N5KG4P containing the cDNA sequence of humanized ATR-5 antibody L chain version "a" with NheI and SalI. The plasmids thus generated were designated as hHvb-hLva/N5KG4P and hHvc-hLva/N5KG4P.

The plasmids hATR5Hvb/CVIDEC, hATR5Hvd/CVIDEC, and hATR5Hve/CVIDEC containing a H chain V region were digested with NheI and SalI, and cDNA fragments of the humanized H chain V region were recovered and introduced to hLvb/N5KG4P (SalI/NheI) prepared by digesting plasmid chHv-hLvb/N5KG4P containing the cDNA sequence of humanized ATR-5 antibody L chain version "b" with NheI and SalI. The plasmids thus generated were designated as hHvb-hLvb/N5KG4P, hHvd-hLvb/N5KG4P, and hHve-hLvb/N5KG4P.

The plasmids hATR5Hvf/CVIDEC, hATR5Hvg/CVIDEC, and hATR5Hvh/CVIDEC containing a H chain V region were digested with NheI and SalI, and cDNA fragments of the humanized H chain V region were recovered and introduced to hLvb/N5KG4P (SalI/NheI) prepared by digesting plasmid chHv-hLvb/N5KG4P containing the cDNA sequence of humanized ATR-5 antibody L chain version "b" with NheI and SalI. The plasmids thus generated were designated as hHvf-hLvb/N5KG4P, hHvg-hLvb/N5KG4P, and hHvh-hLvb/N5KG4P.

The plasmids hATR5Hvi/CVIDEC and hATR5Hvj/CVIDEC containing a H chain V region were digested with NheI and SalI, and cDNA fragments of the humanized H chain V region were recovered and introduced to hLvb/N5KG4P (SalI/NheI) prepared by digesting plasmid chHv-hLvb/N5KG4P containing the cDNA sequence of humanized ATR-5 antibody L chain version "b" with NheI and SalI. The plasmids thus generated were designated as hHvi-hLvb/N5KG4P and hHvj-hLvb/N5KG4P.

The plasmids hATR5Hvb1/CVIDEC and hATR5Hvd1/CVIDEC containing a H chain V region were digested with NheI and SalI, and cDNA fragments of the humanized H chain V region were recovered and introduced to hLvb/N5KG4P (SalI/NheI) prepared by digesting plasmid chHv-hLvb/N5KG4P containing the cDNA sequence of humanized ATR-5 antibody L chain version "b" with NheI and SalI. The plasmids thus generated were designated as hHvb1-hLvb/N5KG4P and hHvd1-hLvb/N5KG4P.

The plasmids hATR5Hvb3/CVIDEC and hATR5Hvd3/CVIDEC containing a H chain V region were digested with NheI and SalI, and cDNA fragments of the humanized H chain V region were recovered and introduced to hLvb/N5KG4P (SalI/NheI) prepared by digesting plasmid chHv-hLvb/N5KG4P containing the cDNA sequence of humanized ATR-5 antibody L chain version "b" with NheI and SalI. The plasmids thus generated were designated as hHvb3-hLvb/N5KG4P and hHvd3-hLvb/N5KG4P.

The plasmid hATR5Hvb/CVIDEC containing a H chain V region was digested with NheI and SalI, and a cDNA fragment of the humanized H chain V region was recovered and introduced to hLvb1/N5KG4P (SalI/NheI) and hLvb2/N5KG4P (SalI/NheI) prepared by digesting plasmids chHv-hLvb1/N5KG4P and chHv-hLvb2/N5KG4P containing the cDNA sequence of humanized ATR-5 antibody L chain versions "b1" and "b2" with NheI and SalI. The plasmids thus generated were designated as hHvb-hLvbl/N5KG4P and hHvb-hLvb2/N5KG4P.

The plasmid hATR5Hvi/CVIDEC containing a H chain V region was digested with NheI and SalI, and a cDNA fragment of the humanized H chain V region was recovered and introduced to hLvb1/N5KG4P (SalI/NheI) and hLvb2/N5KG4P (SalI/NheI) prepared by digesting plasmids chHv-hLvb1/N5KG4P and chHv-hLvb2/N5KG4P containing the cDNA sequence of humanized ATR-5 antibody L chain versions "b1" and "b2" with NheI and SalI. The plasmids thus generated were designated as hHvi-hLvb1/N5KG4P and hHvi-hLvb2/N5KG4P.

### (4) Transfection into COS-7 cells

In order to evaluate the activity of binding to the antigen and neutralizing activity of humanized antibody, the above antibody was transiently expressed in COS-7 cells.

The constructed expression plasmid vector was transduced into COS-7 cells by electroporation using the Gene Pulser instrument (Bio Rad). Fifty µg or 20 µg of the plasmid was added to 0.78 ml of COS-7 cells suspended in PBS at a cell concentration of 1 × 10⁷ cells/ml, which was subjected to pulses of 1,500 V and 25 µF capacity.

After 10 minutes of the recovery period at room temperature, the electroporated cells were suspended in a DMEM medium (GIBCO) containing 5% Ultra low IgG fetal bovine serum (GIBCO), and cultured using a 10 cm culture dish or 15 cm culture dish in a 5% CO₂ incubator. After culturing for 24 hours, the culture supernatant was aspirated off, and then a serum-free medium HBCHO (Irvine Scientific) was added. After further culturing for 72 hours or 96 hours, the culture supernatant was collected and centrifuged to remove cell debris.

### (5) Purification of antibody

From the culture supernatant of the COS-7 cells, the antibody was purified using the AffiGel Protein A MAPSII kit (Bio Rad) or the rProtein A Sepharose Fast Flow (Pharmacia Biotech). Purification using the AffiGel Protein A MAPSII kit was carried out according to the instructions attached to the kit. Purification using the rProtein A Sepharose Fast Flow was carried out as follows:

One ml of rProtein A Sepharose Fast Flow was filled into a column and the column was equilibrated by 10 volumes of TBS. The culture supernatant of COS-7 cells was applied to the equilibrated column, which was then washed with 10 volumes of TBS. The adsorbed antibody fraction was eluted by 13.5 ml of 2.5 mM HCl (pH 3.0). The eluate was neutralized by adding 1.5 ml of 1 M Tris-HCl (pH 8.0).

By performing ultrafiltration two or three times for the purified antibody fraction using the Centriprep 30 or 100 (amicon), the solvent was replaced to TBS, and was finally concentrated to about 1.5 ml.

### Reference Example 6. Antibody quantitation and activity evaluation

### (1) Measurement of antibody concentration by ELISA

ELISA plates for measurement of antibody concentration were prepared as follows: Each well of a 96-well ELISA plate (Maxisorp, NUNC) was immobilized by 100 µl of goat anti-human IgGγ antibody (BIO SOURCE) prepared to a concentration of 1 µg/ml in the immobilization buffer (0.1 M NaHCO₃, 0.02% NaN₃, pH 9.6) (hereinafter referred to as CB). After blocking with 200 µl of the dilution buffer (50 mM Tris-HCl, 1 mM MgCl₂, 0.1 M NaCl, 0.05% Tween 20, 0.02% NaN₃, 1% bovine serum albumin (BSA), pH 8.1) (hereinafter referred to as DB), the culture supernatant of the COS-7 cells in which antibody was expressed or purified antibody were serially diluted with DB, and then added to each well.

After incubating at room temperature for 1 hour followed by washing with the Dulbecco PBS containing 0.05% Tween 20 (hereinafter referred to as RB), 100 µl of alkaline phosphatase-conjugated goat anti-human IgGγ antibody (Biosource) which was diluted 1000-fold with DB was added. After incubating at room temperature for 1 hour followed by washing with the RB, Sigma104 (p-nitrophenyl phosphate, SIGMA) dissolved in the substrate buffer (50 mM NaHCO₃, 10 mM MgCl₂, pH 9.8) to 1 mg/ml was added, and then the absorbance at 405/655 nm was measured using the Microplate Reader (Bio Rad). As the standard for the measurement of concentration, IgG4κ (Binding Site) was used.

### (2) Measurement of the activity of binding to the antigen

Cell ELISA plates for measurement of antigen binding were prepared as follows. Cells used were human bladder carcinoma cells J82 (ATCC HTB-1). To 60 wells of a 96-well cell culture plate, 1 × 10⁵ J82 cells were inoculated. This was cultured (RPMI1640 medium containing 10% fetal bovine serum (GIBCO)) for one day in a CO₂ incubator to allow the cells to be attached thereto. After discarding the culture liquid, each well was washed twice with 300 µl PBS. 100 µl of PBS containing 4% paraformaldehyde (hereinafter referred to as PFA/PBS) was added to each well, and placed on ice for 10 minutes to immobilize the cells.

PFA/PBS was discarded, and each well was washed twice with 300 µl of PBS, and then blocked with 250 µl of DB. The culture supernatant or purified antibody was serially diluted with DB, 100 µl of which was added to each well. After incubating at room temperature for 2 hours followed by washing with RB, 100 µl of alkaline phosphatase-conjugated goat anti-human IgGγ antibody (BioSource) diluted 1000-fold with DB was added. After incubating for 1 hour followed by washing with RB, the substrate solution was added, and then absorbance at 405/655 nm was measured using the Microplate Reader (Bio-Rad).

### (3) Measurement of neutralizing activity

The neutralizing activity of mouse antibody, chimeric antibody, and humanized antibody was measured with the inhibiting activity against the Factor Xa-production activity by human placenta-derived thromboplastin, Thromborel S (Boehringer AG), as an index. Thus, 60 µl of the buffer (TBS containing 5 mM CaCl₂ and 0.1% BSA) was added to 10 µl of 1.25 mg/ml Thromborel S and 10 µl of appropriately diluted antibody, which was then incubated in a 96-well plate at room temperature for 1 hour. Ten µl each of 3.245 µg/ml human Factor X (Celsus Laboratories) and 82.5 ng/ml human Factor VIIa (Enzyme Research) were added thereto, and then were incubated at room temperature for 1 hour.

Ten µl of 0.5 M EDTA was added to stop the reaction, to which 50 µl of the chromogenic substrate solution was added and the absorbance at 405/655 nm was determined using the Microplate Reader (Bio Rad). After reacting at room temperature for 1 hour, the absorbance at 405/655 nm was determined again. The neutralizing activity may be determined by calculating the residual activity (%) from each change in absorbance with the hourly absorbance change at no antibody addition as a 100% activity.

The chromogenic substrate solution was prepared by dissolving the Testzyme chromogenic substrate S-2222 (Chromogenix) according to the attached instructions, diluting 2-fold with purified water and mixing with a polybrene solution (0.6 mg/ml hexadimethylene bromide, SIGMA) at 1:1.

### (4) Evaluation of activity

### (i) Combination of the humanized H chain version "a" and a chimeric L chain

An antibody (a-ch) which is the humanized H chain version "a" combined with a chimeric L chain was generated, and was tested for the binding activity to the antigen by the cell ELISA. The amount bound to the antigen was found to be decreased at a high concentration. The neutralizing activity against the antigen by FXa production-inhibition was weak as compared to that of the positive control chimeric antibody (ch-ch). Therefore, it was decided to perform the version-up of the humanized H chain by FR-shuffling. The chimeric antibody used herein was the one that was expressed in COS-7 cells, purified, and evaluated.

### (ii) Combination of the humanized L chain version "a" and a chimeric H chain

An antibody (ch-a) which is the humanized L chain version "a" combined with a chimeric H chain was generated, and was tested for the binding activity to the antigen by the cell ELISA. It was found to have the binding activity equal to or higher than that of the chimeric antibody. On the other hand, the neutralizing activity against the antigen was weak as compared to that of the positive control chimeric antibody. Therefore, it was decided to perform the version-up of the humanized L chain by FR-shuffling. The chimeric antibody used herein was the one that was expressed in COS-7 cells, purified, and evaluated.

### (iii) Combination of the humanized H chain version "a" and the humanized L chain version "a"

An antibody (a-a) which is the humanized H chain version "a" combined with the humanized L chain version "a" was generated, and was tested for the binding activity to the antigen by the cell ELISA. The amount bound to the antigen was found to be decreased in the high concentration side. The neutralizing activity against the antigen by FXa production-inhibition was weak as compared to that of the positive control chimeric antibody. Therefore, it was decided to perform the version-up of the humanized H chain and L chain by FR-shuffling. The chimeric antibody used herein was the one that was expressed in COS-7 cells, purified, and evaluated.

### (iv) Combination of the humanized H chain versions "b", "c", and "d", and a chimeric L chain

Antibodies ("b-ch", "c-ch", and "d-ch", respectively) which are the humanized H chain subjected to version-up by FR-shuffling combined with a chimeric L chain were generated, and were tested for the binding activity to the antigen by the cell ELISA. "d-ch" exhibited a binding activity equal to that of the chimeric antibody, and "b-ch" and "c-ch" exhibited a slightly lower binding activity. On the other hand, the neutralizing activity against the antigen as compared to that of positive control chimeric antibody was almost equal in "b-ch", and slightly weak in "d-ch". In version "c-ch", it was significantly weaker than that of the chimeric antibody. Therefore, the humanized H chain versions "b" and "d" were considered the ones of the humanized H chain to exhibit a high activity.

### (v) Combination of the humanized H chain version "b" and the humanized L chain version "a"

An antibody (b-a) which is the humanized H chain version "b" subjected to version-up by FR-shuffling combined with the humanized L chain version "a" was generated, and was tested for the binding activity to the antigen by the cell ELISA. The amount bound to the antigen was found to be decreased at a high concentration. On the other hand, the neutralizing activity against the antigen was significantly weak as compared to that of the positive control chimeric antibody. Therefore, "b-a" and "a-a" were the ones that exhibit a high activity. The chimeric antibody used herein was the one that was expressed in COS-7 cells, purified, and evaluated.

### (vi) Combination of the humanized L chain versions "b" and "c", and a chimeric H chain

Antibodies ("ch-b" and "ch-c", respectively) which are the humanized L chain versions "b" and "c" combined with a chimeric H chain were generated, and both of them were found to have the binding activity to the antigen and the neutralizing activity against the antigen equal to the chimeric antibody. Therefore, versions "b" and "c" were chosen as a candidate for a humanized antibody L chain. Mouse antibody-derived version "b" which is one amino acid smaller in the amino acid residue number is considered to be superior to version "c" in terms of antigenicity. The chimeric antibody used herein was the one that was expressed in CHO cells DG44, purified, and evaluated. In the evaluation hereinafter the antibody was used as the positive control.

### (vii) Combination of the humanized H chain version "b" and the humanized L chain versions "b" and "c"

Antibodies ("b-b" and "b-c", respectively) which are the humanized H chain version "b" combined with the humanized L chain versions "b" and "c" were generated, and tested for the binding activity to the antigen and the neutralizing activity against the antigen. Both of them had a slightly lower activity than that of the chimeric antibody in both the binding activity and the neutralizing activity.

### (viii) Combination of the humanized H chain versions "b" and "d", and the humanized L chain version "b"

Antibodies ("b-b" and "d-b", respectively) which are the humanized H chain subjected to version-up by FR-shuffling combined with the humanized L chain version "b" were generated, and were tested for the binding activity to the antigen by the cell ELISA. "d-b" exhibited a binding activity equal to that of the chimeric antibody, and "b-b" exhibited a slightly lower binding activity at the high concentration. On the other hand, the neutralizing activity against the antigen as compared to that of the positive control chimeric antibody was slightly low in "b-b", and significantly weak in "d-b". Therefore, it was shown that "b-b" is a high neutralizing activity version, whereas "d-b" is a high binding activity version.

### (ix) Combination of the humanized H chain version "e", and a chimeric L chain and the humanized L chain version "b"

Antibodies ("e-ch" and "e-b", respectively) which are the humanized L chain version "e" combined with a chimeric L chain and the humanized version "b" were generated. "e-ch" exhibited a binding activity to the antigen equal to that of the chimeric antibody, but in "e-b" the amount of antibody expressed was very little and most of the binding activity was lost. The neutralizing activity against the antigen of "e-ch" was significantly low as compared to that of the chimeric antibody. Therefore, it was concluded that the H chain version "e" combined with L chain version "b" did not work well.

### (x) Combination of the humanized H chain versions "f", "g", and "h", and the humanized L chain version "b"

Antibodies ("f-b", "g-b", and "h-b", respectively) which are the humanized H chain versions "f", "g", and "h" combined with the humanized L chain version "b" were generated. In "f-b" and "h-b" antibody, the amount of antibody expressed was very small. For versions "f" and "h", antibodies combined with the chimeric L chain were generated, but were not expressed. "g-b" reached saturation at a low concentration, and exhibited a binding activity weaker than that of the chimeric antibody. The neutralizing activity against the antigen of "g-b" was significantly weak as compared to that of the chimeric antibody.

### (xi) Combination of the humanized H chain versions "b1" and "d1", and the humanized L chain version "b"

Antibodies ("b1-b" and "d1-b", respectively) which are the humanized H chain versions "b1" and "d1" combined with the humanized L chain version "b" were generated. Almost no antibody was expressed in any of them. For these, antibodies combined with a chimeric L chain were generated, but were not expressed.

### (xii) Combination of the humanized H chain versions "b3" and "d3", and the humanized L chain version "b"

Antibodies ("b3-b" and "d3-b", respectively) which are the humanized H chain versions "b3" and "d3" combined with the humanized L chain version "b" were generated. The binding activity to the antigen of "d3-b" was slightly lower than that of the chimeric antibody, and that of "b3-b" was much lower. The neutralizing activity against the antigen of "b3-b" was higher than that of "b-b", but was lower than that of the chimeric antibody, and "d3-b" and "b-b" remained equal in activity.

### (xiii) Combination of the humanized H chain versions "i" and "j", and a chimeric L chain and the humanized L chain version "b"

Antibodies ("i-ch" and "j-ch", respectively) which are the humanized H chain versions "i" and "j" combined with a chimeric L chain, and antibodies ("i-b" and "j-b", respectively) combined with the humanized L chain version "b" were generated, and were tested for the binding activity to the antigen and the neutralizing activity against the antigen. The binding activity of any of the antibodies was almost equal to that of the chimeric antibody. "i-ch" exhibited the neutralizing activity higher than that of the chimeric antibody, and "j-ch" was significantly lower than that of the chimeric antibody. "i-b" exhibited the neutralizing activity equal to that of the chimeric antibody, and "j-b" exhibited a significantly weaker neutralizing activity than that of that of the chimeric antibody.

### (xiv) The humanized L chain versions "b1" and "b2"

When antibodies ("ch-b1" and "ch-b2", respectively) which are the humanized L chain versions "b1" and "b2" combined with a chimeric H chain were generated, both of them exhibited the binding activity to the antigen equal to that of the chimeric antibody. For the neutralizing activity against the antigen, "ch-b1" exhibited the binding activity equal to that of the chimeric antibody, while "ch-b2" exhibited an activity slightly higher than that of the chimeric antibody at the high concentration. Versions "b1" and "b2" can be candidates of a humanized antibody L chain, but "b2" is superior in that it has a stronger activity.

### (xv) Combination of the humanized H chain version "b" and the humanized L chain version "b2"

An antibody ("b-b2") which is the humanized H chain version "b" combined with the humanized L chain version "b2" was generated, and was tested for the binding activity to the antigen and the neutralizing activity against the antigen. The binding activity was slightly lower than that of the chimeric antibody. The neutralizing activity, though slightly higher than that of "b-b", was lower than that of "i-b".

### (xvi) Combination of the humanized H chain version "i" and, the humanized L chain version "b1" or "b2"

Antibodies ("i-b1" and "i-b2", respectively) which are the humanized H chain version "i" combined with the humanized L chain version "b1" or "b2" were generated, and were tested for the binding activity to the antigen and the neutralizing activity against the antigen. The binding activity of "i-b2" was almost equal to that of the chimeric antibody, and that of "i-b1" was slightly lower than that of chimeric antibody. The neutralizing activity of "i-b1" and "i-b2" was higher than that of the chimeric antibody and "i-b", which was in a decreasing order of "i-b2" > "i-b1".

### Reference Example 7. Preparation of CHO cell-producing humanized antibody and the evaluation of its activity

### (1) Establishment of a cell line that stably produces CHO

In order to establish cell lines that stably produce a humanized antibody (b-b, i-b, and i-b2), an antibody expression gene vector was introduced into CHO cells (DG44) acclimated to a serum-free medium.

Plasmid DNA, hHvb-hLvb/N5KG4P, hHvi-hLvb/N5KG4P, and hHvi-hLvb2/N5KG4P were digested with the restriction enzyme SspI (Takara Shuzo) and linearized, which was extracted with phenol and chloroform, and purified by ethanol precipitation. The linearized expression gene vector was introduced into the DG44 cells using the electroporation instrument (Gene Pulser; Bio Rad). The DG44 cells were suspended in PBS at a cell concentration of 1 × 10⁷ cells/ml, and to about 0.8 ml of this suspension 10 or 50 µg of the DNA was added, which was subjected to pulses of 1,500 v and 25 µF capacity.

After 10 minutes of the recovery period at room temperature, the treated cells were suspended in a CHO-S-SFMII medium (GIBCO) containing hypoxanthine/thymidine (GIBCO) (hereinafter referred to as HT), which was inoculated on two 96-well plates (Falcon) at 100 µl/well, and cultured in a CO₂ incubator. Eight to nine hours after the start of culturing, 100 µl/well of the CHO-S-SFMII medium containing HT and 1 mg/ml GENETICIN (GIBCO) was added to change to 500 µg/ml of the GENETICIN selection medium, and the cells into which the antibody gene had been introduced were selected. The medium was changed with a fresh one once every 3-4 days with 1/2 the volume. At a time point about 2 weeks after changing to the selection medium, an aliquot of the culture supernatant was recovered from the well in which a favorable cell growth was observed 4-5 days later. The concentration of antibody expressed in the culture supernatant was measured by the ELISA described above for measuring antibody concentration, and cells having a high production yield of antibody were selected.

### (2) Large scale purification of humanized antibody

After the DG44 cell lines selected as above that produce the humanized antibody ("b-b", "i-b", and "i-b2") were cultured for a few days in a 500 ml/bottle of the CHO-S-SFMII medium using a 2 liter roller bottle (CORNING), the culture medium was harvested and a fresh CHO-S-SFMII medium was added and cultured again. The culture medium was centrifuged to remove the cell debris, and filtered with a 0.22 µm or 0.45 µm filter. By repeating this, a total of about 2 liters each of the culture supernatant was obtained. From the culture supernatant obtained, antibody was purified by the ConSep LC100 system (Millipore) connected to the Protein A affinity column (Poros).

### (3) Measurement of antibody concentration by ELISA

ELISA plates for measurement of antibody concentration were prepared as follows: Each well of a 96-well ELISA plate (Maxisorp, NUNC) was immobilized with 100 µl of goat anti-human IgGγ antibody (BioSource) prepared to a concentration of 1 µg/ml with CB. After blocking with 200 µl of DB, the culture supernatant of the CHO cells in which antibody had been expressed or the purified antibody was serially diluted with DB, and added to each well.

After incubating at room temperature for 1 hour and washing with RB, 100 µl of alkaline phosphatase-conjugated goat anti-human IgGγ antibody (BioSource) diluted 1000-fold with DB was added. After incubating at room temperature for 1 hour and washing with RB, 100 µl of the substrate solution was added, and then the absorbance at 405/655 nm was measured using the Microplate Reader (Bio Rad). As the standard for the measurement of concentration, human IgG4κ (The Binding Site) was used.

### (4) Measurement of activity of binding to the antigen

Cell ELISA plates for measurement of antigen binding were prepared as follows. Cells used were human bladder carcinoma cells J82 (ATCC HTB-1), which were inoculated onto a 96-well cell culture plate at a cell count of 1 × 10⁶ cells. This was cultured (RPMI1640 medium containing 10% fetal bovine serum (GIBCO)) for one day in a CO₂ incubator to allow the cells to be attached thereto. After discarding the culture liquid, each well was washed twice with PBS. 100 µl of PFA/PBS was added to each well, and placed on ice for 10 minutes to immobilize the cells.

PFA/PBS was discarded, and each well was washed twice with 300 µl of PBS and then blocked with 250 µl of DB. Based on the above result of measurement, the purified antibody was serially diluted with DB starting at 10 µg/ml by a factor of 2, 100 µl of which was added to each well. After incubating at room temperature for 2 hours and washing with RB, 100 µl of alkaline phosphatase-conjugated goat anti-human IgGγ antibody (BioSource) diluted 1000-fold with DB was added. After incubating at room temperature for 1 hour and washing with RB, 100 µl of the substrate solution was added, and then absorbance at 405/655 nm was measured using the Microplate Reader (Bio-Rad).

### (5) Measurement of neutralizing activity against TF (Inhibiting activity against FXa production)

The Factor Xa production-inhibiting activity of humanized antibody was measured with the inhibiting activity against the Factor Xa production activity by the human placenta-derived thromboplastin, Thromborel S (Boehringer AG), as an index. Thus, 60 µl of the buffer (TBS containing 5 mM CaCl₂ and 0.1% BSA) was added to 10 µl of 5 mg/ml Thromborel S and 10 µl of the antibody, which was then incubated in a 96-well plate at room temperature for 1 hour. The antibody was serially diluted with the buffer, starting at 200 µg/ml, by a factor of 5.

Ten µl each of 3.245 µg/ml human Factor X (Celsus Laboratories) and 82.5 ng/ml human Factor VIIa (Enzyme Research) were added thereto, and were further incubated at room temperature for 45 minutes. Ten µl of 0.5 M EDTA was added to stop the reaction. Fifty µl of the chromogenic substrate solution was added thereto and the absorbance at 405/655 nm was determined by the Microplate Reader (Bio Rad). After reacting at room temperature for 30 minutes, the absorbance at 405/655 nm was measured again. The residual activity (%) was determined from each change in absorbance with the absorbance change for 30 minutes at no antibody addition as a 100% activity.

The chromogenic substrate solution was prepared by dissolving the Testzyme chromogenic substrate S-2222 (Chromogenix) according to the attached instructions, and mixing with a polybrene solution (0.6 mg/ml hexadimethylene bromide, SIGMA) at 1:1.

### (6) Measurement of neutralizing activity against TF (inhibiting activity against Factor X-binding)

The inhibiting activity against Factor X-binding of humanized antibody was measured using the human placenta-derived thromboplastin, Thromborel S (Boehringer AG), in which a complex of TF and Factor VIIa had previously been formed and the inhibiting activity against Factor X-binding was measured with the Factor Xa production activity of the TF-Factor VIIa complex as an index. Thus, 60 µl of the buffer (TBS containing 5 mM CaCl₂ and 0.1% BSA) was added to 10 µl of 5 mg/ml Thromborel S and 10 µl of 82.5 ng/ml human Factor VIIa (Enzyme Research), which was preincubated in a 96-well plate at room temperature for 1 hour.

Ten µl of the antibody solution was added thereto, incubated at room temperature for 5 minutes, and 10 µl of 3.245 µg/ml human Factor X (Celsus Laboratories) was added and was further incubated at room temperature for 45 minutes. The antibody was serially diluted with the buffer starting at 200 µg/ml by a factor of 2. Ten µl of 0.5 M EDTA was added to stop the reaction. Fifty µl of the chromogenic substrate solution was added thereto and the absorbance at 405/655 nm was determined by the Microplate Reader (Bio Rad). After reacting at room temperature for 30 minutes, the absorbance at 405/655 nm was measured again. The residual activity (%) was determined from each change in absorbance with the absorbance change for 30 minutes at no antibody addition as a 100% activity.

The chromogenic substrate solution was prepared by dissolving the Testzyme chromogenic substrate S-2222 (Chromogenix) according to the attached instructions, and mixing with a polybrene solution (0.6 mg/ml hexadimethylene bromide, SIGMA) at 1:1.

### (7) Measurement of neutralizing activity against TF (inhibiting activity against the plasma coagulation)

The neutralizing activity against TF (inhibiting activity against the plasma coagulation) of humanized antibody was measured using, as an index, the prothrombin time determined using the human placenta-derived thromboplastin, Thromborel S (Boehringer AG). Thus, 100 µl of human plasma (Cosmo Bio) was placed into a sample cup, to which 50 µl of antibody diluted at various concentrations was added, and heated at 37°C for 3 minutes. Fifty µl of 1.25 mg/ml Thromborel S that had previously been preheated at 37°C was added to start plasma coagulation. The coagulation time was measured using the Amelung KC-10A connected to the Amelung CR-A (both from M. C. Medical).

The antibody was serially diluted with TBS containing 0.1% BSA (hereinafter referred to as BSA-TBS) starting at 80 µg/ml by a factor of 2. With the coagulation time of no antibody addition as 100% TF plasma coagulation activity, the residual TF activity was calculated from each coagulation time at antibody addition based on a standard curve obtained by plotting the concentration of Thromborel S and the coagulation time.

The standard curve was created from the various concentration of Thromborel S and the coagulation time was measured. Fifty µl of BSA-TBS was added to 50 µl of appropriately diluted Thromborel S, which was heated at 37°C for 3 minutes, 100 µl of human plasma preheated at 37°C was added to start coagulation, and the coagulation time was determined. Thromborel S was serially diluted with the Hank's buffer (GIBCO) containing 25 mM CaCl₂ starting at 6.25 mg/ml by a factor of 2. The Thromborel S concentration was plotted on the abscissa, and the coagulation time on the ordinate on a log-log paper, which was rendered a standard curve.

### (8) Activity evaluation

All humanized antibodies, "b-b", "i-b", and "i-b2" had an activity equal to or greater than that of the chimeric antibody (Figure 1). For inhibiting activity against Factor Xa production, inhibiting activity Factor X-binding, and inhibiting activity against plasma coagulation as well, the humanized antibodies, "b-b", "i-b", and "i-b2" had an activity equal to or greater than that of the chimeric antibody, and the activity was of a decreasing order "i-b2" > "i-b" > "b-b" (Figures 2, 3, and 4).

## Claims

1. A blood rheology-improving agent comprising an antibody against human tissue factor (human TF).

2. The blood rheology-improving agent according to claim 1 wherein said antibody is a polyclonal antibody.

3. The blood rheology-improving agent according to claim 1 wherein said antibody is a monoclonal antibody.

4. The blood rheology-improving agent according to claim 1 or 3 wherein said antibody is a recombinant antibody.

5. The blood rheology-improving agent according to claim 1 or 4 wherein said antibody is an altered antibody.

6. The blood rheology-improving agent according to claim 1, 4 or 5 wherein said altered antibody is a chimeric antibody or a humanized antibody.

7. The blood rheology-improving agent according to claim 6 wherein said humanized antibody is a humanized antibody of version b-b, i-b, or i-b2.

8. The blood rheology-improving agent according to any one of claims 1, or 4-7 wherein said antibody is an antibody modification.

9. The blood rheology-improving agent according to claim 8 wherein said antibody modification is an antibody fragment Fab, F(ab')₂, or Fv, or a single chain Fv (scFv).

10. The use of an antibody against human tissue factor (human TF) for the production of a blood rheology-improving agent.

11. The use according to claim 10 wherein said antibody is a polyclonal antibody.

12. The use according to claim 10 wherein said antibody is a monoclonal antibody.

13. The use according to claim 10 or 12 wherein said antibody is a recombinant antibody.

14. The use according to claim 10 or 13 wherein said antibody is an altered antibody.

15. The use according to claim 10, 13 or 14 wherein said altered antibody is a chimeric antibody or a humanized antibody.

16. The use according to claim 15 wherein said humanized antibody is a humanized antibody of version b-b, i-b, or i-b2.

17. The use according to any one of claims 10, or 13-16 wherein said antibody is an antibody modification.

18. The use according to claim 17 wherein said antibody modification is an antibody fragment Fab, F(ab')₂, or Fv, or a single chain Fv (scFv).

19. A method of improving blood rheology comprising administering an antibody against human tissue factor (human TF).

20. The method according to claim 19 wherein said antibody is a polyclonal antibody.

21. The method according to claim 19 wherein said antibody is a monoclonal antibody.

22. The method according to claim 19 or 21 wherein said antibody is a recombinant antibody.

23. The method according to claim 19 or 22 wherein said antibody is an altered antibody.

24. The method according to claim 19, 22 or 23 wherein said altered antibody is a chimeric antibody or a humanized antibody.

25. The method according to claim 24 wherein said humanized antibody is a humanized antibody of version b-b, i-b, or i-b2.

26. The method according to any one of claims 19, or 22-25 wherein said antibody is an antibody modification.

27. The method according to claim 26 wherein said antibody modification is an antibody fragment Fab, F(ab')₂, or Fv, or a single chain Fv (scFv).


SEQUENCE LISTING
<110> CHUGAI SEIYAKU KABUSHIKI KAISHA
<120> Blood rheology improving agent
<130> 994125
<160> 102
<210> 1
<211> 28
<212> DNA
<213> Artificial Sequence
<220>
<223> Primer MHC-G1
<400> 1
ggatcccggg ccagtggata gacagatg 28
<210> 2
<211> 27
<212> DNA
<213> Artificial Sequence
<220>
<223> Primer MKC
<400> 2
ggatcccggg tggatggtgg gaagatg 27
<210> 3
<211> 17
<212> DNA
<213> Artificial Sequence
<220>
<223> M13 Primer M4
<400> 3
gttttcccag tcacgac 17
<210> 4
<211> 17
<212> DNA
<213> Artificial Sequence
<220>
<223> M13 Primer RV

<400> 4
caggaaacag ctatgac                                                    17
<210> 5
<211> 408
<212> DNA
<213> Mouse
<220>
<221> sig-peptide
<222> (1)...(57)
<220>
<221> mat-peptide
<222> (58)...(408)
<223> Nucleotide sequence coding for H chain V region of
anti-TF mouse monoclonal antibody ATR-5
<400> 5
atg aaa tgc agc tgg gtc atc ttc ttc ctg atg gca gtg gtt aca ggg      48
Met Lys Cys Ser Trp Val Ile Phe Phe Leu Met Ala Val Val Thr Gly
                -15                 -10                 -5
gtc aat tca gag gtt cag ctg cag cag tct ggg act aac ctt gtg agg      96
Val Asn Ser Glu Val Gln Leu Gln Gln Ser Gly Thr Asn Leu Val Arg
            1               5                   10
cca ggg gcc tta gtc aag ttg tcc tgc aaa ggt tct ggc ttc aac att     144
Pro Gly Ala Leu Val Lys Leu Ser Cys Lys Gly Ser Gly Phe Asn Ile
    15                  20                  25
aaa gac tac tat atg cac tgg gtg aag cag agg cct gaa cag ggc ctg     192
Lys Asp Tyr Tyr Met His Trp Val Lys Gln Arg Pro Glu Gln Gly Leu
30                  35                  40                  45
gag tgg att gga ggg aat gat cct gcg aat ggt cat agt atg tat gac     240
Glu Trp Ile Gly Gly Asn Asp Pro Ala Asn Gly His Ser Met Tyr Asp
                50                  55                  60
ccg aaa ttc cag ggc aag gcc agt ata aca gca gac aca tcc tcc aac     288
Pro Lys Phe Gln Gly Lys Ala Ser Ile Thr Ala Asp Thr Ser Ser Asn
            65                  70                  75
aca gcc tac ctg cag ctc agc agc ctg aca tct gag gac act gcc gtc     336
Thr Ala Tyr Leu Gln Leu Ser Ser Leu Thr Ser Glu Asp Thr Ala Val
        80                  85                  90

tat ttc tgt gct aga gac tcg ggc tat gct atg gac tac tgg ggt caa 384
Tyr Phe Cys Ala Arg Asp Ser Gly Tyr Ala Met Asp Tyr Trp Gly Gln
95 100 105
gga acc tca gtc acc gtc tcc tca 408
Gly Thr Ser Val Thr Val Ser Ser
110 115
<210> 6
<211> 381
<212> DNA
<213> Mouse
<220>
<221> sig-peptide
<222> (1)...(60)
<220>
<221> mat-peptide
<222> (61)...(381)
<223> Nucleotide sequence coding for L chain V region of
anti-TF mouse monoclonal antibody ATR-5
<400> 6
atg agg gcc cct gct cag ttt ttt ggg atc ttg ttg ctc tgg ttt cca 48
Met Arg Ala Pro Ala Gln Phe Phe Gly Ile Leu Leu Leu Trp Phe Pro
-20 -15 -10 -5
ggt atc aga tgt gac atc aag atg acc cag tct cca tcc tct atg tat 96
Gly Ile Arg Cys Asp Ile Lys Met Thr Gln Ser Pro Ser Ser Met Tyr
1 5 10
gca tcg ctg gga gag aga gtc act atc act tgc aag gcg agt cag gac 144
Ala Ser Leu Gly Glu Arg Val Thr Ile Thr Cys Lys Ala Ser Gln Asp
15 20 25
att aaa agc ttt tta agt tgg tac cag caa aaa cca tgg aaa tct cct 192
Ile Lys Ser Phe Leu Ser Trp Tyr Gln Gln Lys Pro Trp Lys Ser Pro
30 35 40
aag acc ctg atc tat tat gca aca agc ttg gca gat ggg gtc cca tca 240
Lys Thr Leu Ile Tyr Tyr Ala Thr Ser Leu Ala Asp Gly Val Pro Ser
45 50 55 60
aga ttc agt ggc agt gga tct ggg caa gat tat tct cta acc atc aac 288

Arg Phe Ser Gly Ser Gly Ser Gly Gln Asp Tyr Ser Leu Thr Ile Asn
                65                  70                  75
aac ctg gag tct gac gat aca gca act tat tat tgt cta cag cat ggt     336
Asn Leu Glu Ser Asp Asp Thr Ala Thr Tyr Tyr Cys Leu Gln His Gly
            80                  85                  90
gag agc ccg tac acg ttc gga ggg ggg acc aag ctg gaa ata aaa         381
Glu Ser Pro Tyr Thr Phe Gly Gly Gly Thr Lys Leu Glu Ile Lys
        95                  100                 105
<210> 7
<211> 35
<212> DNA
<213> Artificial Sequence
<220>
<223> Primer ch5HS
<400> 7
gtctgtcgac ccaccatgaa atgcagctgg gtcat                              35
<210> 8
<211> 28
<212> DNA
<213> Artificial Sequence
<220>
<223> Primer ch5HA
<400> 8
tgttgctagc tgaggagacg gtgactga                                      28
<210> 9
<211> 35
<212> DNA
<213> Artificial Sequence
<220>
<223> Primer ch5LS
<400> 9
gtctagatct ccaccatgag ggcccctgct cagtt                              35
<210> 10
<211> 28
<212> DNA
<213> Artificial Sequence

<220>
<223> Primer ch5LA
<400> 10
tgttcgtacg ttttatttcc agcttggt 28
<210> 11
<211> 104
<212> DNA
<213> Artificial Sequence
<220>
<223> CDR grafting primer hR5Hv1S
<400> 11
ttctgtcgac ccaccatgaa atgcagctgg gtcatcttct tcctgatggc agtggttaca 60
ggggttaact cacaggtgca gctgttggag tctggagctg tgct 104
<210> 12
<211> 108
<212> DNA
<213> Artificial Sequence
<220>
<223> CDR grafting primer hR5Hv28
<400> 12
acaggtgcag ctgttggagt ctggagctgt gctggcaagg cctgggactt ccgtgaagat 60
ctcctgcaag gcttccggat tcaacattaa agactactat atgcattg 108
<210> 13
<211> 108
<212> DNA
<213> Artificial Sequence
<220>
<223> CDR grafting primer hR5Hv4S
<400> 13
gaatggccat agtatgtatg acccgaaatt ccagggcagg gccaaactga ctgcagccac 60
atccgccagt attgcctact tggagttctc gagcctgaca aatgagga 108
<210> 14
<211> 110
<212> DNA
<213> Artificial Sequence

<220>
<223> CDR grafting primer hR5Hv3A
<400> 14
tcatacatac tatggccatt cgcaggatca ttcccaccaa tccattctag accctgtcca 60
ggcctctgtt ttacccaatg catatagtag tctttaatgt tgaatccgga 110
<210> 15
<211> 110
<212> DNA
<213> Artificial Sequence
<220>
<223> CDR grafting primer hR5Hv5A
<400> 15
agaagctagc tgaggagacg gtgaccaggg tgccttggcc ccagtagtcc atggcatagc 60
ccgagtctct tgcacagtaa tagaccgcag aatcctcatt tgtcaggctc 110
<210> 16
<211> 19
<212> DNA
<213> Artificial Sequence
<220>
<223> Primer hR5HvPrS
<400> 16
ttctgtcgac ccaccatga 19
<210> 17
<211> 19
<212> DNA
<213> Artificial Sequence
<220>
<223> Primer hR5HvPrA
<400> 17
agaagctagc tgaggagac 19
<210> 18
<211> 415
<212> DNA
<213> Artificial Sequence
<220>

<221> sig-peptide
<222> (1)...(57)
<220>
<221> mat-peptide
<222> (58)...(415)
<223> Nucleotide sequence coding for version "a" of humanized
H chain V region
<400> 18
atg aaa tgc agc tgg gtc atc ttc ttc ctg atg gca gtg gtt aca ggg     48
Met Lys Cys Ser Trp Val Ile Phe Phe Leu Met Ala Val Val Thr Gly
                -15                 -10                 -5
gtt aac tca cag gtg cag ctg ttg gag tct gga gct gtg ctg gca agg     96
Val Asn Ser Gln Val Gln Leu Leu Glu Ser Gly Ala Val Leu Ala Arg
            1               5                   10
cct ggg act tcc gtg aag atc tcc tgc aag gct tcc gga ttc aac att    144
Pro Gly Thr Ser Val Lys Ile Ser Cys Lys Ala Ser Gly Phe Asn Ile
    15                  20                  25
aaa gac tac tat atg cat tgg gta aaa cag agg cct gga cag ggt cta    192
Lys Asp Tyr Tyr Met His Trp Val Lys Gln Arg Pro Gly Gln Gly Leu
30                  35                  40                  45
gaa tgg att ggt ggg aat gat cct gcg aat ggc cat agt atg tat gac    240
Glu Trp Ile Gly Gly Asn Asp Pro Ala Asn Gly His Ser Met Tyr Asp
                50                  55                  60
ccg aaa ttc cag ggc agg gcc aaa ctg act gca gcc aca tcc gcc agt    288
Pro Lys Phe Gln Gly Arg Ala Lys Leu Thr Ala Ala Thr Ser Ala Ser
            65                  70                  75
att gcc tac ttg gag ttc tcg agc ctg aca aat gag gat tct gcg gtc    336
Ile Ala Tyr Leu Glu Phe Ser Ser Leu Thr Asn Glu Asp Ser Ala Val
        80                  85                  90
tat tac tgt gca aga gac tcg ggc tat gcc atg gac tac tgg ggc caa    384
Tyr Tyr Cys Ala Arg Asp Ser Gly Tyr Ala Met Asp Tyr Trp Gly Gln
    95                  100                 105
ggc acc ctg gtc acc gtc tcc tca gct agc                            415
Gly Thr Leu Val Thr Val Ser Ser Ala Ser
110                 115
<210> 19
<211> 119

<212> PRT
<213> Artificial Sequence
<220>
<223> Amino acid sequence of version "a" of humanized H chain
V region
<400> 19
Gln Val Gln Leu Leu Glu Ser Gly Ala Val Leu Ala Arg Pro Gly Thr
1               5                   10                  15
Ser Val Lys Ile Ser Cys Lys Ala Ser Gly Phe Asn Ile Lys Asp Tyr
            20                  25                  30
Tyr Met His Trp Val Lys Gln Arg Pro Gly Gln Gly Leu Glu Trp Ile
        35                  40                  45
Gly Gly Asn Asp Pro Ala Asn Gly His Ser Met Tyr Asp Pro Lys Phe
    50                  55                  60
Gln Gly Arg Ala Lys Leu Thr Ala Ala Thr Ser Ala Ser Ile Ala Tyr
65                  70                  75                  80
Leu Glu Phe Ser Ser Leu Thr Asn Glu Asp Ser Ala Val Tyr Tyr Cys
                85                  90                  95
Ala Arg Asp Ser Gly Tyr Ala Met Asp Tyr Trp Gly Gln Gly Thr Leu
            100                 105                 110
Val Thr Val Ser Ser Ala Ser
        115
<210> 20
<211> 100
<212> DNA
<213> Artificial Sequence
<220>
<223> FR Shuffling primer F3RFFS
<400> 20
ttcttggcca tagtatgtat gacccgaaat tccagggccg agtcacaatc actgcagaca   60
catccacgaa cacagcctac atggagctct cgagtctgag                         100
<210> 21
<211> 75
<212> DNA
<213> Artificial Sequence
<220>

<223> FR Shuffling primer F3RFBS
<400> 21
ggagctctcg agtctgagat ctgaggacac agccatttat tactgtgcaa gagactcggg 60
ctatgccatg gttct 75
<210> 22
<211> 100
<212> DNA
<213> Artificial Sequence
<220>
<223> FR Shuffling primer F3RFFA
<400> 22
ctcagactcg agagctccat gtaggctgtg ttcgtggatg tgtctgcagt gattgtgact 60
cggccctgga atttcgggtc atacatacta tggccaagaa 100
<210> 23
<211> 75
<212> DNA
<213> Artificial Sequence
<220>
<223> FR Shuffling primer F3RFBA
<400> 23
agaaccatgg catagcccga gtctcttgca cagtaataaa tggctgtgtc ctcagatctc 60
agactcgaga gctcc 75
<210> 24
<211> 100
<212> DNA
<213> Artificial Sequence
<220>
<223> FR Shuffling primer F3NMFS
<400> 24
ttcttggcca tagtatgtat gacccgaaat tccagggccg agtcacaatg ctggtagaca 60
catccaagaa ccagttctcc ctgaggctct cgagtgtgac 100
<210> 25
<211> 75
<212> DNA
<213> Artificial Sequence

<220>
<223> FR Shuffling primer F3NMBS
<400> 25
gaggctctcg agtgtgacag ccgcggacac agccgtatat tactgtgcaa gagactcggg 60
ctatgccatg gttct 75
<210> 26
<211> 100
<212> DNA
<213> Artificial Sequence
<220>
<223> FR Shuffling primer F3NMFA
<400> 26
gtcacactcg agagcctcag ggagaactgg ttcttggatg tgtctaccag cattgtgact 60
cggccctgga atttcgggtc atacatacta tggccaagaa 100
<210> 27
<211> 75
<212> DNA
<213> Artificial Sequence
<220>
<223> FR Shuffling primer F3NMBA
<400> 27
agaaccatgg catagcccga gtctcttgca cagtaatata cggctgtgtc cgcggctgtc 60
acactcgaga gcctc 75
<210> 28
<211> 414
<212> DNA
<213> Artificial Sequence
<220>
<221> sig-peptide
<222> (1)...(57)
<220>
<221> mat-peptide
<222> (58)...(414)
<223> Nucleotide sequence coding for version "b" of humanized
H chain V region

<400> 28
atg aaa tgc agc tgg gtc atc ttc ttc ctg atg gca gtg gtt aca ggg     48
Met Lys Cys Ser Trp Val Ile Phe Phe Leu Met Ala Val Val Thr Gly
                -15                 -10                 -5
gtt aac tca cag gtg cag ctg ttg gag tct gga gct gtg ctg gca agg     96
Val Asn Ser Gln Val Gln Leu Leu Glu Ser Gly Ala Val Leu Ala Arg
            1               5                   10
cct ggg act tcc gtg aag atc tcc tgc aag gct tcc gga ttc aac att    144
Pro Gly Thr Ser Val Lys Ile Ser Cys Lys Ala Ser Gly Phe Asn Ile
    15                  20                  25
aaa gac tac tat atg cat tgg gta aaa cag agg cct gga cag ggt cta    192
Lys Asp Tyr Tyr Met His Trp Val Lys Gln Arg Pro Gly Gln Gly Leu
30                  35                  40                  45
gaa tgg att ggt ggg aat gat cct gcg aat ggc cat agt atg tat gac    240
Glu Trp Ile Gly Gly Asn Asp Pro Ala Asn Gly His Ser Met Tyr Asp
                50                  55                  60
ccg aaa ttc cag ggc cga gtc aca atc act gca gac aca tcc acg aac    288
Pro Lys Phe Gln Gly Arg Val Thr Ile Thr Ala Asp Thr Ser Thr Asn
            65                  70                  75
aca gcc tac atg gag ctc tcg agt ctg aga tct gag gac aca gcc att    336
Thr Ala Tyr Met Glu Leu Ser Ser Leu Arg Ser Glu Asp Thr Ala Ile
        80                  85                  90
tat tac tgt gca aga gac tcg ggc tat gcc atg gac tac tgg ggc caa    384
Tyr Tyr Cys Ala Arg Asp Ser Gly Tyr Ala Met Asp Tyr Trp Gly Gln
    95                  100                 105
ggc acc ctg gtc acc gtc tcc tca gct agc                            414
Gly Thr Leu Val Thr Val Ser Ser Ala Ser
110                 115
<210> 29
<211> 119
<212> PRT
<213> Artificial Sequence
<220>
<223> Amino acid sequence of version "b" of humanized H chain
V region
<400> 29

Gln Val Gln Leu Leu Glu Ser Gly Ala Val Leu Ala Arg Pro Gly Thr
1               5                   10                  15
Ser Val Lys Ile Ser Cys Lys Ala Ser Gly Phe Asn Ile Lys Asp Tyr
            20                  25                  30
Tyr Met His Trp Val Lys Gln Arg Pro Gly Gln Gly Leu Glu Trp Ile
        35                  40                  45
Gly Gly Asn Asp Pro Ala Asn Gly His Ser Met Tyr Asp Pro Lys Phe
    50                  55                  60
Gln Gly Arg Val Thr Ile Thr Ala Asp Thr Ser Thr Asn Thr Ala Tyr
65                  70                  75                  80
Met Glu Leu Ser Ser Leu Arg Ser Glu Asp Thr Ala Ile Tyr Tyr Cys
                85                  90                  95
Ala Arg Asp Ser Gly Tyr Ala Met Asp Tyr Trp Gly Gln Gly Thr Leu
            100                 105                 110
Val Thr Val Ser Ser Ala Ser
        115
<210> 30
<211> 414
<212> DNA
<213> Artificial Sequence
<220>
<221> sig-peptide
<222> (1)...(57)
<220>
<221> mat-peptide
<222> (58)...(414)
<223> Nucleotide sequence coding for version "c" of humanized
H chain V region
<400> 30
atg aaa tgc agc tgg gtc atc ttc ttc ctg atg gca gtg gtt aca ggg     48
Met Lys Cys Ser Trp Val Ile Phe Phe Leu Met Ala Val Val Thr Gly
                -15                 -10                 -5
gtt aac tca cag gtg cag ctg ttg gag tct gga gct gtg ctg gca agg     96
Val Asn Ser Gln Val Gln Leu Leu Glu Ser Gly Ala Val Leu Ala Arg
            1               5                   10
cct ggg act tcc gtg aag atc tcc tgc aag gct tcc gga ttc aac att    144

Pro Gly Thr Ser Val Lys Ile Ser Cys Lys Ala Ser Gly Phe Asn Ile
15 20 25
aaa gac tac tat atg cat tgg gta aaa cag agg cct gga cag ggt cta 192
Lys Asp Tyr Tyr Met His Trp Val Lys Gln Arg Pro Gly Gln Gly Leu
30 35 40 45
gaa tgg att ggt ggg aat gat cct gcg aat ggc cat agt atg tat gac 240
Glu Trp Ile Gly Gly Asn Asp Pro Ala Asn Gly His Ser Met Tyr Asp
    50                  55                  60
ccg aaa ttc cag ggc cga gtc aca atg ctg gta gac aca tcc aag aac 288
Pro Lys Phe Gln Gly Arg Val Thr Met Leu Val Asp Thr Ser Lys Asn
65 70 75
cag ttc tcc ctg agg ctc tcg agt gtg aca gcc gcg gac aca gcc gta 336
Gln Phe Ser Leu Arg Leu Ser Ser Val Thr Ala Ala Asp Thr Ala Val
80 85 90
tat tac tgt gca aga gac tcg ggc tat gcc atg gac tac tgg ggc caa 384
Tyr Tyr Cys Ala Arg Asp Ser Gly Tyr Ala Met Asp Tyr Trp Gly Gln
95 100 105
ggc acc ctg gtc acc gtc tcc tca gct agc 414
Gly Thr Leu Val Thr Val Ser Ser Ala Ser
110 115
<210> 31
<211> 119
<212> PRT
<213> Artificial Sequence
<220>
<223> Amino acid sequence of version "c" of humanized H chain
V region
<400> 31
Gln Val Gln Leu Leu Glu Ser Gly Ala Val Leu Ala Arg Pro Gly Thr
1               5                   10                  15
Ser Val Lys Ile Ser Cys Lys Ala Ser Gly Phe Asn Ile Lys Asp Tyr
            20                  25                  30
Tyr Met His Trp Val Lys Gln Arg Pro Gly Gln Gly Leu Glu Trp Ile
        35                  40                  45
Gly Gly Asn Asp Pro Ala Asn Gly His Ser Met Tyr Asp Pro Lys Phe
    50                  55                  60

Gln Gly Arg Val Thr Met Leu Val Asp Thr Ser Lys Asn Gln Phe Ser
65                  70                  75                  80
Leu Arg Leu Ser Ser Val Thr Ala Ala Asp Thr Ala Val Tyr Tyr Cys
                85                  90                  95
Ala Arg Asp Ser Gly Tyr Ala Met Asp Tyr Trp Gly Gln Gly Thr Leu
            100                 105                 110
Val Thr Val Ser Ser Ala Ser
        115
<210> 32
<211> 100
<212> DNA
<213> Artificial Sequence
<220>
<223> FR Shuffling primer F3EPS
<400> 32
ttcttggcca tagtatgtat gacccgaaat tccagggcag agtcacgatt actgcggacg 60
aatccacgag cacagcctac atggagctct cgagtctgag 100
<210> 33
<211> 75
<212> DNA
<213> Artificial Sequence
<220>
<223> FR Shuffling primer F3EPA
<400> 33
agaaccatgg catagcccga gtctctcgca cagaaatata cggccgagtc ctcagatctc 60
agactcgaga gctcc 75
<210> 34
<211> 20
<212> DNA
<213> Artificial Sequence
<220>
<223> Primer F3PrS
<400> 34
ttcttggcca tagtatgtat 20
<210> 35
<211> 18

<212> DNA
<213> Artificial Sequence
<220>
<223> Primer F3PrA
<400> 35
agaaccatgg catagccc 18
<210> 36
<211> 100
<212> DNA
<213> Artificial Sequence
<220>
<223> FR Shuffling primer F3vHS
<400> 36
ttcttggcca tagtatgtat gacccgaaat tccagggcag agtctcgatt accgcggacg 60
agtcaacgaa gatagcctac atggagctca acagtctgag 100
<210> 37
<211> 75
<212> DNA
<213> Artificial Sequence
<220>
<223> FR Shuffling primer F3vHA
<400> 37
agaaccatgg catagcccga gtctctcgca cagaaataaa cggccgtgtc ctcagatctc 60
agactgttga gctcc 75
<210> 38
<211> 414
<212> DNA
<213> Artificial Sequence
<220>
<221> sig-peptide
<222> (1)...(57)
<220>
<221> mat-peptide
<222> (58)...(414)
<223> Nucleotide sequence coding for version "d" of humanized

H chain V region
<400> 38
atg aaa tgc agc tgg gtc atc ttc ttc ctg atg gca gtg gtt aca ggg     48
Met Lys Cys Ser Trp Val Ile Phe Phe Leu Met Ala Val Val Thr Gly
                -15                 -10                 -5
gtt aac tca cag gtg cag ctg ttg gag tct gga gct gtg ctg gca agg     96
Val Asn Ser Gln Val Gln Leu Leu Glu Ser Gly Ala Val Leu Ala Arg
              1               5                  10
cct ggg act tcc gtg aag atc tcc tgc aag gct tcc gga ttc aac att    144
Pro Gly Thr Ser Val Lys Ile Ser Cys Lys Ala Ser Gly Phe Asn Ile
     15                  20                  25
aaa gac tac tat atg cat tgg gta aaa cag agg cct gga cag ggt cta    192
Lys Asp Tyr Tyr Met His Trp Val Lys Gln Arg Pro Gly Gln Gly Leu
 30                  35                  40                  45
gaa tgg att ggt ggg aat gat cct gcg aat ggc cat agt atg tat gac    240
Glu Trp Ile Gly Gly Asn Asp Pro Ala Asn Gly His Ser Met Tyr Asp
                 50                  55                  60
ccg aaa ttc cag ggc aga gtc acg att act gcg gac gaa tcc acg agc    288
Pro Lys Phe Gln Gly Arg Val Thr Ile Thr Ala Asp Glu Ser Thr Ser
             65                  70                  75
aca gcc tac atg gag ctc tcg agt ctg aga tct gag gac tcg gcc gta    336
Thr Ala Tyr Met Glu Leu Ser Ser Leu Arg Ser Glu Asp Ser Ala Val
         80                  85                  90
tat ttc tgt gcg aga gac tcg ggc tat gcc atg gac tac tgg ggc caa    384
Tyr Phe Cys Ala Arg Asp Ser Gly Tyr Ala Met Asp Tyr Trp Gly Gln
     95                 100                 105
ggc acc ctg gtc acc gtc tcc tca gct agc                            414
Gly Thr Leu Val Thr Val Ser Ser Ala Ser
110                 115
<210> 39
<211> 119
<212> PRT
<213> Artificial Sequence
<220>
<223> Amino acid sequence of version "d" of humanized H chain
<400> 39

Gln Val Gln Leu Leu Glu Ser Gly Ala Val Leu Ala Arg Pro Gly Thr
1               5                   10                  15
Ser Val Lys Ile Ser Cys Lys Ala Ser Gly Phe Asn Ile Lys Asp Tyr
            20                  25                  30
Tyr Met His Trp Val Lys Gln Arg Pro Gly Gln Gly Leu Glu Trp Ile
        35                  40                  45
Gly Gly Asn Asp Pro Ala Asn Gly His Ser Met Tyr Asp Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Ser Ala Val Tyr Phe Cys
                85                  90                  95
Ala Arg Asp Ser Gly Tyr Ala Met Asp Tyr Trp Gly Gln Gly Thr Leu
            100                 105                 110
Val Thr Val Ser Ser Ala Ser
        115
<210> 40
<211> 414
<212> DNA
<213> Artificial Sequence
<220>
<221> sig-peptide
<222> (1)...(57)
<220>
<221> mat-peptide
<222> (58)...(414)
<223> Nucleotide sequence coding for version "e" of humanized
H chain V region
<400> 40
atg aaa tgc agc tgg gtc atc ttc ttc ctg atg gca gtg gtt aca ggg     48
Met Lys Cys Ser Trp Val Ile Phe Phe Leu Met Ala Val Val Thr Gly
                -15                 -10                 -5
gtt aac tca cag gtg cag ctg ttg gag tct gga gct gtg ctg gca agg     96
Val Asn Ser Gln Val Gln Leu Leu Glu Ser Gly Ala Val Leu Ala Arg
            1               5                   10
cct ggg act tcc gtg aag atc tcc tgc aag gct tcc gga ttc aac att    144

Pro Gly Thr Ser Val Lys Ile Ser Cys Lys Ala Ser Gly Phe Asn Ile
15                  20                  25
aaa gac tac tat atg cat tgg gta aaa cag agg cct gga cag ggt cta    192
Lys Asp Tyr Tyr Met His Trp Val Lys Gln Arg Pro Gly Gln Gly Leu
30                  35                  40                  45
gaa tgg att ggt ggg aat gat cct gcg aat ggc cat agt atg tat gac    240
Glu Trp Ile Gly Gly Asn Asp Pro Ala Asn Gly His Ser Met Tyr Asp
                50                  55                  60
ccg aaa ttc cag ggc aga gtc tcg att acc gcg gac gag tca acg aag    288
Pro Lys Phe Gln Gly Arg Val Ser Ile Thr Ala Asp Glu Ser Thr Lys
            65                  70                  75
ata gcc tac atg gag ctc aac agt ctg aga tct gag gac acg gcc gtt    336
Ile Ala Tyr Met Glu Leu Asn Ser Leu Arg Ser Glu Asp Thr Ala Val
        80                  85                  90
tat ttc tgt gcg aga gac tcg ggc tat gcc atg gac tac tgg ggc caa    384
Tyr Phe Cys Ala Arg Asp Ser Gly Tyr Ala Met Asp Tyr Trp Gly Gln
    95                  100                 105
ggc acc ctg gtc acc gtc tcc tca gct agc                            414
Gly Thr Leu Val Thr Val Ser Ser Ala Ser
110                 115
<210> 41
<211> 119
<212> PRT
<213> Artificial Sequence
<220>
<223> Amino acid sequence of version "e" of humanized H chain
V region
<400> 41
Gln Val Gln Leu Leu Glu Ser Gly Ala Val Leu Ala Arg Pro Gly Thr
 1               5                  10                  15
Ser Val Lys Ile Ser Cys Lys Ala Ser Gly Phe Asn Ile Lys Asp Tyr
            20                  25                  30
Tyr Met His Trp Val Lys Gln Arg Pro Gly Gln Gly Leu Glu Trp Ile
        35                  40                  45
Gly Gly Asn Asp Pro Ala Asn Gly His Ser Met Tyr Asp Pro Lys Phe
    50                  55                  60

Gln Gly Arg Val Ser Ile Thr Ala Asp Glu Ser Thr Lys Ile Ala Tyr
65                  70                  75                  80
Met Glu Leu Asn Ser Leu Arg Ser Glu Asp Thr Ala Val Tyr Phe Cys
                85                  90                  95
Ala Arg Asp Ser Gly Tyr Ala Met Asp Tyr Trp Gly Gln Gly Thr Leu
            100                 105                 110
Val Thr Val Ser Ser Ala Ser
        115
<210> 42
<211> 100
<212> DNA
<213> Artificial Sequence
<220>
<223> FR Shuffling primer F3SSS
<400> 42
ttcttggcca tagtatgtat gacccgaaat tccagggcag agtcacgatt accgcggaca   60
catccacgag cacagcctac atggagctca ggagcctgag                         100
<210> 43
<211> 75
<212> DNA
<213> Artificial Sequence
<220>
<223> FR Shuffling primer F3SSA
<400> 43
agaaccatgg catagcccga gtctctcgca cagtaataca cggccgtgtc gtcagatctc   60
aggctcctga gctcc                                                    75
<210> 44
<211> 100
<212> DNA
<213> Artificial Sequence
<220>
<223> FR Shuffling primer F3CDS
<400> 44
ttcttggcca tagtatgtat gacccgaaat tccagggcaa agccactctg actgcagacg   60
aatcctccag cacagcctac atgcaactct cgagcctacg                         100
<210> 45

<211> 75
<212> DNA
<213> Artificial Sequence
<220>
<223> FR Shuffling primer F3CDA
<400> 45
agaaccatgg catagcccga gtctcttgca caagaataga ccgcagagtc ctcagatcgt 60
aggctcgaga gttgc 75
<210> 46
<211> 414
<212> DNA
<213> Artificial Sequence
<220>
<221> sig-peptide
<222> (1)...(57)
<220>
<221> mat-peptide
<222> (58)...(414)
<223> Nucleotide sequence coding for version "f" of humanized
H chain V region
<400> 46
atg aaa tgc agc tgg gtc atc ttc ttc ctg atg gca gtg gtt aca ggg 48
Met Lys Cys Ser Trp Val Ile Phe Phe Leu Met Ala Val Val Thr Gly
-15 -10 -5
gtt aac tca cag gtg cag ctg ttg gag tct gga gct gtg ctg gca agg 96
Val Asn Ser Gln Val Gln Leu Leu Glu Ser Gly Ala Val Leu Ala Arg
1 5 10
cct ggg act tcc gtg aag atc tcc tgc aag gct tcc gga ttc aac att 144
Pro Gly Thr Ser Val Lys Ile Ser Cys Lys Ala Ser Gly Phe Asn Ile
15 20 25
aaa gac tac tat atg cat tgg gta aaa cag agg cct gga cag ggt cta 192
Lys Asp Tyr Tyr Met His Trp Val Lys Gln Arg Pro Gly Gln Gly Leu
30 35 40 45
gaa tgg att ggt ggg aat gat cct gcg aat ggc cat agt atg tat gac 240
Glu Trp Ile Gly Gly Asn Asp Pro Ala Asn Gly His Ser Met Tyr Asp
    50                  55                  60

ccg aaa ttc cag ggc aga gtc acg att acc gcg gac aca tcc acg agc 288
Pro Lys Phe Gln Gly Arg Val Thr Ile Thr Ala Asp Thr Ser Thr Ser
65 70 75
aca gcc tac atg gag ctc agg agc ctg aga tct gac gac acg gcc gtg 336
Thr Ala Tyr Met Glu Leu Arg Ser Leu Arg Ser Asp Asp Thr Ala Val
80 85 90
tat tac tgt gcg aga gac tcg ggc tat gcc atg gac tac tgg ggc caa 384
Tyr Tyr Cys Ala Arg Asp Ser Gly Tyr Ala Met Asp Tyr Trp Gly Gln
95 100 105
ggc acc ctg gtc acc gtc tcc tca gct agc 414
Gly Thr Leu Val Thr Val Ser Ser Ala Ser
110 115
<210> 47
<211> 119
<212> PRT
<213> Artificial Sequence
<220>
<223> Amino acid sequence of version "f" of humanized H chain
V region
<400> 47
Gln Val Gln Leu Leu Glu Ser Gly Ala Val Leu Ala Arg Pro Gly Thr
1               5                   10                  15
Ser Val Lys Ile Ser Cys Lys Ala Ser Gly Phe Asn Ile Lys Asp Tyr
            20                  25                  30
Tyr Met His Trp Val Lys Gln Arg Pro Gly Gln Gly Leu Glu Trp Ile
        35                  40                  45
Gly Gly Asn Asp Pro Ala Asn Gly His Ser Met Tyr Asp Pro Lys Phe
    50                  55                  60
Gln Gly Arg Val Thr Ile Thr Ala Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Asp Ser Gly Tyr Ala Met Asp Tyr Trp Gly Gln Gly Thr Leu
            100                 105                 110
Val Thr Val Ser Ser Ala Ser
        115
<210> 48

<211> 414
<212> DNA
<213> Artificial Sequence
<220>
<221> sig-peptide
<222> (1)...(57)
<220>
<221> mat-peptide
<222> (58)...(414)
<223> Nucleotide sequence coding for version "g" of humanized
H chain V region
<400> 48
atg aaa tgc agc tgg gtc atc ttc ttc ctg atg gca gtg gtt aca ggg 48
Met Lys Cys Ser Trp Val Ile Phe Phe Leu Met Ala Val Val Thr Gly
-15 -10 -5
gtt aac tca cag gtg cag ctg ttg gag tct gga gct gtg ctg gca agg 96
Val Asn Ser Gln Val Gln Leu Leu Glu Ser Gly Ala Val Leu Ala Arg
1 5 10
cct ggg act tcc gtg aag atc tcc tgc aag gct tcc gga ttc aac att 144
Pro Gly Thr Ser Val Lys Ile Ser Cys Lys Ala Ser Gly Phe Asn Ile
15 20 25
aaa gac tac tat atg cat tgg gta aaa cag agg cct gga cag ggt cta 192
Lys Asp Tyr Tyr Met His Trp Val Lys Gln Arg Pro Gly Gln Gly Leu
30 35 40 45
gaa tgg att ggt ggg aat gat cct gcg aat ggc cat agt atg tat gac 240
Glu Trp Ile Gly Gly Asn Asp Pro Ala Asn Gly His Ser Met Tyr Asp
    50                  55                  60
ccg aaa ttc cag ggc aaa gcc act ctg act gca gac gaa tcc tcc agc 288
Pro Lys Phe Gln Gly Lys Ala Thr Leu Thr Ala Asp Glu Ser Ser Ser
65 70 75
aca gcc tac atg caa ctc tcg agc cta cga tct gag gac tct gcg gtc 336
Thr Ala Tyr Met Gln Leu Ser Ser Leu Arg Ser Glu Asp Ser Ala Val
80 85 90
tat tct tgt gca aga gac tcg ggc tat gcc atg gac tac tgg ggc caa 384
Tyr Ser Cys Ala Arg Asp Ser Gly Tyr Ala Met Asp Tyr Trp Gly Gln
95 100 105

ggc acc ctg gtc acc gtc tcc tca gct agc 414
Gly Thr Leu Val Thr Val Ser Ser Ala Ser
110 115
<210> 49
<211> 119
<212> PRT
<213> Artificial Sequence
<220>
<223> Amino acid sequence of version "g" of humanized H chain
V region
<400> 49
Gln Val Gln Leu Leu Glu Ser Gly Ala Val Leu Ala Arg Pro Gly Thr
1               5                   10                  15
Ser Val Lys Ile Ser Cys Lys Ala Ser Gly Phe Asn Ile Lys Asp Tyr
            20                  25                  30
Tyr Met His Trp Val Lys Gln Arg Pro Gly Gln Gly Leu Glu Trp Ile
        35                  40                  45
Gly Gly Asn Asp Pro Ala Asn Gly His Ser Met Tyr Asp Pro Lys Phe
    50                  55                  60
Gln Gly Lys Ala Thr Leu Thr Ala Asp Glu Ser Ser Ser Thr Ala Tyr
65                  70                  75                  80
Met Gln Leu Ser Ser Leu Arg Ser Glu Asp Ser Ala Val Tyr Ser Cys
                85                  90                  95
Ala Arg Asp Ser Gly Tyr Ala Met Asp Tyr Trp Gly Gln Gly Thr Leu
            100                 105                 110
Val Thr Val Ser Ser Ala Ser
        115
<210> 50
<211> 100
<212> DNA
<213> Artificial Sequence
<220>
<223> FR Shuffling primer F3ADS
<400> 50
ttcttggcca tagtatgtat gacccgaaat tccagggccg cgtcaccatg tcagccgaca 60
agtcctccag cgccgcctat ttacagtgga ccagccttaa 100

<210> 51
<211> 75
<212> DNA
<213> Artificial Sequence
<220>
<223> FR Shuffling primer F3ADA
<400> 51
agaaccatgg catagcccga gtctctcgcg cagaaatata tggcggtgtc cgaggcctta 60
aggctggtcc actgt 75
<210> 52
<211> 414
<212> DNA
<213> Artificial Sequence
<220>
<221> sig-peptide
<222> (1)...(57)
<220>
<221> mat-peptide
<222> (58)...(414)
<223> Nucleotide sequence coding for version "h" of humanized
H chain
<400> 52
atg aaa tgc agc tgg gtc atc ttc ttc ctg atg gca gtg gtt aca ggg 48
Met Lys Cys Ser Trp Val Ile Phe Phe Leu Met Ala Val Val Thr Gly
-15 -10 -5
gtt aac tca cag gtg cag ctg ttg gag tct gga gct gtg ctg gca agg 96
Val Asn Ser Gln Val Gln Leu Leu Glu Ser Gly Ala Val Leu Ala Arg
1 5 10
cct ggg act tcc gtg aag atc tcc tgc aag gct tcc gga ttc aac att 144
Pro Gly Thr Ser Val Lys Ile Ser Cys Lys Ala Ser Gly Phe Asn Ile
15 20 25
aaa gac tac tat atg cat tgg gta aaa cag agg cct gga cag ggt cta 192
Lys Asp Tyr Tyr Met His Trp Val Lys Gln Arg Pro Gly Gln Gly Leu
30 35 40 45
gaa tgg att ggt ggg aat gat cct gcg aat ggc cat agt atg tat gac 240

Glu Trp Ile Gly Gly Asn Asp Pro Ala Asn Gly His Ser Met Tyr Asp
    50                  55                  60
ccg aaa ttc cag ggc cgc gtc acc atg tca gcc gac aag tcc tcc agc 288
Pro Lys Phe Gln Gly Arg Val Thr Met Ser Ala Asp Lys Ser Ser Ser
65 70 75
gcc gcc tat tta cag tgg acc agc ctt aag gcc tcg gac acc gcc ata 336
Ala Ala Tyr Leu Gln Trp Thr Ser Leu Lys Ala Ser Asp Thr Ala Ile
80 85 90
tat ttc tgc gcg aga gac tcg ggc tat gcc atg gac tac tgg ggc caa 384
Tyr Phe Cys Ala Arg Asp Ser Gly Tyr Ala Met Asp Tyr Trp Gly Gln
95 100 105
ggc acc ctg gtc acc gtc tcc tca gct agc 414
Gly Thr Leu Val Thr Val Ser Ser Ala Ser
110 115
<210> 53
<211> 119
<212> PRT
<213> Artificial Sequence
<220>
<223> Amino acid sequence of version "h" of humanized H chain
V region
<400> 53
Gln Val Gln Leu Leu Glu Ser Gly Ala Val Leu Ala Arg Pro Gly Thr
1               5                   10                  15
Ser Val Lys Ile Ser Cys Lys Ala Ser Gly Phe Asn Ile Lys Asp Tyr
            20                  25                  30
Tyr Met His Trp Val Lys Gln Arg Pro Gly Gln Gly Leu Glu Trp Ile
        35                  40                  45
Gly Gly Asn Asp Pro Ala Asn Gly His Ser Met Tyr Asp Pro Lys Phe
    50                  55                  60
Gln Gly Arg Val Thr Met Ser Ala Asp Lys Ser Ser Ser Ala Ala Tyr
65                  70                  75                  80
Leu Gln Trp Thr Ser Leu Lys Ala Ser Asp Thr Ala Ile Tyr Phe Cys
                85                  90                  95
Ala Arg Asp Ser Gly Tyr Ala Met Asp Tyr Trp Gly Gln Gly Thr Leu
            100                 105                 110

Val Thr Val Ser Ser Ala Ser
        115
<210> 54
<211> 100
<212> DNA
<213> Artificial Sequence
<220>
<223> FR Shuffling primer F3MMS
<400> 54
ttcttggcca tagtatgtat gacccgaaat tccagggcag agtcacgatt accgcggaca   60
catcgacgag cacagtcttc atggaactga gcagcctgag                         100
<210> 55
<211> 75
<212> DNA
<213> Artificial Sequence
<220>
<223> FR Shuffling primer F3MMA
<400> 55
agaaccatgg catagcccga gtctctcgca cagtaataca cggccgtgtc ttcagatctc   60
aggctgctca gttcc                                                    75
<210> 56
<211> 100
<212> DNA
<213> Artificial Sequence
<220>
<223> FR Shuffling primer F3BMS
<400> 56
ttcttggcca tagtatgtat gacccgaaat tccagggcag agtcaccttt accgcggaca   60
catccgcgaa cacagcctac atggagttga ggagcctcag                         100
<210> 57
<211> 75
<212> DNA
<213> Artificial Sequence
<220>
<223> FR Shuffling primer F3BMA

<400> 57
agaaccatgg catagcccga gtctctcgca caataataaa cagccgtgtc tgcagatctg 60
aggctcctca actcc 75
<210> 58
<211> 414
<212> DNA
<213> Artificial Sequence
<220>
<221> sig-peptide
<222> (1)...(57)
<220>
<221> mat-peptide
<222> (58)...(414)
<223> Nucleotide sequence coding for version "i" of humanized
H chain V region
<400> 58
atg aaa tgc agc tgg gtc atc ttc ttc ctg atg gca gtg gtt aca ggg 48
Met Lys Cys Ser Trp Val Ile Phe Phe Leu Met Ala Val Val Thr Gly
-15 -10 -5
gtt aac tca cag gtg cag ctg ttg gag tct gga gct gtg ctg gca agg 96
Val Asn Ser Gln Val Gln Leu Leu Glu Ser Gly Ala Val Leu Ala Arg
1 5 10
cct ggg act tcc gtg aag atc tcc tgc aag gct tcc gga ttc aac att 144
Pro Gly Thr Ser Val Lys Ile Ser Cys Lys Ala Ser Gly Phe Asn Ile
15 20 25
aaa gac tac tat atg cat tgg gta aaa cag agg cct gga cag ggt cta 192
Lys Asp Tyr Tyr Met His Trp Val Lys Gln Arg Pro Gly Gln Gly Leu
30 35 40 45
gaa tgg att ggt ggg aat gat cct gcg aat ggc cat agt atg tat gac 240
Glu Trp Ile Gly Gly Asn Asp Pro Ala Asn Gly His Ser Met Tyr Asp
    50                  55                  60
ccg aaa ttc cag ggc aga gtc acg att acc gcg gac aca tcg acg agc 288
Pro Lys Phe Gln Gly Arg Val Thr Ile Thr Ala Asp Thr Ser Thr Ser
65 70 75
aca gtc ttc atg gaa ctg agc agc ctg aga tct gaa gac acg gcc gtg 336

Thr Val Phe Met Glu Leu Ser Ser Leu Arg Ser Glu Asp Thr Ala Val
80 85 90
tat tac tgt gcg aga gac tcg ggc tat gcc atg gac tac tgg ggc caa 384
Tyr Tyr Cys Ala Arg Asp Ser Gly Tyr Ala Met Asp Tyr Trp Gly Gln
95 100 105
ggc acc ctg gtc acc gtc tcc tca gct agc 414
Gly Thr Leu Val Thr Val Ser Ser Ala Ser
110 115
<210> 59
<211> 119
<212> PRT
<213> Artificial Sequence
<220>
<223> Amino acid sequence of version "i" of humanized H chain
V region
<400> 59
Gln Val Gln Leu Leu Glu Ser Gly Ala Val Leu Ala Arg Pro Gly Thr
1               5                   10                  15
Ser Val Lys Ile Ser Cys Lys Ala Ser Gly Phe Asn Ile Lys Asp Tyr
            20                  25                  30
Tyr Met His Trp Val Lys Gln Arg Pro Gly Gln Gly Leu Glu Trp Ile
        35                  40                  45
Gly Gly Asn Asp Pro Ala Asn Gly His Ser Met Tyr Asp Pro Lys Phe
    50                  55                  60
Gln Gly Arg Val Thr Ile Thr Ala Asp Thr Ser Thr Ser Thr Val Phe
65                  70                  75                  80
Met Glu Leu Ser Ser Leu Arg Ser Glu Asp Thr Ala Val Tyr Tyr Cys
                85                  90                  95
Ala Arg Asp Ser Gly Tyr Ala Met Asp Tyr Trp Gly Gln Gly Thr Leu
            100                 105                 110
Val Thr Val Ser Ser Ala Ser
        115
<210> 60
<211> 414
<212> DNA
<213> Artificial Sequence

<220>
<221> sig-peptide
<222> (1)...(57)
<220>
<221> mat-peptide
<222> (58)...(414)
<223> Nucleotide sequence coding for version "j" of humanized
H chain V region
<400> 60
atg aaa tgc agc tgg gtc atc ttc ttc ctg atg gca gtg gtt aca ggg 48
Met Lys Cys Ser Trp Val Ile Phe Phe Leu Met Ala Val Val Thr Gly
-15 -10 -5
gtt aac tca cag gtg cag ctg ttg gag tct gga gct gtg ctg gca agg 96
Val Asn Ser Gln Val Gln Leu Leu Glu Ser Gly Ala Val Leu Ala Arg
1 5 10
cct ggg act tcc gtg aag atc tcc tgc aag gct tcc gga ttc aac att 144
Pro Gly Thr Ser Val Lys Ile Ser Cys Lys Ala Ser Gly Phe Asn Ile
15 20 25
aaa gac tac tat atg cat tgg gta aaa cag agg cct gga cag ggt cta 192
Lys Asp Tyr Tyr Met His Trp Val Lys Gln Arg Pro Gly Gln Gly Leu
30 35 40 45
gaa tgg att ggt ggg aat gat cct gcg aat ggc cat agt atg tat gac 240
Glu Trp Ile Gly Gly Asn Asp Pro Ala Asn Gly His Ser Met Tyr Asp
    50                  55                  60
ccg aaa ttc cag ggc aga gtc acc ttt acc gcg gac aca tcc gcg aac 288
Pro Lys Phe Gln Gly Arg Val Thr Phe Thr Ala Asp Thr Ser Ala Asn
65 70 75
aca gcc tac atg gag ttg agg agc ctc aga tct gca gac acg gct gtt 336
Thr Ala Tyr Met Glu Leu Arg Ser Leu Arg Ser Ala Asp Thr Ala Val
80 85 90
tat tat tgt gcg aga gac tcg ggc tat gcc atg gac tac tgg ggc caa 384
Tyr Tyr Cys Ala Arg Asp Ser Gly Tyr Ala Met Asp Tyr Trp Gly Gln
95 100 105
ggc acc ctg gtc acc gtc tcc tca gct agc 414
Gly Thr Leu Val Thr Val Ser Ser Ala Ser
110 115
<210> 61

<211> 119
<212> PRT
<213> Artificial Sequence
<220>
<223> Amino acid sequence of version "j" of humanized H chain
V region
<400> 61
Gln Val Gln Leu Leu Glu Ser Gly Ala Val Leu Ala Arg Pro Gly Thr
1               5                   10                  15
Ser Val Lys Ile Ser Cys Lys Ala Ser Gly Phe Asn Ile Lys Asp Tyr
            20                  25                  30
Tyr Met His Trp Val Lys Gln Arg Pro Gly Gln Gly Leu Glu Trp Ile
        35                  40                  45
Gly Gly Asn Asp Pro Ala Asn Gly His Ser Met Tyr Asp Pro Lys Phe
    50                  55                  60
Gln Gly Arg Val Thr Phe Thr Ala Asp Thr Ser Ala Asn Thr Ala Tyr
65                  70                  75                  80
Met Glu Leu Arg Ser Leu Arg Ser Ala Asp Thr Ala Val Tyr Tyr Cys
                85                  90                  95
Ala Arg Asp Ser Gly Tyr Ala Met Asp Tyr Trp Gly Gln Gly Thr Leu
            100                 105                 110
Val Thr Val Ser Ser Ala Ser
        115
<210> 62
<211> 79
<212> DNA
<213> Artificial Sequence
<220>
<223> FR shuffling primer F2MPS
<400> 62
ttctatgcat tgggtgcgcc aggctccagg acagggcctg gagtggatgg gagggaatga    60
tcctgcgaat ggccattct                                                 79
<210> 63
<211> 79
<212> DNA
<213> Artificial Sequence

<220>
<223> FR shuffling primer F2MPA
<400> 63
agaatggcca ttcgcaggat cattccctcc catccactcc aggccctgtc ctggagcctg 60
gcgcacccaa tgcatagaa 79
<210> 64
<211> 414
<212> DNA
<213> Artificial Sequence
<220>
<221> sig-peptide
<222> (1)...(57)
<220>
<221> mat-peptide
<222> (58)...(414)
<223> Nucleotide sequence coding for version "b1" of
humanized H chain V region
<400> 64
atg aaa tgc agc tgg gtc atc ttc ttc ctg atg gca gtg gtt aca ggg 48
Met Lys Cys Ser Trp Val Ile Phe Phe Leu Met Ala Val Val Thr Gly
-15 -10 -5
gtt aac tca cag gtg cag ctg ttg gag tct gga gct gtg ctg gca agg 96
Val Asn Ser Gln Val Gln Leu Leu Glu Ser Gly Ala Val Leu Ala Arg
1 5 10
cct ggg act tcc gtg aag atc tcc tgc aag gct tcc gga ttc aac att 144
Pro Gly Thr Ser Val Lys Ile Ser Cys Lys Ala Ser Gly Phe Asn Ile
15 20 25
aaa gac tac tat atg cat tgg gtg cgc cag gct cca gga cag ggc ctg 192
Lys Asp Tyr Tyr Met His Trp Val Arg Gln Ala Pro Gly Gln Gly Leu
30 35 40 45
gag tgg atg gga ggg aat gat cct gcg aat ggc cat agt atg tat gac 240
Glu Trp Met Gly Gly Asn Asp Pro Ala Asn Gly His Ser Met Tyr Asp
    50                  55                  60
ccg aaa ttc cag ggc cga gtc aca atc act gca gac aca tcc acg aac 288
Pro Lys Phe Gln Gly Arg Val Thr Ile Thr Ala Asp Thr Ser Thr Asn
65 70 75

aca gcc tac atg gag ctc tcg agt ctg aga tct gag gac aca gcc att 336
Thr Ala Tyr Met Glu Leu Ser Ser Leu Arg Ser Glu Asp Thr Ala Ile
80 85 90
tat tac tgt gca aga gac tcg ggc tat gcc atg gac tac tgg ggc caa 384
Tyr Tyr Cys Ala Arg Asp Ser Gly Tyr Ala Met Asp Tyr Trp Gly Gln
95 100 105
ggc acc ctg gtc acc gtc tcc tca gct agc 414
Gly Thr Leu Val Thr Val Ser Ser Ala Ser
110 115
<210> 65
<211> 119
<212> PRT
<213> Artificial Sequence
<220>
<223> Amino acid sequence of version "b1" of humanized H
chain V region
<400> 65
Gln Val Gln Leu Leu Glu Ser Gly Ala Val Leu Ala Arg Pro Gly Thr
1               5                   10                  15
Ser Val Lys Ile Ser Cys Lys Ala Ser Gly Phe Asn Ile Lys Asp Tyr
            20                  25                  30
Tyr Met His Trp Val Arg Gln Ala Pro Gly Gln Gly Leu Glu Trp Met
        35                  40                  45
Gly Gly Asn Asp Pro Ala Asn Gly His Ser Met Tyr Asp Pro Lys Phe
    50                  55                  60
Gln Gly Arg Val Thr Ile Thr Ala Asp Thr Ser Thr Asn Thr Ala Tyr
65                  70                  75                  80
Met Glu Leu Ser Ser Leu Arg Ser Glu Asp Thr Ala Ile Tyr Tyr Cys
                85                  90                  95
Ala Arg Asp Ser Gly Tyr Ala Met Asp Tyr Trp Gly Gln Gly Thr Leu
            100                 105                 110
Val Thr Val Ser Ser Ala Ser
        115
<210> 66
<211> 414
<212> DNA

<213> Artificial Sequence
<220>
<221> sig-peptide
<222> (1)...(57)
<220>
<221> mat-peptide
<222> (58)...(414)
<223> Nucleotide sequence coding for version "d1" of
humanized H chain V region
<400> 66
atg aaa tgc agc tgg gtc atc ttc ttc ctg atg gca gtg gtt aca ggg 48
Met Lys Cys Ser Trp Val Ile Phe Phe Leu Met Ala Val Val Thr Gly
-15 -10 -5
gtt aac tca cag gtg cag ctg ttg gag tct gga gct gtg ctg gca agg 96
Val Asn Ser Gln Val Gln Leu Leu Glu Ser Gly Ala Val Leu Ala Arg
1 5 10
cct ggg act tcc gtg aag atc tcc tgc aag gct tcc gga ttc aac att 144
Pro Gly Thr Ser Val Lys Ile Ser Cys Lys Ala Ser Gly Phe Asn Ile
15 20 25
aaa gac tac tat atg cat tgg gtg cgc cag gct cca gga cag ggc ctg 192
Lys Asp Tyr Tyr Met His Trp Val Arg Gln Ala Pro Gly Gln Gly Leu
30 35 40 45
gag tgg atg gga ggg aat gat cct gcg aat ggc cat agt atg tat gac 240
Glu Trp Met Gly Gly Asn Asp Pro Ala Asn Gly His Ser Met Tyr Asp
    50                  55                  60
ccg aaa ttc cag ggc aga gtc acg att act gcg gac gaa tcc acg agc 288
Pro Lys Phe Gln Gly Arg Val Thr Ile Thr Ala Asp Glu Ser Thr Ser
65 70 75
aca gcc tac atg gag ctc tcg agt ctg aga tct gag gac tcg gcc gta 336
Thr Ala Tyr Met Glu Leu Ser Ser Leu Arg Ser Glu Asp Ser Ala Val
80 85 90
tat ttc tgt gcg aga gac tcg ggc tat gcc atg gac tac tgg ggc caa 384
Tyr Phe Cys Ala Arg Asp Ser Gly Tyr Ala Met Asp Tyr Trp Gly Gln
95 100 105
ggc acc ctg gtc acc gtc tcc tca gct agc 414
Gly Thr Leu Val Thr Val Ser Ser Ala Ser
110 115

<210> 67
<211> 119
<212> PRT
<213> Artificial Sequence
<220>
<223> Amino acid sequence of version "d1" of humanized H
chain V region
<400> 67
Gln Val Gln Leu Leu Glu Ser Gly Ala Val Leu Ala Arg Pro Gly Thr
1               5                   10                  15
Ser Val Lys Ile Ser Cys Lys Ala Ser Gly Phe Asn Ile Lys Asp Tyr
            20                  25                  30
Tyr Met His Trp Val Arg Gln Ala Pro Gly Gln Gly Leu Glu Trp Met
        35                  40                  45
Gly Gly Asn Asp Pro Ala Asn Gly His Ser Met Tyr Asp Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Ser Ala Val Tyr Phe Cys
                85                  90                  95
Ala Arg Asp Ser Gly Tyr Ala Met Asp Tyr Trp Gly Gln Gly Thr Leu
            100                 105                 110
Val Thr Val Ser Ser Ala Ser
        115
<210> 68
<211> 79
<212> DNA
<213> Artificial Sequence
<220>
<223> FR shuffling primer F2VHS
<400> 68
ttctatgcat tgggtgcgac aggcccctgg acaagggctt gagtggattg gagggaatga   60
tcctgcgaat ggccatctt                                                79
<210> 69
<211> 79
<212> DNA

<213> Artificial Sequence
<220>
<223> FR shuffling primer F2VHA
<400> 69
aagatggcca ttcgcaggat cattccctcc aatccactca agcccttgtc caggggcctg 60
tcgcacccaa tgcatagaa 79
<210> 70
<211> 414
<212> DNA
<213> Artificial Sequence
<220>
<221> sig-peptide
<222> (1)...(57)
<220>
<221> mat-peptide
<222> (58)...(414)
<223> Nucleotide sequence coding for version "b3" of
humanized H chain V region
<400> 70
atg aaa tgc agc tgg gtc atc ttc ttc ctg atg gca gtg gtt aca ggg 48
Met Lys Cys Ser Trp Val Ile Phe Phe Leu Met Ala Val Val Thr Gly
-15 -10 -5
gtt aac tca cag gtg cag ctg ttg gag tct gga gct gtg ctg gca agg 96
Val Asn Ser Gln Val Gln Leu Leu Glu Ser Gly Ala Val Leu Ala Arg
1 5 10
cct ggg act tcc gtg aag atc tcc tgc aag gct tcc gga ttc aac att 144
Pro Gly Thr Ser Val Lys Ile Ser Cys Lys Ala Ser Gly Phe Asn Ile
15 20 25
aaa gac tac tat atg cat tgg gtg cga cag gcc cct gga caa ggg ctt 192
Lys Asp Tyr Tyr Met His Trp Val Arg Gln Ala Pro Gly Gln Gly Leu
30 35 40 45
gag tgg att gga ggg aat gat cct gcg aat ggc cat agt atg tat gac 240
Glu Trp Ile Gly Gly Asn Asp Pro Ala Asn Gly His Ser Met Tyr Asp
    50                  55                  60
ccg aaa ttc cag ggc cga gtc aca atc act gca gac aca tcc acg aac 288

Pro Lys Phe Gln Gly Arg Val Thr Ile Thr Ala Asp Thr Ser Thr Asn
65 70 75
aca gcc tac atg gag ctc tcg agt ctg aga tct gag gac aca gcc att 336
Thr Ala Tyr Met Glu Leu Ser Ser Leu Arg Ser Glu Asp Thr Ala Ile
80 85 90
tat tac tgt gca aga gac tcg ggc tat gcc atg gac tac tgg ggc caa 384
Tyr Tyr Cys Ala Arg Asp Ser Gly Tyr Ala Met Asp Tyr Trp Gly Gln
95 100 105
ggc acc ctg gtc acc gtc tcc tca gct agc 414
Gly Thr Leu Val Thr Val Ser Ser Ala Ser
110 115
<210> 71
<211> 119
<212> PRT
<213> Artificial Sequence
<220>
<223> Amino acid sequence of version "b3" of humanized H
chain V region
<400> 71
Gln Val Gln Leu Leu Glu Ser Gly Ala Val Leu Ala Arg Pro Gly Thr
1               5                   10                  15
Ser Val Lys Ile Ser Cys Lys Ala Ser Gly Phe Asn Ile Lys Asp Tyr
            20                  25                  30
Tyr Met His Trp Val Arg Gln Ala Pro Gly Gln Gly Leu Glu Trp Ile
        35                  40                  45
Gly Gly Asn Asp Pro Ala Asn Gly His Ser Met Tyr Asp Pro Lys Phe
    50                  55                  60
Gln Gly Arg Val Thr Ile Thr Ala Asp Thr Ser Thr Asn Thr Ala Tyr
65                  70                  75                  80
Met Glu Leu Ser Ser Leu Arg Ser Glu Asp Thr Ala Ile Tyr Tyr Cys
                85                  90                  95
Ala Arg Asp Ser Gly Tyr Ala Met Asp Tyr Trp Gly Gln Gly Thr Leu
            100                 105                 110
Val Thr Val Ser Ser Ala Ser
        115
<210> 72
<211> 414

<212> DNA
<213> Artificial Sequence
<220>
<221> sig-peptide
<222> (1)...(57)
<220>
<221> mat-peptide
<222> (58)...(414)
<223> Nucleotide sequence coding for version "d3" of
humanized H chain V region
<400> 72
atg aaa tgc agc tgg gtc atc ttc ttc ctg atg gca gtg gtt aca ggg 48
Met Lys Cys Ser Trp Val Ile Phe Phe Leu Met Ala Val Val Thr Gly
-15 -10 -5
gtt aac tca cag gtg cag ctg ttg gag tct gga gct gtg ctg gca agg 96
Val Asn Ser Gln Val Gln Leu Leu Glu Ser Gly Ala Val Leu Ala Arg
1 5 10
cct ggg act tcc gtg aag atc tcc tgc aag gct tcc gga ttc aac att 144
Pro Gly Thr Ser Val Lys Ile Ser Cys Lys Ala Ser Gly Phe Asn Ile
15 20 25
aaa gac tac tat atg cat tgg gtg cga cag gcc cct gga caa ggg ctt 192
Lys Asp Tyr Tyr Met His Trp Val Arg Gln Ala Pro Gly Gln Gly Leu
30 35 40 45
gag tgg att gga ggg aat gat cct gcg aat ggc cat agt atg tat gac 240
Glu Trp Ile Gly Gly Asn Asp Pro Ala Asn Gly His Ser Met Tyr Asp
    50                  55                  60
ccg aaa ttc cag ggc aga gtc acg att act gcg gac gaa tcc acg agc 288
Pro Lys Phe Gln Gly Arg Val Thr Ile Thr Ala Asp Glu Ser Thr Ser
65 70 75
aca gcc tac atg gag ctc tcg agt ctg aga tct gag gac tcg gcc gta 336
Thr Ala Tyr Met Glu Leu Ser Ser Leu Arg Ser Glu Asp Ser Ala Val
80 85 90
tat ttc tgt gcg aga gac tcg ggc tat gcc atg gac tac tgg ggc caa 384
Tyr Phe Cys Ala Arg Asp Ser Gly Tyr Ala Met Asp Tyr Trp Gly Gln
95 100 105
ggc acc ctg gtc acc gtc tcc tca gct agc 414

Gly Thr Leu Val Thr Val Ser Ser Ala Ser
110                 115
<210> 73
<211> 119
<212> PRT
<213> Artificial Sequence
<220>
<223> Amino acid sequence of version "d3" of humanized H
chain V region
<400> 73
Gln Val Gln Leu Leu Glu Ser Gly Ala Val Leu Ala Arg Pro Gly Thr
1               5                   10                  15
Ser Val Lys Ile Ser Cys Lys Ala Ser Gly Phe Asn Ile Lys Asp Tyr
            20                  25                  30
Tyr Met His Trp Val Arg Gln Ala Pro Gly Gln Gly Leu Glu Trp Ile
        35                  40                  45
Gly Gly Asn Asp Pro Ala Asn Gly His Ser Met Tyr Asp Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Ser Ala Val Tyr Phe Cys
                85                  90                  95
Ala Arg Asp Ser Gly Tyr Ala Met Asp Tyr Trp Gly Gln Gly Thr Leu
            100                 105                 110
Val Thr Val Ser Ser Ala Ser
        115
<210> 74
<211> 98
<212> DNA
<213> Artificial Sequence
<220>
<223> FR shuffling vector Lv1S
<400> 74
gtctagatct ccaccatgag ggcccctgct cagttttttg ggatcttgtt gctctggttt   60
ccagggatcc gatgtgacat ccagatgacc cagtctcc                          98
<210> 75

<211> 98
<212> DNA
<213> Artificial Sequence
<220>
<223> FR shuffling vector h5Lv4S
<400> 75
ttggcagatg gggtcccatc aaggttcagt ggctccggat ctggtaccga tttcactctc 60
accatctcga gtctgcaacc tgaagatttt gcaactta 98
<210> 76
<211> 98
<212> DNA
<213> Artificial Sequence
<220>
<223> FR shuffling vector h5Lv2A
<400> 76
cttaagaagc ttttaatgtc ctgtgaggcc ttgcacgtga tggtgactct gtctcctaca 60
gatgcagaca gggaggatgg agactgggtc atctggat 98
<210> 77
<211> 98
<212> DNA
<213> Artificial Sequence
<220>
<223> FR shuffling vector h5Lv3A
<400> 77
gatgggaccc catctgccaa actagttgca taatagatca ggagcttagg ggctttccct 60
ggtttctgct gataccaact taagaagctt ttaatgtc 98
<210> 78
<211> 94
<212> DNA
<213> Artificial Sequence
<220>
<223> FR shuffling vector h5Lv5A
<400> 78
tgttcgtacg tttgatctcc accttggtcc ctccgccgaa cgtgtacggg ctctcaccat 60
gctgcagaca gtagtaagtt gcaaaatctt cagg 94

<210> 79
<211> 20
<212> DNA
<213> Artificial Sequence
<220>
<223> Primer h5LvS
<400> 79
gtctagatct ccaccatgag                                                    20
<210> 80
<211> 19
<212> DNA
<213> Artificial Sequence
<220>
<223> Primer h5LvA
<400> 80
tgttcgtacg tttgatctc                                                     19
<210> 81
<211> 381
<212> DNA
<213> Artificial Sequence
<220>
<221> sig-peptide
<222> (1)...(60)
<220>
<221> mat-peptide
<222> (61)...(381)
<223> Nucleotide sequence coding for version "a" of humanized
L chain V region
<400> 81
atg agg gcc cct gct cag ttt ttt ggg atc ttg ttg ctc tgg ttt cca     48
Met Arg Ala Pro Ala Gln Phe Phe Gly Ile Leu Leu Leu Trp Phe Pro
-20                 -15                 -10                 -5
ggg atc cga tgt gac atc cag atg acc cag tct cca tcc tcc ctg tct     96
Gly Ile Arg Cys Asp Ile Gln Met Thr Gln Ser Pro Ser Ser Leu Ser
                1               5                   10
gca tct gta gga gac aga gtc acc atc acg tgc aag gcc tca cag gac    144

Ala Ser Val Gly Asp Arg Val Thr Ile Thr Cys Lys Ala Ser Gln Asp
15 20 25
att aaa agc ttc tta agt tgg tat cag cag aaa cca ggg aaa gcc cct 192
Ile Lys Ser Phe Leu Ser Trp Tyr Gln Gln Lys Pro Gly Lys Ala Pro
30 35 40
aag ctc ctg atc tat tat gca act agt ttg gca gat ggg gtc cca tca 240
Lys Leu Leu Ile Tyr Tyr Ala Thr Ser Leu Ala Asp Gly Val Pro Ser
45 50 55 60
agg ttc agt ggc tcc gga tct ggt acc gat ttc act ctc acc atc tcg 288
Arg Phe Ser Gly Ser Gly Ser Gly Thr Asp Phe Thr Leu Thr Ile Ser
65 70 75
agt ctg caa cct gaa gat ttt gca act tac tac tgt ctg cag cat ggt 336
Ser Leu Gln Pro Glu Asp Phe Ala Thr Tyr Tyr Cys Leu Gln His Gly
80 85 90
gag agc ccg tac acg ttc ggc gga ggg acc aag gtg gag atc aaa 381
Glu Ser Pro Tyr Thr Phe Gly Gly Gly Thr Lys Val Glu Ile Lys
95 100 105
<210> 82
<211> 107
<212> PRT
<213> Artificial Sequence
<220>
<223> Amino acid sequence of version "a" of humanized L chain
V region
<400> 82
Asp Ile Gln Met Thr Gln Ser Pro Ser Ser Leu Ser Ala Ser Val Gly
1               5                   10                  15
Asp Arg Val Thr Ile Thr Cys Lys Ala Ser Gln Asp Ile Lys Ser Phe
            20                  25                  30
Leu Ser Trp Tyr Gln Gln Lys Pro Gly Lys Ala Pro Lys Leu Leu Ile
        35                  40                  45
Tyr Tyr Ala Thr Ser Leu Ala Asp Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Leu Gln His Gly Glu Ser Pro Tyr
                85                  90                  95

Thr Phe Gly Gly Gly Thr Lys Val Glu Ile Lys
            100                 105
<210> 83
<211> 77
<212> DNA
<213> Artificial Sequence
<220>
<223> FR shuffling primer F3SS
<400> 83
gtctggtacc gattacactc tcaccatctc gagcctccag cctgaagatt ttgcaactta 60
ctattgtctg cagaaca 77
<210> 84
<211> 77
<212> DNA
<213> Artificial Sequence
<220>
<223> FR shuffling primer F3SA
<400> 84
tgttctgcag acaatagtaa gttgcaaaat cttcaggctg gaggctcgag atggtgagag 60
tgtaatcggt accagac 77
<210> 85
<211> 77
<212> DNA
<213> Artificial Sequence
<220>
<223> FR shuffling primer F3RS
<400> 85
gtctggtacc gattacactc tcaccatctc gagcctccag cctgaagata ttgcaactta 60
ctattgtctg cagaaca 77
<210> 86
<211> 77
<212> DNA
<213> Artificial Sequence
<220>
<223> FR shuffling primer F3RA

<400> 86
tgttctgcag acaatagtaa gttgcaatat cttcaggctg gaggctcgag atggtgagag 60
tgtaatcggt accagac 77
<210> 87
<211> 381
<212> DNA
<213> Artificial Sequence
<220>
<221> sig-peptide
<222> (1)...(60)
<220>
<221> mat-peptide
<222> (61)...(381)
<223> Nucleotide sequence coding for version "b" of humanized
L chain V region
<400> 87
atg agg gcc cct gct cag ttt ttt ggg atc ttg ttg ctc tgg ttt cca 48
Met Arg Ala Pro Ala Gln Phe Phe Gly Ile Leu Leu Leu Trp Phe Pro
-20 -15 -10 -5
ggg atc cga tgt gac atc cag atg acc cag tct cca tcc tcc ctg tct 96
Gly Ile Arg Cys Asp Ile Gln Met Thr Gln Ser Pro Ser Ser Leu Ser
1 5 10
gca tct gta gga gac aga gtc acc atc acg tgc aag gcc tca cag gac 144
Ala Ser Val Gly Asp Arg Val Thr Ile Thr Cys Lys Ala Ser Gln Asp
15 20 25
att aaa agc ttc tta agt tgg tat cag cag aaa cca ggg aaa gcc cct 192
Ile Lys Ser Phe Leu Ser Trp Tyr Gln Gln Lys Pro Gly Lys Ala Pro
30 35 40
aag ctc ctg atc tat tat gca act agt ttg gca gat ggg gtc cca tca 240
Lys Leu Leu Ile Tyr Tyr Ala Thr Ser Leu Ala Asp Gly Val Pro Ser
45 50 55 60
agg ttc agt ggc tcc gga tct ggt acc gat tac act ctc acc atc tcg 288
Arg Phe Ser Gly Ser Gly Ser Gly Thr Asp Tyr Thr Leu Thr Ile Ser
65 70 75
agc ctc cag cct gaa gat ttt gca act tac tat tgt ctg cag cat ggt 336

Ser Leu Gln Pro Glu Asp Phe Ala Thr Tyr Tyr Cys Leu Gln His Gly
80 85 90
gag agc ccg tac acg ttc ggc gga ggg acc aag gtg gag atc aaa 381
Glu Ser Pro Tyr Thr Phe Gly Gly Gly Thr Lys Val Glu Ile Lys
95 100 105
<210> 88
<211> 107
<212> PRT
<213> Artificial Sequence
<220>
<223> Amino acid sequence of version "b" of humanized L chain
V region
<400> 88
Asp Ile Gln Met Thr Gln Ser Pro Ser Ser Leu Ser Ala Ser Val Gly
1               5                   10                  15
Asp Arg Val Thr Ile Thr Cys Lys Ala Ser Gln Asp Ile Lys Ser Phe
            20                  25                  30
Leu Ser Trp Tyr Gln Gln Lys Pro Gly Lys Ala Pro Lys Leu Leu Ile
        35                  40                  45
Tyr Tyr Ala Thr Ser Leu Ala Asp Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Tyr Cys Leu Gln His Gly Glu Ser Pro Tyr
                85                  90                  95
Thr Phe Gly Gly Gly Thr Lys Val Glu Ile Lys
            100                 105
<210> 89
<211> 381
<212> DNA
<213> Artificial Sequence
<220>
<221> sig-peptide
<222> (1)...(60)
<220>
<221> mat-peptide

<222> (61)...(381)
<223> Nucleotide sequence coding for version "c" of humanized
L chain V region
<400> 89
atg agg gcc cct gct cag ttt ttt ggg atc ttg ttg ctc tgg ttt cca     48
Met Arg Ala Pro Ala Gln Phe Phe Gly Ile Leu Leu Leu Trp Phe Pro
-20                 -15                 -10                 -5
ggg atc cga tgt gac atc cag atg acc cag tct cca tcc tcc ctg tct     96
Gly Ile Arg Cys Asp Ile Gln Met Thr Gln Ser Pro Ser Ser Leu Ser
                1               5                   10
gca tct gta gga gac aga gtc acc atc acg tgc aag gcc tca cag gac     144
Ala Ser Val Gly Asp Arg Val Thr Ile Thr Cys Lys Ala Ser Gln Asp
        15                  20                  25
att aaa agc ttc tta agt tgg tat cag cag aaa cca ggg aaa gcc cct     192
Ile Lys Ser Phe Leu Ser Trp Tyr Gln Gln Lys Pro Gly Lys Ala Pro
    30                  35                  40
aag ctc ctg atc tat tat gca act agt ttg gca gat ggg gtc cca tca     240
Lys Leu Leu Ile Tyr Tyr Ala Thr Ser Leu Ala Asp Gly Val Pro Ser
45                  50                  55                  60
agg ttc agt ggc tcc gga tct ggt acc gat tac act ctc acc atc tcg     288
Arg Phe Ser Gly Ser Gly Ser Gly Thr Asp Tyr Thr Leu Thr Ile Ser
                65                  70                  75
agc ctc cag cct gaa gat att gca act tac tat tgt ctg cag cat ggt     336
Ser Leu Gln Pro Glu Asp Ile Ala Thr Tyr Tyr Cys Leu Gln His Gly
            80                  85                  90
gag agc ccg tac acg ttc ggc gga ggg acc aag gtg gag atc aaa         381
Glu Ser Pro Tyr Thr Phe Gly Gly Gly Thr Lys Val Glu Ile Lys
        95                  100                 105
<210> 90
<211> 107
<212> PRT
<213> Artificial Sequence
<220>
<223> Amino acid sequence of version "c" of humanized L chain
V region
<400> 90

Asp Ile Gln Met Thr Gln Ser Pro Ser Ser Leu Ser Ala Ser Val Gly
1               5                   10                  15
Asp Arg Val Thr Ile Thr Cys Lys Ala Ser Gln Asp Ile Lys Ser Phe
            20                  25                  30
Leu Ser Trp Tyr Gln Gln Lys Pro Gly Lys Ala Pro Lys Leu Leu Ile
        35                  40                  45
Tyr Tyr Ala Thr Ser Leu Ala Asp Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Ile Ala Thr Tyr Tyr Cys Leu Gln His Gly Glu Ser Pro Tyr
                85                  90                  95
Thr Phe Gly Gly Gly Thr Lys Val Glu Ile Lys
            100                 105
<210> 91
<211> 72
<212> DNA
<213> Artificial Sequence
<220>
<223> FR shuffling primer F2SS
<400> 91
gtctcttaag ttggttccag cagaaaccag ggaaatctcc taagaccctg atctactatg   60
caactagtaa ca                                                       72
<210> 92
<211> 72
<212> DNA
<213> Artificial Sequence
<220>
<223> FR shuffling primer F2SA
<400> 92
tgttactagt tgcatagtag atcagggtct taggagattt ccctggtttc tgctggaacc   60
aacttaagag ac                                                       72
<210> 93
<211> 72
<212> DNA
<213> Artificial Sequence

<220>
<223> FR shuffling primer F2XS
<400> 93
gtctcttaag ttggtatcag cagaaaccag agaaagcccc taagtccctg atctattatg 60
caactagtaa ca 72
<210> 94
<211> 72
<212> DNA
<213> Artificial Sequence
<220>
<223> FR shuffling primer F2XA
<400> 94
tgttactagt tgcataatag atcagggact taggggcttt ctctggtttc tgctgatacc 60
aacttaagag ac 72
<210> 95
<211> 381
<212> DNA
<213> Artificial Sequence
<220>
<221> sig-peptide
<222> (1)...(60)
<220>
<221> mat-peptide
<222> (61)...(381)
<223> Nucleotide sequence coding for version "b1" of
humanized L chain V region
<400> 95
atg agg gcc cct gct cag ttt ttt ggg atc ttg ttg ctc tgg ttt cca 48
Met Arg Ala Pro Ala Gln Phe Phe Gly Ile Leu Leu Leu Trp Phe Pro
-20 -15 -10 -5
ggg atc cga tgt gac atc cag atg acc cag tct cca tcc tcc ctg tct 96
Gly Ile Arg Cys Asp Ile Gln Met Thr Gln Ser Pro Ser Ser Leu Ser
1 5 10
gca tct gta gga gac aga gtc acc atc acg tgc aag gcc tca cag gac 144
Ala Ser Val Gly Asp Arg Val Thr Ile Thr Cys Lys Ala Ser Gln Asp
15 20 25

att aaa agc ttc tta agt tgg ttc cag cag aaa cca ggg aaa tct cct 192
Ile Lys Ser Phe Leu Ser Trp Phe Gln Gln Lys Pro Gly Lys Ser Pro
30 35 40
aag acc ctg atc tac tat gca act agt ttg gca gat ggg gtc cca tca 240
Lys Thr Leu Ile Tyr Tyr Ala Thr Ser Leu Ala Asp Gly Val Pro Ser
45 50 55 60
agg ttc agt ggc tcc gga tct ggt acc gat tac act ctc acc atc tcg 288
Arg Phe Ser Gly Ser Gly Ser Gly Thr Asp Tyr Thr Leu Thr Ile Ser
65 70 75
agc ctc cag cct gaa gat ttt gca act tac tat tgt ctg cag cat ggt 336
Ser Leu Gln Pro Glu Asp Phe Ala Thr Tyr Tyr Cys Leu Gln His Gly
80 85 90
gag agc ccg tac acg ttc ggc gga ggg acc aag gtg gag atc aaa 381
Glu Ser Pro Tyr Thr Phe Gly Gly Gly Thr Lys Val Glu Ile Lys
95 100 105
<210> 96
<211> 107
<212> PRT
<213> Artificial Sequence
<220>
<223> Amino acid sequence of version "b1" of humanized L
chain V region
<400> 96
Asp Ile Gln Met Thr Gln Ser Pro Ser Ser Leu Ser Ala Ser Val Gly
1               5                   10                  15
Asp Arg Val Thr Ile Thr Cys Lys Ala Ser Gln Asp Ile Lys Ser Phe
            20                  25                  30
Leu Ser Trp Phe Gln Gln Lys Pro Gly Lys Ser Pro Lys Thr Leu Ile
        35                  40                  45
Tyr Tyr Ala Thr Ser Leu Ala Asp Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Tyr Cys Leu Gln His Gly Glu Ser Pro Tyr
                85                  90                  95
Thr Phe Gly Gly Gly Thr Lys Val Glu Ile Lys
            100                 105

<210> 97
<211> 381
<212> DNA
<213> Artificial Sequence
<220>
<221> sig-peptide
<222> (1)...(60)
<220>
<221> mat-peptide
<222> (61)...(381)
<223> Nucleotide sequence coding for version "b2" of
humanized L chain V region
<400> 97
atg agg gcc cct gct cag ttt ttt ggg atc ttg ttg ctc tgg ttt cca 48
Met Arg Ala Pro Ala Gln Phe Phe Gly Ile Leu Leu Leu Trp Phe Pro
-20 -15 -10 -5
ggg atc cga tgt gac atc cag atg acc cag tct cca tcc tcc ctg tct 96
Gly Ile Arg Cys Asp Ile Gln Met Thr Gln Ser Pro Ser Ser Leu Ser
1 5 10
gca tct gta gga gac aga gtc acc atc acg tgc aag gcc tca cag gac 144
Ala Ser Val Gly Asp Arg Val Thr Ile Thr Cys Lys Ala Ser Gln Asp
15 20 25
att aaa agc ttc tta agt tgg tat cag cag aaa cca gag aaa gcc cct 192
Ile Lys Ser Phe Leu Ser Trp Tyr Gln Gln Lys Pro Glu Lys Ala Pro
30 35 40
aag tcc ctg atc tat tat gca act agt ttg gca gat ggg gtc cca tca 240
Lys Ser Leu Ile Tyr Tyr Ala Thr Ser Leu Ala Asp Gly Val Pro Ser
45 50 55 60
agg ttc agt ggc tcc gga tct ggt acc gat tac act ctc acc atc tcg 288
Arg Phe Ser Gly Ser Gly Ser Gly Thr Asp Tyr Thr Leu Thr Ile Ser
65 70 75
agc ctc cag cct gaa gat ttt gca act tac tat tgt ctg cag cat ggt 336
Ser Leu Gln Pro Glu Asp Phe Ala Thr Tyr Tyr Cys Leu Gln His Gly
80 85 90
gag agc ccg tac acg ttc ggc gga ggg acc aag gtg gag atc aaa 381

Glu Ser Pro Tyr Thr Phe Gly Gly Gly Thr Lys Val Glu Ile Lys
95 100 105
<210> 98
<211> 107
<212> PRT
<213> Artificial Sequence
<220>
<223> Amino acid sequence of version "b2" of humanized L
chain V region
<400> 98
Asp Ile Gln Met Thr Gln Ser Pro Ser Ser Leu Ser Ala Ser Val Gly
1               5                   10                  15
Asp Arg Val Thr Ile Thr Cys Lys Ala Ser Gln Asp Ile Lys Ser Phe
            20                  25                  30
Leu Ser Trp Tyr Gln Gln Lys Pro Glu Lys Ala Pro Lys Ser Leu Ile
        35                  40                  45
Tyr Tyr Ala Thr Ser Leu Ala Asp Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Tyr Cys Leu Gln His Gly Glu Ser Pro Tyr
                85                  90                  95
Thr Phe Gly Gly Gly Thr Lys Val Glu Ile Lys
            100                 105
<210> 99
<211> 117
<212> PRT
<213> Mouse
<220>
<223> Amino acid sequence of H chain V region of anti TF
mouse monoclonal antibody ATR-5
<400> 99
Glu Val Gln Leu Gln Gln Ser Gly Thr Asn Leu Val Arg Pro Gly Ala
                5                   10                  15
Leu Val Lys Leu Ser Cys Lys Gly Ser Gly Phe Asn Ile Lys Asp Tyr
            20                  25                  30

Tyr Met His Trp Val Lys Gln Arg Pro Glu Gln Gly Leu Glu Trp Ile
        35                  40                  45
Gly Gly Asn Asp Pro Ala Asn Gly His Ser Met Tyr Asp Pro Lys Phe
    50                  55                  60
Gln Gly Lys Ala Ser Ile Thr Ala Asp Thr Ser Ser Asn Thr Ala Tyr
65                  70                  75                  80
Leu Gln Leu Ser Ser Leu Thr Ser Glu Asp Thr Ala Val Tyr Phe Cys
                85                  90                  95
Ala Arg Asp Ser Gly Tyr Ala Met Asp Tyr Trp Gly Gln Gly Thr Ser
            100                 105                 110
Val Thr Val Ser Ser
        115
<210> 100
<211> 107
<212> PRT
<213> Mouse
<220>
<223> Amino acid sequence of L chain V region of anti TF
mouse monoclonal antibody ATR-5
<400> 100
Asp Ile Lys Met Thr Gln Ser Pro Ser Ser Met Tyr Ala Ser Leu Gly
                5                   10                  15
Glu Arg Val Thr Ile Thr Cys Lys Ala Ser Gln Asp Ile Lys Ser Phe
            20                  25                  30
Leu Ser Trp Tyr Gln Gln Lys Pro Trp Lys Ser Pro Lys Thr Leu Ile
        35                  40                  45
Tyr Tyr Ala Thr Ser Leu Ala Asp Gly Val Pro Ser Arg Phe Ser Gly
    50                  55                  60
Ser Gly Ser Gly Gln Asp Tyr Ser Leu Thr Ile Asn Asn Leu Glu Ser
65                  70                  75                  80
Asp Asp Thr Ala Thr Tyr Tyr Cys Leu Gln His Gly Glu Ser Pro Tyr
                85                  90                  95
Thr Phe Gly Gly Gly Thr Lys Leu Glu Ile Lys
            100                 105
<210> 101
<211> 780

<212> DNA
<213> Homo sapiens
<220>
<223> DNA coding for soluble human TF
<400> 101
atg gag acc cct gcc tgg ccc cgg gtc ccg cgc ccc gag acc gcc gtc 48
Met Glu Thr Pro Ala Trp Pro Arg Val Pro Arg Pro Glu Thr Ala Val
-30 -25 -20
gct cgg acg ctc ctg ctc ggc tgg gtc ttc gcc cag gtg gcc ggc gct 96
Ala Arg Thr Leu Leu Leu Gly Trp Val Phe Ala Gln Val Ala Gly Ala
-15 -10 -5 -1
tca ggc act aca aat act gtg gca gca tat aat tta act tgg aaa tca 144
Ser Gly Thr Thr Asn Thr Val Ala Ala Tyr Asn Leu Thr Trp Lys Ser
1               5                   10                  15
act aat ttc aag aca att ttg gag tgg gaa ccc aaa ccc gtc aat caa 192
Thr Asn Phe Lys Thr Ile Leu Glu Trp Glu Pro Lys Pro Val Asn Gln
            20                  25                  30
gtc tac act gtt caa ata agc act aag tca gga gat tgg aaa agc aaa 240
Val Tyr Thr Val Gln Ile Ser Thr Lys Ser Gly Asp Trp Lys Ser Lys
        35                  40                  45
tgc ttt tac aca aca gac aca gag tgt gac ctc acc gac gag att gtg 288
Cys Phe Tyr Thr Thr Asp Thr Glu Cys Asp Leu Thr Asp Glu Ile Val
    50                  55                  60
aag gat gtg aag cag acg tac ttg gca cgg gtc ttc tcc tac ccg gca 366
Lys Asp Val Lys Gln Thr Tyr Leu Ala Arg Val Phe Ser Tyr Pro Ala
65                  70                  75                  80
ggg aat gtg gag agc acc ggt tct gct ggg gag cct ctg tat gag aac 384
Gly Asn Val Glu Ser Thr Gly Ser Ala Gly Glu Pro Leu Tyr Glu Asn
                85                  90                  95
tcc cca gag ttc aca cct tac ctg gag aca aac ctc gga cag cca aca 432
Ser Pro Glu Phe Thr Pro Tyr Leu Glu Thr Asn Leu Gly Gln Pro Thr
            100                 105                 110
att cag agt ttt gaa cag gtg gga aca aaa gtg aat gtg acc gta gaa 480
Ile Gln Ser Phe Glu Gln Val Gly Thr Lys Val Asn Val Thr Val Glu
        115                 120                 125
gat gaa cgg act tta gtc aga agg aac aac act ttc cta agc ctc cgg 528

Asp Glu Arg Thr Leu Val Arg Arg Asn Asn Thr Phe Leu Ser Leu Arg
    130                 135                 140
gat gtt ttt ggc aag gac tta att tat aca ctt tat tat tgg aaa tct 576
Asp Val Phe Gly Lys Asp Leu Ile Tyr Thr Leu Tyr Tyr Trp Lys Ser
145                 150                 155                 160
tca agt tca gga aag aaa aca gcc aaa aca aac act aat gag ttt ttg 624
Ser Ser Ser Gly Lys Lys Thr Ala Lys Thr Asn Thr Asn Glu Phe Leu
                165                 170                 175
att gat gtg gat aaa gga gaa aac tac tgt ttc agt gtt caa gca gtg 672
Ile Asp Val Asp Lys Gly Glu Asn Tyr Cys Phe Ser Val Gln Ala Val
            180                 185                 190
att ccc tcc cga aca gtt aac cgg aag agt aca gac agc ccg gta gag 720
Ile Pro Ser Arg Thr Val Asn Arg Lys Ser Thr Asp Ser Pro Val Glu
        195                 200                 205
tgt atg ggc cag gag aaa ggg gaa ttc aga gaa gac tac aaa gac gat 768
Cys Met Gly Gln Glu Lys Gly Glu Phe Arg Glu Asp Tyr Lys Asp Asp
    210                 215                 220
gac gat aaa taa 780
Asp Asp Lys
225
<210> 102
<211> 259
<212> PRT
<220>
<223> Amino acid sequence of soluble human TF
<400> 102
Met Glu Thr Pro Ala Trp Pro Arg Val Pro Arg Pro Glu Thr Ala Val
-30 -25 -20
Ala Arg Thr Leu Leu Leu Gly Trp Val Phe Ala Gln Val Ala Gly Ala
-15 -10 -5 -1
Ser Gly Thr Thr Asn Thr Val Ala Ala Tyr Asn Leu Thr Trp Lys Ser
1               5                   10                  15
Thr Asn Phe Lys Thr Ile Leu Glu Trp Glu Pro Lys Pro Val Asn Gln
            20                  25                  30
Val Tyr Thr Val Gln Ile Ser Thr Lys Ser Gly Asp Trp Lys Ser Lys
        35                  40                  45

Cys Phe Tyr Thr Thr Asp Thr Glu Cys Asp Leu Thr Asp Glu Ile Val
    50                  55                  60
Lys Asp Val Lys Gln Thr Tyr Leu Ala Arg Val Phe Ser Tyr Pro Ala
65                  70                  75                  80
Gly Asn Val Glu Ser Thr Gly Ser Ala Gly Glu Pro Leu Tyr Glu Asn
                85                  90                  95
Ser Pro Glu Phe Thr Pro Tyr Leu Glu Thr Asn Leu Gly Gln Pro Thr
            100                 105                 110
Ile Gln Ser Phe Glu Gln Val Gly Thr Lys Val Asn Val Thr Val Glu
        115                 120                 125
Asp Glu Arg Thr Leu Val Arg Arg Asn Asn Thr Phe Leu Ser Leu Arg
    130                 135                 140
Asp Val Phe Gly Lys Asp Leu Ile Tyr Thr Leu Tyr Tyr Trp Lys Ser
145                 150                 155                 160
Ser Ser Ser Gly Lys Lys Thr Ala Lys Thr Asn Thr Asn Glu Phe Leu
                165                 170                 175
Ile Asp Val Asp Lys Gly Glu Asn Tyr Cys Phe Ser Val Gln Ala Val
            180                 185                 190
Ile Pro Ser Arg Thr Val Asn Arg Lys Ser Thr Asp Ser Pro Val Glu
        195                 200                 205
Cys Met Gly Gln Glu Lys Gly Glu Phe Arg Glu Asp Tyr Lys Asp Asp
    210                 215                 220
Asp Asp Lys
225